(19) Europäisches Patentamt
European Patent Office
Office européen des brevets

(11) EP 2 947 160 B1

(12) # EUROPEAN PATENT SPECIFICATION

(45) Date of publication and mention of the grant of the patent:
**12.07.2017 Bulletin 2017/28**

(51) Int Cl.:
***C12Q 1/68** (2006.01)*

(21) Application number: **15173816.8**

(22) Date of filing: **07.04.2005**

(54) **GENE EXPRESSION MARKERS FOR PREDICTING RESPONSE TO CHEMOTHERAPY**

GENEXPRESSIONSMARKER ZUR VORHERSAGE DER REAKTION AUF CHEMOTHERAPIE

MARQUEURS D'EXPRESSION DE GENES PERMETTANT DE PREDIRE UNE REPONSE A LA CHIMIOTHERAPIE

(84) Designated Contracting States:
**AT BE BG CH CY CZ DE DK EE ES FI FR GB GR HU IE IS IT LI LT LU MC NL PL PT RO SE SI SK TR**

(30) Priority: **09.04.2004 US 561035 P**

(43) Date of publication of application:
**25.11.2015 Bulletin 2015/48**

(62) Document number(s) of the earlier application(s) in accordance with Art. 76 EPC:
**09014283.7 / 2 163 650**
**05735478.9 / 1 737 980**

(73) Proprietor: **Genomic Health, Inc.**
**Redwood City, CA 94063 (US)**

(72) Inventors:
- **Baker, Joffre**
  **Montara, CA 94037 (US)**
- **Shak, Steven**
  **Hillsborough, CA 94010 (US)**
- **Gianni, Luca**
  **20122 Milano (IT)**

(74) Representative: **Gowshall, Jonathan Vallance**
**Forresters IP LLP**
**Skygarden**
**Erika-Mann-Strasse 11**
**80636 München (DE)**

(56) References cited:
**WO-A2-02/46467 WO-A2-2005/008213**

- **DUAN ZHENFENG ET AL: "GBP1 overexpression is associated with a paclitaxel resistance phenotype", CANCER CHEMOTHERAPY AND PHARMACOLOGY, vol. 57, no. 1, January 2006 (2006-01), pages 25-33, XP002743213, ISSN: 0344-5704**
- **BERTUCCI F ET AL: "GENE EXPRESSION PROFILES OF POOR-PROGNOSIS PRIMARY BREAST CANCER CORRELATE WITH SURVIVAL", HUMAN MOLECULAR GENETICS, OXFORD UNIVERSITY PRESS, SURREY, GB, vol. 11, no. 8, 15 April 2002 (2002-04-15) , pages 863-872, XP001146244, ISSN: 0964-6906**
- **RING A E ET AL: "PREDICTORS OF RESPONSE TO SYSTEMIC THERAPY IN BREAST CANCER", FORUM, GENOVA, IT, vol. 12, no. 1, 2002, pages 19-32, XP009012703, ISSN: 1121-8142**
- **MUSS H B ET AL: "C-ERBB-2 EXPRESSION AND RESPONSE TO ADJUVANT THERAPY IN WOMEN WITH NODE-POSITIVE EARLY BREAST CANCER", NEW ENGLAND JOURNAL OF MEDICINE, MASSACHUSETTS MEDICAL SOCIETY, BOSTON, MA, US, vol. 330, no. 18, 5 May 1994 (1994-05-05), pages 1260-1266, XP009040793, ISSN: 1533-4406**
- **LUBESEDER-MARTELLATO C ET AL: "GUANYLATE-BINDING PROTEIN-1 EXPRESSION IS SELECTIVELY INDUCED BY INFLAMMATORY CYTOKINES AND IS AN ACTIVATION MARKER OF ENDOTHELIAL CELLS DURING INFLAMMATORY DISEASES", AMERICAN JOURNAL OF PATHOLOGY; [10640], ELSEVIER INC, US, vol. 161, no. 5, 1 November 2002 (2002-11-01), pages 1749-1759, XP009029105, ISSN: 0002-9440**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 947 160 B1

## Description

The present invention provides sets of genes the expression of which is important in the prognosis of cancer. In particular, the invention provides gene expression information useful for predicting whether cancer patients are likely to have a beneficial treatment response to chemotherapy.

Oncologists have a number of treatment options available to them, including different combinations of chemotherapeutic drugs that are characterized as "standard of care," and a number of drugs that do not carry a label claim for particular cancer, but for which there is evidence of efficacy in that cancer. Best likelihood of good treatment outcome requires that patients be assigned to optimal available cancer treatment, and that this assignment be made as quickly as possible following diagnosis. In particular, it is important to determine the likelihood of patient response to "standard of care" chemotherapy because chemotherapeutic drugs such as anthracyclines and taxanes have limited efficacy and are toxic. The identification of patients who are most or least likely to respond thus could increase the net benefit these drugs have to offer, and decrease the net morbidity and toxicity, via more intelligent patient selection.

Currently, diagnostic tests used in clinical practice are single analyte, and therefore do not capture the potential value of knowing relationships between dozens of different markers. Moreover, diagnostic tests are frequently not quantitative, relying on immunohistochemistry. This method often yields different results in different laboratories, in part because the reagents are not standardized, and in part because the interpretations are subjective and cannot be easily quantified. RNA-based tests have not often been used because of the problem of RNA degradation over time and the fact that it is difficult to obtain fresh tissue samples from patients for analysis. Fixed paraffin-embedded tissue is more readily available and methods have been established to detect RNA in fixed tissue. However, these methods typically do not allow for the study of large numbers of genes (DNA or RNA) from small amounts of material. Thus, traditionally fixed tissue has been rarely used other than for immunohistochhemistry detection of proteins.

In the last few years, several groups have published studies concerning the classification of various cancer types by microarray gene expression analysis (see, e.g. Golub et al., Science 286:531-537 (1999); Bhattacharjae et al., Proc. Natl. Acad. Sci. USA 98:13790-13795 (2001); Chen-Hsiang et al., Bioinformatics 17 (Suppl. 1):S316-S322 (2001); Ramaswamy et al., Proc. Natl. Acad. Sci. USA 98:15149-15154 (2001)). Certain classifications of human breast cancers based on gene expression patterns have also been reported (Martin et al., Cancer Res. 60:2232-2238 (2000); West et al., Proc. Natl. Acad. Sci. USA 98:11462-11467 (2001); Sorlie et al., Proc. Natl. Acad. Sci. USA 98:10869-10874 (2001); Yan et al., Cancer Res. 61:8375-8380 (2001)). However, these studies mostly focus on improving and refining the already established classification of various types of cancer, including breast cancer, and generally do not provide new insights into the relationships of the differentially expressed genes, and do not link the findings to treatment strategies in order to improve the clinical outcome of cancer therapy.

Although modern molecular biology and biochemistry have revealed hundreds of genes whose activities influence the behavior of tumor cells, state of their differentiation, and their sensitivity or resistance to certain therapeutic drugs, with a few exceptions, the status of these genes has not been exploited for the purpose of routinely making clinical decisions about drug treatments. One notable exception is the use of estrogen receptor (ER) protein expression in breast carcinomas to select patients to treatment with anti-estrogen drugs, such as tamoxifen. Another exceptional example is the use of BrbB2 (Her2) protein expression in breast carcinomas to select patients with the Her2 antagonist drug Herceptin® (Genentech, Inc., South San Francisco, CA).

Despite recent advances, the challenge of cancer treatment remains to target specific treatment regimens to pathogenically distinct tumor types, and ultimately personalize tumor treatment in order to maximize outcome. Hence, a need exists for tests that simultaneously provide predictive information about patient responses to the variety of treatment options. This is particularly true for breast cancer, the biology of which is poorly understood. It is clear that the classification of breast cancer into a few subgroups, such as the ErbB2 positive subgroup, and subgroups characterized by low to absent gene expression of the estrogen receptor (ER) and a few additional transcriptional factors (Perou et al., Nature 406:747-752 (2000)), does not reflect the cellular and molecular heterogeneity of breast cancer, and does not allow the design of treatment strategies maximizing patient response.

Breast cancer is the most common type of cancer among women in the United States and is the leading cause of cancer deaths among women ages 40 - 59. Therefore, there is a particularly great need for a clinically validated breast cancer test predictive of patient response to chemotherapy.

The present invention provides gene sets useful in predicting the response of cancer, e.g. breast cancer patients to chemotherapy. In addition, the invention provides a clinically validated cancer, e.g. breast cancer, test, predictive of patient response to chemotherapy, using multi-gene RNA analysis. The present invention accommodates the use of archived paraffin-embedded biopsy material for assay of all markers in the relevant gene sets, and therefore is compatible with the most widely available type of biopsy material.

In one aspect, the present invention concerns a method for predicting the response of a subject diagnosed with cancer to chemotherapy comprising determining the expression level of one or more prognostic RNA transcripts or their expression products in a biological sample comprising cancer cells obtained from said subject, wherein the predictive RNA transcript is the transcript of one or more genes selected from the group consisting of TBP; ILT.2; ABCC5; CD18; GATA3; DICER1; MSH3; GBP1; IRS1; CD3z; fas1; TUBB; BAD; ERCC1; MCM6; PR; APC; GGPS1; KRT18; ESRRG; E2F1; AKT2; A.Catenin; CEGP1; NPD009; MAPK14; RUNX1; ID2; G.Catenin; FBXO5; FHIT; MTA1; ERBB4; FUS; BBC3; IGF1R; CD9; TP53BP1; MUC1; IGFBP5; rhoC; RALBP1; CDC20; STAT3; ERK1; HLA.DPB1; SGCB; CGA; DHPS; MGMT; CRIP2; MMP12; ErbB3; RAP1GDS1; CDC25B; IL6; CCND1; CYBA; PRKCD; DR4; Hepsin; CRABP1; AK055699; Contig.51037; VCAM1; FYN; GRB7; AKAP.2; RASSF1; MCP1; ZNF38; MCM2; GBP2; SEMA3F; CD31; COL1A1; ER2; BAG1; AKT1; COL1A2; STAT1; Wnt.5a; PTPD1; RAB6C; TK1, ErbB2, CCNB1, BIRC5, STK6, MKI67, MYBL2, MMP11, CTSL2, CD68, GSTM1, BCL2, ESR1 wherein

(a) for every unit of increased expression of one or more of ILT.2; CD18; GBP1; CD3z; fas1; MCM6; E2F1; ID2; FBXO5; CDC20; HLA.DPB1; CGA; MMP12; CDC25B; IL6; CYBA; DR4; CRABP1; Contig.51037; VCAM1; FYN; GRB7; AKAP.2; RASSF11; MCP1; MCM2; GBP2; CD31; ER2; STAT1; TK1; ERBB2, CCNB1, BIRC5, STK6, MKI67, MYBL2, MMP11, CTSL2 and CD68; or the corresponding expression product, said subject is predicted to have an increased likelihood of response to chemotherapy; and

(b) for every unit of increased expression of one or more of TBP; ABCC5; GATA3; DICER1; MSH3; IRS1; TUBB; BAD; ERCC1; PR; APC; GGPS1; KRT18; ESRRG; AKT2; A.Catenin; CEGP1; NPD009; MAPK14; RUNX1; G.Catenin; FHIT; MTA1; ErbB4; FUS; BBC3; IGF1R; CD9; TP53BP1; MUC1; IGFBP5; rhoC; RALBP1; STAT3; ERK1; SGCB; DHPS; MGMT; CRIP2; ErbB3; RAP1GDS1; CCND1; PRKCD; Hepsin; AK055699; ZNF38; SEMA3F; COL1A1; BAG1; AKT1; COL1A2; Wnt.5a; PTPD1; RAB6C; GSTM1, BCL2, ESR1; or the corresponding expression product, said subject is predicted to have a decreased likelihood of response to chemotherapy.

In a particular embodiment, in the above method the predictive RNA transcript is the transcript of one or more genes selected from the group consisting of TBP; ILT.2; ABCC5; CD18; GATA3; DICER1; MSH3; GBP1; IRS1; CD3z; fas1; TUBB; BAD; ERCC1; MCM6; PR; APC; GGPS1; KRT18; ESRRG; E2F1; AKT2; A.Catenin; CEGP1; NPD009; MAPK14; RUNX1; ID2; G,Catenin; FBXO5; FHIT; MTA1; ERBB4; FUS; BBC3; IGF1R; CD9; TP53BP1; MUC1; IGFBP5; rhoC; RALBP1; CDC20; STAT3; ERK1; HLA.DPB1; SGCB; CGA; DHPS; MGMT; CRIP2; MMP12; ErbB3; RAP1GDS1; CDC25B; IL6; CCND1; CYBA; PRKCD; DR4; Hepsin; CRABP1; AK055699; Contig.51037; VCAM1; FYN; GRB7; AKAP.2; RASSF1; MCP1; ZNF38; MCM2; GBP2; SEMA3F; CD31; COL1A1; ER2; BAG1; AKT1; COL1A2; STAT1; Wnt.5a; PTPD1; RAB6C; and TK1.

In another embodiment, the response is a complete pathological response.

In a preferred embodiment, the subject is a human patient.

The cancer can be any types of cancer but preferably is a solid tumor, such as breast cancer, ovarian cancer, gastric cancer, colon cancer, pancreatic cancer, prostate cancer and lung cancer.

Of the tumor is breast cancer, it can, for example, be invasive breast cancer, or stage II or stage III breast cancer.

In a particular embodiment, the chemotherapy is adjuvant chemotherapy.

In another embodiment, the chemotherapy is neoadjuvant chemotherapy.

The neoadjuvant chemotherapy may, for example, comprise the administration of a taxane derivativ, such as docetaxel and/or paclitaxel, and/or other anti-cancer agents, such as, members of the anthracycline class of anti-cancer agents, doxorubicin, topoisomerase inhibitors, etc..

The method may involve determination of the expression levels of at least two, or at least five, or at least ten, or at least 15 of the prognostic transcripts listed above, or their expression products.

The biological sample may be e.g. a tissue sample comprising cancer cells, where the tissue can be fixed, paraffin-embedded, or fresh, or frozen.

In a particular embodiment, the tissue is from fine needle, core, or other types of biopsy.

In another embodiment, the tissue sample is obtained by fine needle aspiration, bronchial lavage, or transbronchial biopsy.

The expression level of said prognostic RNA transcript or transcripts can be determined, for example, by RT-PCR or an other PCR-based method, immunohistochemistry, proteomics techniques, or any other methods known in the art, or their combination.

In an embodiment, the assay for the measurement of said prognostic RNA transcripts or their expression products is provided is provided in the form of a kit or kits.

In another aspect, the invention concerns an array comprising polynucleotides hybridizing to a plurality of the following genes: TBP; ILT.2; ABCC5; CD18; GATA3; DICER1; MSH3; GBP1; IRS1; CD3z; fasl; TUBB; BAD; ERCC1; MCM6; PR; APC; GGPS1; KRT18; ESRRG; E2F1; AKT2; A. Catenin; CEGP1; NPD009; MAPK14; RUNX1; ID2; G.Catenin; FBXO5; FHIT; MTA1; ERBB4; FUS; BBC3; IGF1R; CD9; TP53BP1; MUC1; IGFBP5; rhoC; RALBP1; CDC20; STAT3; ERK1; HLA.DPB1; SGCB; CGA; DHPS; MGMT; CRIP2; MMP12; ErbB3; RAP1GDS1; CDC25B; IL6; CCND1; CYBA; PRKCD; DR4; Hepsin; CRABP1; AK055699; Contig.51037; VCAM1; FYN; GRB7; AKAP.2; RASSF1; MCP1; ZNF38; MCM2; GBP2; SEMA3F; CD31; COL1A1; ER2; BAG1; AKT1; COL1A2; STAT1; Wnt.5a; PTPD1; RAB6C; TK1, ErbB2, CCNB1, BIRC5, STK6, MKI67, MYBL2, MMP11, CTSL2, CD68, GSTM1, BCL2, ESR1.

In an embodiment, the array compises polynucleotides hybridizing to a plurality of the following genes: TBP; ILT.2; ABCC5; CD18; GATA3; DICER1; MSH3; GBP1; IRS1; CD3z; fas1; TUBB; BAD; ERCC1; MCM6; PR; APC; GGPS1; KRT18; ESRRG; E2F1; AKT2; A.Catenin; CEGP1; NPD009; MAPK14; RUNX1; ID2; G.Catenin; FBXO5; FHIT; MTA1; ERBB4; FUS; BBC3; IGF1R; CD9; TP53BP1; MUC1; IGFBP5; rhoC; RALBP1; CDC20; STAT3; ERK1; HLA.DPB1; SGCB; CGA; DHPS; MGMT; CRIP2; MMP12; ErbB3; RAP1GDS1; CDC25B; IL6; CCND1; CYBA; PRKCD; DR4; Hepsin; CRABP1; AK055699; Contig.51037; VCAM1; FYN; GRB7; AKAP.2; RASSF11; MCP1; ZNF38; MCM2; GBP2; SEMA3F; CD31; COL1A1; ER2; BAG1; AKT1; COL1A2; STAT1; Wnt.5a; PTPD1; RAB6C; TK1.

In another embodiment, the array comprises polynucleotides hybridizing to a plurality of the following genes: ILT.2; CD18; GBP1; CD3z; fas1; MCM6; E2F1; ID2; FBXO5; CDC20; HLA.DPB1; CGA; MMP12; CDC25B; IL6; CYBA; DR4; CRABP1; Contig.51037; VCAM1; FYN; GRB7; AKAP.2; RASSF1; MCP1; MCM2; GBP2; CD31; ER2; STAT1; TK1; ERBB2, CCNB1, BIRC5, STK6, MKI67, MYBL2, MMP11, CTSL2 and CD68.

In yet another embodiment, the array comprises polynucleotides hybridizing to a plurality of the following genes: ILT.2; CD18; GBP1; CD3z; fasl; MCM6; E2F1; ID2; FBXO5; CDC20; HLA.DPB1; CGA; MMP12; CDC25B; IL6; CYBA; DR4; CRABP1; Contig.51037; VCAM1; FYN; GRB7; AKAP.2; RASSF1; MCP1; MCM2; GBP2; CD31; ER2; STAT1; TK1

In a still further embodiment, the array comprises polynucleotides hybridizing to a plurality of the following genes: TBP; ABCC5; GATA3; DICER1; MSH3; IRS1; TUBB; BAD; ERCC1; PR; APC; GGPS1; KRT18; ESRRG; AKT2; A.Catenin; CEGP1; NPD009; MAPK14; RUNX1; G.Catenin; FHIT; MTA1; ErbB4; FUS; BBC3; IGF1R; CD9; TP53BP1; MUC1; IGFBP5; rhoC; RALBP1; STAT3; ERK1; SGCB; DHPS; MGMT; CRIP2; ErbB3; RAP1GDS1; CCND1; PRKCD; Hepsin; AK055699; ZNF38; SEMA3F; COL1A1; BAG1; AKT1; COL1A2; Wnt.5a; PTPD1; RAB6C; GSTM1, BCL2, ESR1.

In another embodiment, the array comprises polynucleotides hybridizing to a plurality of the following genes: TBP; ABCC5; GATA3; DICER1; MSH3; IRS1; TUBB; BAD; ERCC1; PR; APC; GGPS1; KRT18; ESRRG; AKT2; A.Catenin; CEGP1; NPD009; MAPK14; RUNX1; G.Catenin; FHIT; MTA1; ErbB4; FUS; BBC3; IGF1R; CD9; TP53BP1; MUC1; IGFBP5; rhoC; RALBP1; STAT3; ERK1; SGCB; DHPS; MGMT; CRIP2; ErbB3; RAP1GDS1; CCND1; PRKCD; Hepsin; AK055699; ZNF38; SEMA3F; COL1A1; BAG1; AKT1; COL1A2; Wnt.5a; PTPD1;RAB6C.

In various embodiments, the array comprises at least five, or at least 10, or at least 15, or at least 10 of such polynucleotides.

In a particular embodiment, the array comprises polynucleotides hybridizing to all of the genes listed above.

In another particular embodiment, the array comprises more than one polynucleotide hybridizing to the same gene.

In another embodiment, at least one of the the polynucleotides comprises an intron-based sequence the expression of which correlates with the expression of a corresponding exon sequence.

In various embodiments, the polynucleotides can be cDNAs or oligonucleotides.

In another aspect, the invention concerns a method of preparing a personalized genomics profile for a patient comprising the steps of:

(a) determining the normalized expression levels of the RNA transcripts or the expression products of a gene or gene set selected from the group consisting of TBP; ILT.2; ABCC5; CD18; GATA3; DICER1; MSH3; GBP1;IRS1; CD3z; fas1; TUBB; BAD; ERCC1; MCM6; PR; APC; GGPS1; KRT18; ESRRG; E2F1; AKT2; A.Catenin; CEGP1; NPD009; MAPK14; RUNX1; ID2; G.Catenin; FBXO5; FHIT; MTA1; ERBB4; FUS; BBC3; IGF1R; CD9; TP53BP1; MUC1; IGFBP5; rhoC; RALBP1; CDC20; STAT3; ERK1; HLA.DPB1; SGCB; CGA; DHPS; MGMT; CRIP2; MMP12; ErbB3; RAP1GDS1; CDC25B; IL6; CCND1; CYBA; PRKCD; DR4; Hepsin; CRABP1; AK055699; Contig.51037; VCAM1; FYN; GRB7; AKAP.2; RASSF1; MCP1; ZNF38; MCM2; GBP2; SEMA3F; CD31; COL1A1; ER2; BAG1; AKT1; COL1A2; STAT1; Wnt.5a; PTPD1; RAB6C; TK1, ErbB2, CCNB1, BIRC5, STK6, MKI67, MYBL2, MMP11, CTSL2, CD68, GSTM1, BCL2, ESR1, in a cancer cell obtained from said patient; and

(b) creating a report summarizing the data obtained by the gene expression analysis.

In a specific embodiment, if increased expression of one or more of ILT.2; CD18; GBP1; CD3z; fas1; MCM6; E2F1; ID2; FBXO5; CDC20; HLA.DPB1; CGA; MMP12; CDC25B; IL6; CYBA; DR4; CRABP1; Contig.51037; VCAM1; FYN; GRB7; AKAP.2; RASSF1; MCP1; MCM2; GBP2; CD31; ER2; STAT1; TK1; ERBB2, CCNB1, BIRC5, STK6, MKI67, MYBL2, MMP11, CTSL2 and CD68; or the corresponding expression product, is determined, the report includes a prediction that said subject has an increased likelihood of response to chemotherapy. In this case, in a particular embodiment, the method includes the additional step of treating the patient with a chemotherapeutic agent.

In the foregoing method, if increased expression of one or more of TBP; ABCC5; GATA3; DICER1; MSH3; IRS1; TUBB; BAD; ERCC1; PR; APC; GGPS1; KRT18; ESRRG; AKT2; A.Catenin; CEGP1; NPD009; MAPK14; RUNX1; G.Catenin; FHIT; MTA1; ErbB4; FUS; BBC3; IGF1R; CD9; TP53BP1; MUC1; IGFBP5; rhoC; RALBP1; STAT3; ERK1; SGCB; DHPS; MGMT; CRIP2; ErbB3; RAP1GDS1; CCND1; PRKCD; Hepsin; AK055699; ZNF38; SEMA3F; COL1A1; BAG1; AKT1; COL1A2; Wnt.5a; PTPD1; RAB6C; GSTM1, BCL2, ESR1; or the corresponding expression product, is determined, the report includes a prediction that said subject has a decreased likelihood of response to chemotherapy.

In another aspect, the invention concerns a method for determining the likelihood of the response of a patient to chemotherapy, comprising:

(a) determining the expression levels of the RNA transcripts of following genes .ACTB, BAG1, BCL2, CCNB1, CD68, SCUBE2, CTSL2, ESR1, GAPD, GRB7, GSTM1, GUSB, ERBB2, MKI67, MYBL2, PGR, RPLPO, STK6, MMP11, BIRC5, TFRC, or their expression products, and
(b) calculating the recurrence score (RS).
In an embodiment, patients having an RS > 50 are in the upper 50 percentile of patients who are likely to respond to chemotherapy.
In enother embodiment, patients having an RS < 35 are in the lower 50 percentile of patients who are likely to response to chemotherapy.
In a further embodiment, RS is determined by creating the following gene subsets:

(i) growth factor subset: GRB7 and HER2;
(ii) estrogen receptor subset: ER, PR, Bc12, and CEGP1;
(iii) proliferation subset: SURV, Ki.67, MYBL2, CCNB1, and STK15; and
(iv) invasion subset: CTSL2, and STMY3;

wherein a gene within any of subsets (i)-(iv) can be substituted by substitute gene which coexpresses with said gene in said tumor with a Pearson correlation coefficient of ≥ 0.40; and
(c) calculating the recurrence score (RS) for said subject by weighting the contributions of each of subsets (i) - (iv), to breast cancer recurrence.

The foregoing method may further comprise determining the RNA transcripts of CD68, GSTM1 and BAG1 or their expression products, or corresponding substitute genes or their expression products, and including the contribution of said genes or substitute genes to breast cancer recurrence in calculating the RS

RS may, for example, be determined by using the following equation:

$$RS = (0.23\ to\ 0.70) \times GRB7axisthresh - (0.17\ to\ 0.55) \times ERaxis + (0.52\ to\ 1.56) \times prolifaxisthresh + (0.07\ to\ 0.21) \times invasionaxis + (0.03\ to\ 0.15) \times CD68 - (0.04\ to\ 0.25) \times GSTM1 - (0.05\ to\ 0.22) \times BAG1$$

wherein

(i) GRB7 axis = (0.45 to 1.35) x GRB7 + (0.05 to 0.15) x HER2;
(ii) if GRB7 axis < -2, then GRB7 axis thresh = -2, and - if GRB7 axis ≥ -2, then GRB7 axis thresh = GRB7 axis;
(iii) ER axis = (Est1 + PR + Bc12 + CEGP1)/4;
(iv) prolifaxis = (SURV + Ki.67 + MYBL2 + CCNB1 + STK15)/5;
(v) if prolifaxis < -3.5, then prolifaxisthresh = -3.5, if prolifaxis ≥ -3.5, then prolifaxishresh = prolifaxis; and
(vi) invasionaxis = (CTSL2 + STMY3)/2,

wherein the individual contributions of the genes in (iii), (iv) and (vi) are weighted by a factor of 0.5 to 1.5, and wherein a higher RS represents an increased likelihood of breast cancer recurrence.

In another embodiment, RS is determined by using the following equation:

$$
\begin{aligned}
\text{RS (range, 0 – 100)} = \; & + 0.47 \times \text{HER2 Group Score} \\
& - 0.34 \times \text{ER Group Score} \\
& + 1.04 \times \text{Proliferation Group Score} \\
& + 0.10 \times \text{Invasion Group Score} \\
& + 0.05 \times \text{CD68} \\
& - 0.08 \times \text{GSTM1} \\
& - 0.07 \times \text{BAG1}
\end{aligned}
$$

Figure 1 shows the relationship between recurrence score (RS) and likelihood of patient response to chemotherapy, based on results from a clinical trial with pathologic complete response endpoint.

Table 1 shows a list of genes, the expression of which correlates, positively or negatively, with breast cancer response to adriamycin and taxane neoadjuvant chemotherapy. Results from a clinical trial with pathologic complete response endpoint. Statistical analysis utilized univarite generalized linear models with a probit link function.

Table 2 presents a list of genes, the expression of which predicts breast cancer response to chemotherapy. Results from a retrospective clinical trial. The table includes accession numbers for the genes, sequences for the forward and reverse primers (designated by "f" and "r", respectively) and probes (designated by "p") used for PCR amplification.

Table 3 shows the amplicon sequences used in PCR amplification of the indicated genes.

A. Definitions

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), and March, Advanced Organic Chemistry Reactions, Mechanisms and Structure 4th ed., John Wiley & Sons (New York, NY 1992), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

The term "microarray" refers to an ordered arrangement of hybridizable array elements, preferably polynucleotide probes, on a substrate.

The term "polynucleotide," when used in singular or plural, generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including in vitro recombinant DNA-mediated techniques and by expression of DNAs in cells and organisms.

The terms "differentially expressed gene," "differential gene expression" and their synonyms, which are used interchangeably, refer to a gene whose expression is activated to a higher or lower level in a subject suffering from a disease, specifically cancer, such as breast cancer, relative to its expression in a normal or control subject. The terms also include genes whose expression is activated to a higher or lower level at different stages of the same disease. It is also understood that a differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example. Differential gene expression may include a comparison of expression between two or more genes or their gene products, or a comparison of the ratios of the expression between two or more genes or their gene products, or even a comparison of two differently processed products of the same gene, which differ between normal subjects and subjects suffering from a disease, specifically cancer, or between various stages of the same disease. Differential expression includes both quantitative, as well as qualitative, differences in the temporal or cellular expression pattern in a gene or its expression products among, for example, normal and diseased cells, or among cells which have undergone different disease events or disease stages. For the purpose of this invention, "differential gene expression" is considered to be present when there is at least an about two-fold, preferably at least about four-fold, more preferably at least about six-fold, most preferably at least about ten-fold difference between the expression of a given gene in normal and diseased subjects, or in various stages of disease development in a diseased subject.

The term "normalized" with regard to a gene transcript or a gene expression product refers to the level of the transcript or gene expression product relative to the mean levels of transcripts/products of a set of reference genes, wherein the reference genes are either selected based on their minimal variation across, patients, tissues or treatments ("housekeeping genes"), or the reference genes are the totality of tested genes. In the latter case, which is commonly referred to as "global normalization", it is important that the total number of tested genes be relatively large, preferably greater than 50. Specifically, the term 'normalised' with respect to an RNA transcript refers to the transcript level relative to the mean of transcript levels of a set of reference genes. More specifically, the mean level of an RNA transcript as measured by TaqMan® RT-PCR refers to the Ct value minus the mean Ct values of a set of reference gene transcripts.

The terms "expression threshold," and "defined expression threshold" are used interchangeably and refer to the level of a gene or gene product in question above which the gene or gene product serves as a predictive marker for patient response or resistance to a drug. The threshold typically is defined experimentally from clinical studies. The expression threshold can be selected either for maximum sensitivity (for example, to detect all responders to a drug), or for maximum selectivity (for example to detect only responders to a drug), or for minimum error.

The phrase "gene amplification" refers to a process by which multiple copies of a gene or gene fragment are formed in a particular cell or cell line. The duplicated region (a stretch of amplified DNA) is often referred to as "amplicon." Often, the amount of the messenger RNA (mRNA) produced, *i.e.,* the level of gene expression, also increases in the proportion to the number of copies made of the particular gene.

The term "prognosis" is used herein to refer to the prediction of the likelihood of cancer-attributable death or progression, including recurrence, metastatic spread, and drug resistance, of a neoplastic disease, such as breast cancer. The term "prediction" is used herein to refer to the likelihood that a patient will respond either favorably or unfavorably to a drug or set of drugs, and also the extent of those responses, or that a patient will survive, following surgical removal or the primary tumor and/or chemotherapy for a certain period of time without cancer recurrence. The predictive methods of the present invention can be used clinically to make treatment decisions by choosing the most appropriate treatment modalities for any particular patient. The predictive methods of the present invention are valuable tools in predicting if a patient is likely to respond favorably to a treatment regimen, such as surgical intervention, chemotherapy with a given drug or drug combination, and/or radiation therapy, or whether long-term survival of the patient, following surgery and/or termination of chemotherapy or other treatment modalities is likely.

The term "long-term" survival is used herein to refer to survival for at least 3 years, more preferably for at least 8 years, most preferably for at least 10 years following surgery or other treatment.

The term "tumor," as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, breast cancer, colon cancer, lung cancer, prostate cancer, hepatocellular cancer, gastric cancer, pancreatic cancer, cervical cancer, ovarian cancer, liver cancer, bladder cancer, cancer of the urinary tract, thyroid cancer, renal cancer, carcinoma, melanoma, and brain cancer.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

"Patient response" can be assessed using any endpoint indicating a benefit to the patient, including, without limitation, (1) inhibition, to some extent, of tumor growth, including slowing down and complete growth arrest; (2) reduction in the number of tumor cells; (3) reduction in tumor size; (4) inhibition (i.e., reduction, slowing down or complete stopping) of tumor cell infiltration into adjacent peripheral organs and/or tissues; (5) inhibition (i.e. reduction, slowing down or complete stopping) of metastasis; (6) enhancement of anti-tumor immune response, which may, but does not have to, result in the regression or rejection of the tumor; (7) relief, to some extent, of one or more symptoms associated with the tumor; (8) increase in the length of survival following treatment; and/or (9) decreased mortality at a given point of time following treatment.

"Neoadjuvant therapy" is adjunctive or adjuvant therapy given prior to the primary (main) therapy. Neoadjuvant therapy includes, for example, chemotherapy, radiation therapy, and hormone therapy. Thus, chemotherapy may be administered prior to surgery to shrink the tumor, so that surgery can be more effective, or, in the case of previously unoperable tumors, possible.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

In the context of the present invention, reference to "at least one," "at least two," "at least five," etc. of the genes listed in any particular gene set means any one or any and all combinations of the genes listed.

## B. Detailed Description

The practice of the present invention will employ, unless otherwise indicated, conventional techniques of molecular biology (including recombinant techniques), microbiology, cell biology, and biochemistry, which are within the skill of the art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

### 1. *Gene Expression Profiling*

Methods of gene expression profiling include methods based on hybridization analysis of polynucleotides, methods based on sequencing of polynucleotides, and proteomics-based methods. The most commonly used methods known in the art for the quantification of mRNA expression in a sample include northern blotting and *in situ* hybridization (Parker & Barnes, Methods in Molecular Biology 106:247-283 (1999)); RNAse protection assays (Hod, Biotechniques 13:852-854 (1992)); and PCR-based methods, such as reverse transcription polymerase chain reaction (RT-PCR) (Weis et al., Trends in Genetics 8:263-264 (1992)). Alternatively, antibodies may be employed that can recognize specific duplexes, including DNA duplexes, RNA duplexes, and DNA-RNA hybrid duplexes or DNA-protein duplexes. Representative methods for sequencing-based gene expression analysis include Serial Analysis of Gene Expression (SAGE), and gene expression analysis by massively parallel signature sequencing (MPSS).

2. *PCR-based Gene Expression Profiling Methods*

a. *Reverse Transcriptase PCR (RT-PCR)*

One of the most sensitive and most flexible quantitative PCR-based gene expression profiling methods is RT-PCR, which can be used to compare mRNA levels in different sample populations, in normal and tumor tissues, with or without drug treatment, to characterize patterns of gene expression, to discriminate between closely related mRNAs, and to analyze RNA structure.

The first step is the isolation of mRNA from a target sample. The starting material is typically total RNA isolated from human tumors or tumor cell lines, and corresponding normal tissues or cell lines, respectively. Thus RNA can be isolated from a variety of primary tumors, including breast, lung, colon, prostate, brain, liver, kidney, pancreas, spleen, thymus, testis, ovary, uterus, etc., tumor, or tumor cell lines, with pooled DNA from healthy donors. If the source of mRNA is a primary tumor, mRNA can be extracted, for example, from frozen or archived paraffin-embedded and fixed (e.g. formalin-fixed) tissue samples.

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from tumor can be isolated, for example, by cesium chloride density gradient centrifugation.

As RNA cannot serve as a template for PCR, the first step in gene expression profiling by RT-PCR is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7700™ Sequence Detection System™ (Perkin-Elmer-Applied Biosystems, Foster City, CA, USA), or Lightcycler (Roche Molecular Biochemicals, Mannheim, Germany). In a preferred embodiment, the 5' nuclease procedure is run on a real-time quantitative PCR device such as the ABI PRISM 7700™ Sequence Detection System™. The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle ($C_t$).

To minimize errors and the effect of sample-to-sample variation, RT-PCR is usually performed using a reference RNA which ideally is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPD) and β-actin (ACTB).

A more recent variation of the RT-PCR technique is the real time quantitative PCR, which measures PCR product accumulation through a dual-labeled fluorigenic probe (i.e., TaqMan® probe). Real time PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al., Genome Research 6:986-994 (1996).

b. *MassARRAY System*

In the MassARRAY-based gene expression profiling method, developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is pre-loaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059-3064 (2003).

c. *Other PCR-based Methods*

Further PCR-based techniques include, for example, differential display (Liang and Pardee, Science 257:967-971 (1992)); amplified fragment length polymorphism (iAFLP) (Kawamoto et al., Genome Res. 12:1305-1312 (1999)); BeadArray™ technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression (BADGE), using the commercially available Luminex$^{100}$ LabMAP system and multiple color-coded microspheres (Luminex Corp., Austin, TX) in a rapid assay for gene expression (Yang et al., Genome Res. 11:1888-1898 (2001)); and high coverage expression profiling (HiCEP) analysis (Fukumura et al., Nucl. Acids. Res. 31(16) e94 (2003)).

3. *Microarrays*

Differential gene expression can also be identified, or confirmed using the microarray technique. Thus, the expression profile of breast cancer-associated genes can be measured in either fresh or paraffin-embedded tumor tissue, using microarray technology. In this method, polynucleotide sequences of interest (including cDNAs and oligonucleotides) are plated, or arrayed, on a microchip substrate. The arrayed sequences are then hybridized with specific DNA probes from cells or tissues of interest. Just as in the RT-PCR method, the source of mRNA typically is total RNA isolated from human tumors or tumor cell lines, and corresponding normal tissues or cell lines. Thus RNA can be isolated from a variety of primary tumors or tumor cell lines. If the source of mRNA is a primary tumor, mRNA can be extracted, for example, from frozen or archived paraffin-embedded and fixed (e.g. formalin-fixed) tissue samples, which are routinely prepared and preserved in everyday clinical practice.

In a specific embodiment of the microarray technique, PCR amplified inserts of cDNA clones are applied to a substrate in a dense array. Preferably at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pairwise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93(2):106-149 (1996)). Microarray analysis can be performed by commercially available equipment, following manufacturer's protocols, such as by using the Affymetrix GenChip technology, or Incyte's microarray technology.

The development of microarray methods for large-scale analysis of gene expression makes it possible to search systematically for molecular markers of cancer classification and outcome prediction in a variety of tumor types.

4. *Serial Analysis of Gene Expression (SAGE)*

Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculoscu et al., Science 270:484-487 (1995); and Velculescu et al., Cell 88:243-51 (1997).

*5. Gene Expression Analysis by Massively Parallel Signature Sequencing (MPSS)*

This method, described by Brenner et al., Nature Biotechnology 18:630-634 (2000), is a sequencing approach that combines non-gel-based signature sequencing with *in vitro* cloning of millions of templates on separate 5 μm diameter microbeads. First, a microbead library of DNA templates is constructed by *in vitro* cloning. This is followed by the assembly of a planar array of the template-containing microbeads in a flow cell at a high density (typically greater than 3 x $10^6$ microbeads/$cm^2$). The free ends of the cloned templates on each microbead are analyzed simultaneously, using a fluorescence-based signature sequencing method that does not require DNA fragment separation. This method has been shown to simultaneously and accurately provide, in a single operation, hundreds of thousands of gene signature sequences from a yeast cDNA library.

*6. Immunohistochemistry*

Immunohistochemistry methods are also suitable for detecting the expression levels of the prognostic markers of the present invention. Thus, antibodies or antisera, preferably polyclonal antisera, and most preferably monoclonal antibodies specific for each marker are used to detect expression. The 'antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

7. *Proteomics*

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N-terminal sequencing, and (3) analysis of the data using bioinformatics. Proteomics methods are valuable supplements to other methods of gene expression profiling, and can be used, alone or in combination with other methods, to detect the products of the prognostic markers of the present invention.

8. *General Description of mRNA Isolation, Purification and Amplification*

The steps of a representative protocol for profiling gene expression using fixed, paraffin-embedded tissues as the RNA source, including mRNA isolation, purification, primer extension and amplification are given in various published journal articles (for example: T.E. Godfrey et al. J. Molec. Diagnostics 2: 84-91 [2000]; K. Specht et al., Am. J. Pathol. 158: 419-29 [2001]). Briefly, a representative process starts with cutting about 10 μm thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific promoters followed by RT-PCR. Finally, the data are analyzed to identify the best treatment option(s) available to the patient on the basis of the characteristic gene expression pattern identified in the tumor sample examined.

9. *Cancer Chemotherapy*

Chemotherapeutic agents used in cancer treatment can be divided into several groups, depending on their mechanism of action. Some chemotherapeutic agents directly damage DNA and RNA. By disrupting replication of the DNA such chemotherapeutics either completely halt replication, or result in the production of nonsense DNA or RNA. This category includes, for example, cisplatin (Platinol®), daunorubicin (Cerubidine®), doxorubicin (Adriamycin®), and etoposide (VePesid®). Another group of cancer chemotherapeutic agents interfere with the formation of nucleotides or deoxyribonucleotides, so that RNA synthesis and cell replication is blocked. Examples of drugs in this class include methotrexate (Abitrexate®), mercaptopurine (Purinethol®), fluorouracil (Adrucil®), and hydroxyurea (Hydrea®). A third class of chemotherapeutic agents effects the synthesis or breakdown of mitotic spindles, and, as a result, interrupt cell division. Examples of drugs in this class include Vinblastine (Velban®), Vincristine (Oncovin®) and taxenes, such as, Pacitaxel (Taxol®), and Tocetaxel (Taxotere®) Tocetaxel is currently approved in the United States to treat patients with locally advanced or metastatic breast cancer after failure of prior chemotherapy, and patients with locally advanced or metastatic non-small cell lung cancer after failure of prior platinum-based chemotherapy.

A common problem with chemotherapy is the high toxicity of chemotherapeutic agents, such as anthracyclines and taxenes, which limits the clinical benefits of this treatment approach.

Most patients receive chemotherapy immediately following surgical removal of tumor. This approach is commonly referred to as adjuvant therapy. However, chemotherapy can be administered also before surgery, as so called neoadjuvant treatment. Although the use of neoadjuvant chemotherapy originates from the treatment of advanced and inoperable breast cancer, it has gained acceptance in the treatment of other types of cancers as well. The efficacy of neoadjuvant chemotherapy has been tested in several clinical trials. In the multi-center National Surgical Adjuvant Breast and Bowel Project B-18 (NSAB B-18) trial (Fisher et al., J. Clin. Oncology 15:2002-2004 (1997); Fisher et al., J. Clin. Oncology 16:2672-2685 (1998)) neoadjuvant therapy was performed with a combination of adriamycin and cyclophosphamide ("AC regimen"). In another clinical trial, neoadjuvant therapy was administered using a combination of 5-fluorouracil, epirubicin and cyclophosphamide ("FEC regimen") (van Der Hage et al., J. Clin. Oncol. 19:4224-4237 (2001)). Newer clinical trials have also used taxane-containing neoadjuvant treatment regiments. See, e.g. Holmes et al., J. Natl. Cancer Inst. 83:1797-1805 (1991) and Moliterni et al., Seminars in Oncology, 24:S17-10-S-17-14 (1999). For further information about neoadjuvant chemotherapy for breast cancer see, Cleator et al., Endocrine-Related Cancer 9:183-195 (2002).

10. *Cancer Gene Set, Assayed Gene Subsequences, and Clinical Application of Gene Expression Data*

An important aspect of the present invention is to use the measured expression of certain genes by breast cancer tissue to provide prognostic information. For this purpose it is necessary to correct for (normalize away) differences in the amount of RNA assayed, variability in the quality of the RNA used, and other factors, such as machine and operator differences. Therefore, the assay typically measures and incorporates the use of reference RNAs, including those transcribed from well-known housekeeping genes, such as GAPD and ACTB. A precise method for normalizing gene expression data is given in "User Bulletin #2" for the ABI PRISM 7700 Sequence Detection System (Applied Biosystems; 1997). Alternatively, normalization can be based on the mean or median signal (Ct) of all of the assayed genes or a large subset thereof (global normalization approach). In the study described in the following Example, a so called central normalization strategy was used, which utilized a subset of the screened genes, selected based on lack of correlation with clinical outcome, for normalization.

11. *Recurrence and Response to Therapy Scores and their Applications*

Copending application Serial No. 60/486,302, filed on July 10, 2003, describes an algorithm-based prognostic test for determining the likelihood of cancer recurrence and/or the likelihood that a patient responds well to a treatment modality. Features of the algorithm that distinguish it from other cancer prognostic methods include: 1) a unique set of test mRNAs (or the corresponding gene expression products) used to determine recurrence likelihood, 2) certain weights used to combine the expression data into a formula, and 3) thresholds used to divide patients into groups of different levels of risk, such as low, medium, and high risk groups. The algorithm yields a numerical recurrence score (RS) or, if patient response to treatment is assessed, response to therapy score (RTS).

The test requires a laboratory assay to measure the levels of the specified mRNAs or their expression products, but can utilize very small amounts of either fresh tissue, or frozen tissue or fixed, paraffin-embedded tumor biopsy specimens that have already been necessarily collected from patients and archived. Thus, the test can be noninvasive. It is also compatible with several different methods of tumor tissue harvest, for example, via core biopsy or fine needle aspiration.

According to the method, cancer recurrence score (RS) is determined by:

(a) subjecting a biological sample comprising cancer cells obtained from said subject to gene or protein expression profiling;
(b) quantifying the expression level of multiple individual genes [i.e., levels of mRNAs or proteins] so as to determine an expression value for each gene;
(c) creating subsets of the gene expression values, each subset comprising expression values for genes linked by a cancer-related biological function and/or by co-expression;
(d) multiplying the expression level of each gene within a subset by a coefficient reflecting its relative contribution to cancer recurrence or response to therapy within said subset and adding the products of multiplication to yield a term for said subset;
(e) multiplying the term of each subset by a factor reflecting its contribution to cancer recurrence or response to therapy; and
(f) producing the sum of terms for each subset multiplied by said factor to produce a recurrence score (RS) or a response to therapy (RTS) score,

wherein the contribution of each subset which does not show a linear correlation with cancer recurrence or response to therapy is included only above a predetermined threshold level, and
wherein the subsets in which increased expression of the specified genes reduce risk of cancer recurrence are assigned a negative value, and the subsets in which expression of the specified genes increase risk of cancer recurrence are assigned a positive value.

In a particular embodiment, RS is determined by:

(a) determining the expression levels of GRB7, HER2, EstR1, PR, Bcl2, CEGP1, SURV, Ki.67, MYBL2, CCNB1, STK15, CTSL2, STMY3, CD68, GSTM1, and BAG1, or their expression products, in a biological sample containing tumor cells obtained from said subject; and
(b) calculating the recurrence score (RS) by the following equation:

$$RS = (0.23\text{ to }0.70) \times GRB7axisthresh - (0.17\text{ to }0.51) \times ERaxis + (0.53\text{ to }1.56) \times prolifaxisthresh + (0.07\text{ to }0.21) \times invasionaxis + (0.03\text{ to }0.15) \times CD68 - (0.04\text{ to }0.25) \times GSTM1 - (0.05\text{ to }0.22) \times BAG1$$

wherein

(i) GRB7 axis = (0.45 to 1.35) x GRB7 + (0.05 to 0.15) x HER2;
(ii) if GRB7 axis < -2, then GRB7 axis thresh = -2, and if GRB7 axis ≥ -2, then GRB7 axis thresh = GRB7 axis;
(iii) ER axis = (Est1 + PR + Bcl2 + CEGP1)/4;
(iv) prolifaxis = (SURV + Ki.67 + MYBL2 + CCNB1 + STK15)/5;
(v) if prolifaxis < -3.5, then prolifaxisthresh = -3.5, if prolifaxis ≥ -3.5, then prolifaxishresh = prolifaxis; and
(vi) invasionaxis = (CTSL2 + STMY3)/2,

wherein the terms for all individual genes for which ranges are not specifically shown can vary between about 0.5 and 1.5, and wherein a higher RS represents an increased likelihood of cancer recurrence.

Further details of the invention will be described in the following non-limiting Example.

Example

A Retrospective Study of Neoadjuvant Chemotherapy in Invasive Breast Cancer: Gene Expression Profiling of Paraffin-Embodded Core Biopsy Tissue

This was a collaborative study involving Genomic Health, Inc., (Redwood City California), and Institute Tumori, Milan, Italy. The primary objective of the study was to explore the correlation between pre-treatment molecular profiles and pathologic complete response (pCR) to neoadjuvant chemotherapy in locally advanced breast cancer.

*Patient inclusion criteria:*

Histologic diagnosis of invasive breast cancer (date of surgery 1998-2002); diagnosis of locally advanced breast cancer defined by skin infiltration and or N2 axillary status and or homolateral supraclavicular positive nodes; core biopsy, neoadjuvant chemotherapy and surgical resection performed at Istituto Nazionale Tumori, Milan; signed informed consent that the biological material obtained for histological diagnosis or diagnostic procedures would be used for research; and histopathologic assessment indicating adequate amounts of tumor tissue for inclusion in this research study.

*Exclusion criteria:*

Distant metastases; no tumor block available from initial core biopsy or from the surgical resection; or no tumor or very little tumor (<5% of the overall tissue on the slide) in block as assessed by examination of the H&E slide by the Pathologist.

*Study design*

Eighty-nine evaluable patients (from a set of 96 clinically evaluable patients) were identified and studied. The levels of 384 mRNA species were measured by RT-PCR, representing products of candidate cancer-related genes that were selected from the biomedical research literature. Only one gene was lost due to inadequate signal.

Patient characteristics were as follows: Mean age: 50 years; Tumor grades: 24% Well, 55% Moderate, and 21% Poor; Sixty-three % of patients were ER positive {by immunohistochemistry}; Seventy % of patients had positive lymph nodes.

All patients were given primary neoadjuvant chemotherapy: Doxorubicin plus Taxol 3weeks/3 cycles followed by Taxol® (paclitaxel) 1week/12 cycles. Surgical removal of the tumor followed completion of chemotherapy. Core tumor biopsy specimens were taken prior to start of chemotherapy, and served as the source of RNA for the RT-PCR assay.

*Materials and Methods*

Fixed paraffin-embedded (FPE) tumor tissue from biopsy was obtained prior to and after chemotherapy. Core biopsies were taken prior to chemotherapy. In that case, the pathologist selected the most representative primary tumor block, and submitted nine 10 micron sections for RNA analysis. Specifically, a total of 9 sections (10 microns in thickness each) were prepared and placed in three Costar Brand Microcentrifuge Tubes (Polypropylene, 1.7 mL tubes, clear; 3 sections in each tube) and pooled.

Messenger RNA was extracted using the *MasterPure™ RNA Purification Kit* (Epicentre Technologies) and quantified by the RiboGreen® fluorescence method (Molecular probes). Molecular assays of quantitative gene expression were performed by RT-PGR, using the ABI PRISM 7900™ Sequence Detection System™ (Perkin-Elmer-Applied Biosystems, Foster City, CA, USA). ABI PRISM 7900™ consists of a thermocycler, laser, charge-coupled device (CCD), camera and computer. The system amplifies samples in a 384-well format on a thermocycler. During amplification, laser-induced fluorescent signal is collected in real-time for all 384 wells, and detected at the CCD. The system includes software for running the instrument and for analyzing the data.

*Analysis and Results*

Tumor tissue was analyzed for 384 genes. The threshold cycle ($C_T$) values for each patient were normalized based on the median of a subset of the screened genes for that particular patient, selected based on lack of correlation with clinical outcome (central normalization strategy). Patient beneficial response to chemotherapy was defined as pathologic complete response (pCR). Patients were formally assessed for response at the completion of all chemotherapy.

A clinical complete response (cCR) requires complete disappearance of all clinically detectable disease, either by physical examination or diagnostic breast imaging.

A pathologic complete response (pCR) requires absence of residual breast cancer on histologic examination of biopsied breast tissue, lumpectomy or mastectomy specimens following primary chemotherapy. Residual ductal carcinoma *in situ* (DCIS) may be present. Residual cancer in regional nodes may not be present. Of the 89 evaluable patients 11 (12%) had a pathologic complete response (pCR). Seven of these patients were ER negative.

A partial clinical response was defined as a ≥ 50% decrease in tumor area (sum of the products of the longest perpendicular diameters) or a ≥ 50% decrease in the sum of the products of the longest perpendicular diameters of multiple lesions in the breast and axilla. No area of disease may increase by > 25% and no new lesions may appear.

Analysis was performed by comparing the relationship between normalized gene expression and the binary outcomes of pCR or no pCR. Univariate generalized models were used with probit or logit link functions. See, e.g. Van K. Borooah, LOGIT and PROBIT, Ordered Multinominal Models, Sage University Paper, 2002.

Table 1 presents pathologic response correlations with gene expression, and lists the 86 genes for which the p-value for the differences between the groups was <0.1. The second column (with the heading "Direction") denotes whether increased expression correlates with decreasing or increasing likelihood of response to chemotherapy. The statistical significance of the predictive value for each gene is given by P-value (right hand column)

| | | Probit Link | | |
|---|---|---|---|---|
| **Gene** | **Direction** | **Intercept** | **Slope** | **P-value** |
| TBP | Decreasing | 0.0575 | 2.4354 | 0.0000 |
| ILT.2 | Increasing | 0.5273 | -0.9489 | 0.0003 |
| ABCC5 | Decreasing | 0.9872 | 0.8181 | 0.0003 |
| CD18 | Increasing | 3.4735 | -1.0787 | 0.0007 |
| GATA3 | Decreasing | 0.6175 | 0.2975 | 0.0008 |
| DICER1 | Decreasing | -0.9149 | 1.4875 | 0.0013 |
| MSH3 | Decreasing | 2.6875 | 0.9270 | 0.0013 |
| GBP1 | Increasing | 1.7649 | -0.5410 | 0.0014 |
| IRS1 | Decreasing | 1.3576 | 0.5214 | 0.0016 |
| CD3z | Increasing | 0.1567 | -0.5162 | 0.0018 |
| Fasl | Increasing | -0.6351 | -0.4050 | 0.0019 |
| TUBB | Decreasing | 1.2745 | 0.8267 | 0.0026 |
| BAD | Decreasing | 0.9993 | 1.1325 | 0.0033 |
| ERCC1 | Decreasing | 0.0327 | 1.0784 | 0.0039 |
| MCM6 | Increasing | 0.1371 | -0.8008 | 0.0052 |
| PR | Decreasing | 1.6079 | 0.1764 | 0.0054 |
| APC | Decreasing | 0.7264 | 1.0972 | 0.0061 |
| GGPS1 | Decreasing | 1.0906 | 0.8124 | 0.0062 |
| KRT18 | Decreasing | -0.8029 | 0.4506 | 0.0063 |
| ESRRG | Decreasing | 2.0198 | 0.2262 | 0.0063 |
| E2F1 | Increasing | 0.2188 | -0.5277 | 0.0068 |
| AKT2 | Decreasing | -1.3566 | 1.1902 | 0.0074 |
| A.Catenin | Decreasing | -0.6859 | 0.9279 | 0.0079 |
| CEGP1 | Decreasing | 1.3355 | 0.1875 | 0.0091 |
| NPD009 | Decreasing | 1.3996 | 0.2971 | 0.0092 |
| MAPK14 | Decreasing | 2.6253 | 1.6007 | 0.0093 |
| RUNX1 | Decreasing | -0.4138 | 0.7214 | 0.0103 |
| ID2 | Increasing | 1.7326 | -0.7032 | 0.0104 |
| G.Catenin | Decreasing | -0.1221 | 0.5954 | 0.0110 |
| FBXO5 | Increasing | 0.3421 | -0.4935 | 0.0110 |
| FHIT | Decreasing | 1.9966 | 0.4989 | 0.0113 |
| MTA1 | Decreasing | 0.3127 | 0.6069 | 0.0133 |
| ERBB4 | Decreasing | 1.4591 | 0.1436 | 0.0135 |
| FUS | Decreasing | -0.6150 | 0.9415 | 0.0137 |
| BBC3 | Decreasing | 2.4796 | 0.6495 | 0.0138 |
| IGF1R | Decreasing | 1.1998 | 0.3116 | 0.0147 |
| CD9 | Decreasing | -0.9292 | 0.5747 | 0.0156 |
| TP53BP1 | Decreasing | 1.4325 | 0.8122 | 0.0169 |
| MUC1 | Decreasing | 0.8881 | 0.2140 | 0.0175 |
| IGFBP5 | Decreasing | -0.6180 | 0.4880 | 0.0181 |
| rhoC | Decreasing | -0.1726 | 0.6860 | 0.0184 |
| RALBP1 | Decreasing | 0.2383 | 0.9509 | 0.0185 |
| CDC20 | Increasing | 1.3204 | -0.4390 | 0.0186 |

(continued)

| | | Probit Link | | |
|---|---|---|---|---|
| Gene | Direction | Intercept | Slope | P-value |
| STAT3 | Decreasing | -0.9763 | 0.7023 | 0.0194 |
| ERK1 | Decreasing | 0.8577 | 0.6496 | 0.0198 |
| HLA.DPB1 | Increasing | 3.6300 | -0.6035 | 0.0202 |
| SGCB | Decreasing | 0.6171 | 0.7823 | 0.0208 |
| CGA | Increasing | 0.0168 | -0.1450 | 0.0209 |
| DHPS | Decreasing | 0.2957 | 0.7840 | 0.0216 |
| MGMT | Decreasing | 0.9238 | 0.6876 | 0.0226 |
| CRIP2 | Decreasing | 0.5524 | 0.4394 | 0.0230 |
| MMP12 | Increasing | 0.4208 | -0.2419 | 0.0231 |
| ErbB3 | Decreasing | 0.9438 | 0.2798 | 0.0233 |
| RAP1GDS1 | Decreasing | 0.2617 | 0.7672 | 0.0235 |
| CDC25B | Increasing | 1.6965 | -0.5356 | 0.0264 |
| IL6 | Increasing | 0.0592 | -0.2388 | 0.0272 |
| CCND1 | Decreasing | 0.2260 | 0.2992 | 0.0272 |
| CYBA | Increasing | 2.6493 | -0.5175 | 0.0287 |
| PRKCD | Decreasing | 0.2125 | 0.6745 | 0.0291 |
| DR4 | Increasing | 0.3039 | -0.5321 | 0.0316 |
| Hepsin | Decreasing | 1.9211 | 0.1873 | 0.0318 |
| CRABP1 | Increasing | 1.0309 | -0.1287 | 0.0320 |
| AK055699 | Decreasing | 2.0442 | 0.1765 | 0.0343 |
| Contig.51037 | Increasing | 0.7857 | -0.1131 | 0.0346 |
| VCAM1 | Increasing | 1.1866 | -0.3560 | 0.0346 |
| FYN | Increasing | 1.5502 | -0.5624 | 0.0359 |
| GRB7 | Increasing | 1.3592 | -0.1646 | 0.0375 |
| AKAP.2 | Increasing | 1.7946 | -0.7008 | 0.0382 |
| RASSF1 | Increasing | 1.1972 | -0.0390 | 0.0384 |
| MCP1 | Increasing | 1.3700 | -0.3805 | 0.0388 |
| ZNF38 | Decreasing | 1.7957 | 0.4993 | 0.0395 |
| MCM2 | Increasing | 1.0574 | -0.4695 | 0.0426 |
| GBP2 | Increasing | 1.4095 | -0.4559 | 0.0439 |
| SEMA3F | Decreasing | 1.2706 | 0.3725 | 0.0455 |
| CD31 | Increasing | 1.9913 | -0.5955 | 0.0459 |
| COL1A1 | Decreasing | -1.9861 | 0.3812 | 0.0466 |
| ER2 | Increasing | -0.5204 | -0.2617 | 0.0471 |
| BAG1 | Decreasing | 0.6731 | 0.5070 | 0.0472 |
| AKT1 | Decreasing | -0.4467 | 0.5768 | 0.0480 |
| COL1A2 | Decreasing | -1.0233 | 0.3804 | 0.0490 |
| STAT1 | Increasing | 1.9447 | -0.4062 | 0.0498 |
| Wnt.5a | Decreasing | 2.2244 | 0.2983 | 0.0518 |
| PTPD1 | Decreasing | 1.2950 | 0.4834 | 0.0552 |
| RAB6C | Decreasing | 0.4841 | 0.5635 | 0.0717 |
| TK1 | Increasing | 0.6127 | -0.3625 | 0.0886 |
| Bcl2 | Decreasing | 1.1459 | 0.2509 | 0.0959 |

Based on the data set forth in Table 1, increased expression of the following genes correlates with increased likelihood of complete pathologic response to treatment: ILT.2; CD18; GBP1; CD3z; fasl; MCM6; E2F1; ID2; FBXO5; CDC20; HLA.DPB1; CGA; MMP12; CDC25B; IL6; CYBA; DR4; CRABP1; Contig.51037; VCAM1; FYN; GRB7; AKAP.2; RASSF1; MCP1; MCM2; GBP2; CD31; ER2; STAT1; TK1; while increased expression of the following genes correlates with decreased likelihood of complete pathologic response to treatment: TBP; ABCC5; GATA3; DICER1; MSH3; IRS1; TUBB; BAD; BRCC1; PR; APC; GGPS1; KRT18; ESRRG; AKT2; A.Catenin; CEGP1; NPD009; MAPK14; RUNX1; G.Catenin; FHIT; MTA1; ErbB4; FUS; BBC3; IGF1R; CD9; TP53BP1; MUC1; IGFBP5; rhoC; RALBP1; STAT3; ERK1; SGCB; DHPS; MGMT; CRIP2; ErbB3; RAP1GDS1; CCND1; PRKCD; Hepsin; AK055699; ZNF38; SEMA3F; COL1A1; BAG1; AKT1; COL1A2; Wnt.5a; PTPD1; RAB6C; Bcl2.

The relationship between the recurrence risk algorithm (described in copending U.S. application Serial No. 60/486,302) and pCR was also investigated. The algorithm incorporates the measured levels of 21 mRNA species. Sixteen mRNAs (named below) were candidate clinical markers and the remaining 5 (ACTB, GAPD, GUSB, RPLPO, and TFRC) were reference genes. Reference-normalized expression measurements range from 0 to 15, where a one unit increase reflects a 2-fold increase in RNA.

The Recurrence Score (RS) is calculated from the quantitative expression of four sets of marker genes (an estrogen receptor group of 4 genes-ESR1, PGR, BCL2, and SCUBE2; a proliferation set of 5 genes-MKI67, MYBL2, BIRC5, CCNB1, and STK6; a HER2 set of 2 genes-ERBB2 and GRB7, an invasion group of 2 genes-MMP11 and CTSL2) and 3 other individual genes-GSTM1, BAG1, and CD68.

Although the genes and the multiplication factors used in the equation may vary, in a typical embodiment, the following equation may be used to calculate RS:

$$\begin{aligned}\text{RS (range, 0 – 100)} = &+ 0.47 \times \text{HER2 Group Score}\\ &- 0.34 \times \text{ER Group Score}\\ &+ 1.04 \times \text{Proliferation Group Score}\\ &+ 0.10 \times \text{Invasion Group Score}\\ &+ 0.05 \times \text{CD68}\\ &- 0.08 \times \text{GSTM1}\\ &- 0.07 \times \text{BAG1}\end{aligned}$$

Application of this algorithm to study clinical and gene expression data sets yields a continuous curve relating RS to pCR values, as shown in Figure 1. Examination of these data shows that patients with RS > 50 are in the upper 50 percentile of patients in terms of likelihood of response to chemotherapy, and that patients with RS < 35 are in the lower 50 percentile of patients in terms of likelihood of response to chemotherapy.

While the invention has been described with emphasis upon certain specific embodiments, it is be apparent to those skilled in the art that variations and modification in the specific methods and techniques are possible. Accordingly, this invention includes all modifications encompassed within the spirit and scope of the invention as defined by the following claims.

**TABLE 2**

| | | | | |
|---|---|---|---|---|
| A-Catenin | MM_00190 | S2138/A-Cate.f2 | CGTTCCGATCCTCTATACTGCAT | 23 |
| A-Catenin | NM_00190 | S2139/A-Cate.r2 | AGGTCCCTGTTGGCCTTATAGG | 22 |
| A-Catenin | NM_00190 | S4725/A-Cate.p2 | ATGCCTACAGCACCCTGATGTCGCA | 25 |
| ABCC5 | NM_00568 | S5605/ABCC5.f1 | TGCAGACTGTACCATGCTGA | 20 |
| ABCC5 | NM_00568 | S5806/ABCC5.r1 | GGCCAGCACCATAATCCTAT | 20 |
| ABCC5 | NM_00588 | S5607/ABCC5.p1 | CTGCACACGGTTCTAGGCTCCG | 22 |
| AK055699 | AK055699 | S2097/AK0556.f1 | CTGCATGTGATTGAATAAGAAACAAGA | 27 |
| AK055699 | AK055699 | S2098/AK0556.r1 | TGTGGACCTGATCCCTGTACAC | 22 |
| AK055699 | AK055699 | S5057/AK0556.p1 | TGACCACACCAAAGCCTCCCTGG | 23 |
| AKAP-2 | NM_00720 | S1374/AKAP-2.f1 | ACGAATTGTCGGTGAGGTCT | 20 |
| AKAP-2 | NM_00720 | S1375/AKAP-2.r1 | GTCCATGCTGAAATCATTGG | 20 |
| AKAP-2 | NM_00720 | S4934/AKAP-2.p1 | CAGGATACCACAGTCCTGGAGACCC | 25 |
| AKT1 | NM_00516 | S0010/AKT1.f3 | CGCTTCTATGGCGCTGAGAT | 20 |
| AKT1 | NM_00516 | S0012/AKT1.r3 | TCCCGGTACACCACGTTCTT | 20 |
| AKT1 | NM_00516 | S4776/AKT1.p3 | CAGCCCTGGACTACCTGCACTCGG | 24 |
| AKT2 | NM_00162 | S0828/AKT2.f3 | TCCTGCCACCCTTCAAACC | 19 |

(continued)

| | | | | |
|---|---|---|---|---|
| AKT2 | NM_00162 | S0829/AKT2.r3 | GGCGGTAAATTCATCATCGAA | 21 |
| AKT2 | NM_00162 | S4727/AKT2.p3 | CAGGTCACGTCCGAGGTCGACACA | 24 |
| APC | NM_00003 | S0022/APC.f4 | GGAGAGCAGGAATGTGTTTC | 20 |
| APC | NM_00003 | S0024/APC.r4 | ACCCACTCGATTTGTTTCTG | 20 |
| APC | NM_00003 | S4888/APC.p4 | CATTGGCTCCCCGTGACCTGTA | 22 |
| BAD | NM_03298 | S2011/BAD.f1 | GGGTCAGGTGCCTCGAGAT | 19 |
| BAD | NM_03298 | S2012/BAD.r1 | CTGCTCACTCGGCTCAAACTC | 21 |
| BAD | NM_03298 | S5058/BAD.p1 | TGGGCCCAGAGCATGTTCCAGATC | 24 |
| BAG1 | NM_00432 | S1386/BAG1.f2 | CGTTGTCAGCACTTGGAATACAA | 23 |
| BAG1 | NM_00432 | S1387/BAG1.r2 | GTTCAACCTCTTCCTGTGGACTGT | 24 |
| BAG1 | NM_00432 | S4731/BAG1.p2 | CCCAATTAACATGACCCGGCAACCAT | 26 |
| BBC3 | NM_01441 | S1584/BBC3.f2 | CCTGGAGGGTCCTGTACAAT | 20 |
| BBC3 | NM_01441 | S1585/BBC3.r2 | CTAATTGGGCTCCATCTCG | 19 |
| BBC3 | NM_01441 | S4890/BBC3.p2 | CATCATGGGACTCCTGCCCTTACC | 24 |
| Bcl2 | NM_00063 | S0043/Bcl2.f2 | CAGATGGACCTAGTACCCACTGAGA | 25 |
| Bcl2 | NM_00063 | S0045/Bcl2.r2 | CCTATGATTTAAGGGCATTTTTCC | 24 |
| Bcl2 | NM_00063 | S4732/Bcl2.p2 | TTCCACGCCGAAGGACAGCGAT | 22 |
| CCND1 | NM_00175 | S0058/CCND1.f3 | GCATGTTCGTGGCCTCTAAGA | 21 |
| CCND1 | NM_00175 | S0060/CCND1.r3 | CGGTGTAGATGCACAGCTTCTC | 22 |
| CCND1 | NM_00175 | S4986/CCND1.p3 | AAGGAGACCATCCCCCTGACGGC | 23 |
| CD18 | NM_00021 | S0061/CD18.f2 | CGTCAGGACCCACCATGTCT | 20 |
| CD18 | NM_00021 | S0063/CD18.r2 | GGTTAATTGGTGACATCCTCAAGA | 24 |
| CD18 | NM_00021 | S4987/CD18.p2 | CGCGGCCGAGACATGGCTTG | 20 |
| CD31 | NM_00044 | S1407/CD31.f3 | TGTATTTCAAGACCTCTGTGCACTT | 25 |
| CD31 | NM_00044 | S1408/CD31.r3 | TTAGCCTGAGGAATTGCTGTGTT | 23 |
| CD31 | NM_00044 | S4939/CD31.p3 | TTTATGAACCTGCCCTGCTCCCACA | 25 |
| CD3z | NM_00073 | S0064/CD3z.f1 | AGATGAAGTGGAAGGCGCTT | 20 |
| CD3z | NM_00073 | S0066/CD3z.r1 | TGCCTCTGTAATCGGCAACTG | 21 |
| CD3z | NM_00073 | S4988/CD3z.p1 | CACCGCGGCCATCCTGCA | 18 |
| CD9 | NM_00176 | S0686/CD9.f1 | GGGCGTGGAACAGTTTATCT | 20 |
| CD9 | NM_00176 | S0687/CD9.r1 | CACGGTGAAGGTTTCGAGT | 19 |
| CD9 | NM_00176 | S4792/CD9.p1 | AGACATCTGCCCCAAGAAGGACGT | 24 |
| CDC20 | NM_00125 | S4447/CDC20.f1 | TGGATTGGAGTTCTGGGAATG | 21 |
| CDC20 | NM_00125 | S4448/CDC20.r1 | GCTTGCACTCCACAGGTACACA | 22 |
| CDC20 | NM_00125 | S4449/CDC20.p1 | ACTGGCCGTGGCACTGGACAACA | 23 |
| CDC25B | NM_02187 | S1160/CDC25B.f1 | AAACGAGCAGTTTGCCATCAG | 21 |
| CDC25B | NM_02187 | S1161/CDC25B.r1 | GTTGGTGATGTTCCGAAGCA | 20 |
| CDC25B | NM_02187 | S4842/CDC25B.p1 | CCTCACCGGCATAGACTGGAAGCG | 24 |
| CEGP1 | NM_02097 | S1494/CEGP1.f2 | TGACAATCAGCACACCTGCAT | 21 |
| CEGP1 | NM_02097 | S1495/CEGP1.r2 | TGTGACTACAGCCGTGATCCTTA | 23 |
| CEGP1 | NM_02097 | S4735/CEGP1.p2 | CAGGCCCTCTTCCGAGCGGT | 20 |
| CGA (CHG) | NM_00127 | S3221/CGA (C.f3 | CTGAAGGAGCTCCAAGACCT | 20 |
| CGA (CHG) | NM_00127 | S3222/CGA (C.r3 | CAAAACCGCTGTGTTTCTTC | 20 |
| CGA (CHG) | NM_00127 | S3254/CGA (C.p3 | TGCTGATGTGCCCTCTCCTTGG | 22 |
| COL1A1 | NM_00008 | S4531/COL1A1.f1 | GTGGCCATCCAGCTGACC | 18 |
| COL1A1 | NM_00008 | S4532/COL1A1.r1 | CAGTGGTAGGTGATGTTCTGGGA | 23 |
| COL1A1 | NM_00008 | S4533/COL1A1.p1 | TCCTGCGCCTGATGTCCACCG | 21 |
| COL1A2 | NM_00008 | S4534/COL1A2.f1 | CAGCCAAGAACTGGTATAGGAGCT | 24 |
| COL1A2 | NM_00008 | S4535/COL1A2.r1 | AAACTGGCTGCCAGCATTG | 19 |
| COL1A2 | NM_00008 | S4536/COL1A2.p1 | TCTCCTAGCCAGACGTGTTTCTTGTCCTTG | 30 |
| Contig 510 | XM_05894 | S2070/Contig.r1 | CGACAGTTGCGATGAAAGTTCTAA | 24 |

(continued)

| | | | | |
|---|---|---|---|---|
| Contig 510 | XM_05894 | S2071/Contig.r1 | GGCTGCTAGAGACCATGGACAT | 22 |
| Contig 510 | XM_05894 | S5059/Contig.p1 | CCTCCTCCTGTTGCTGCCACTAATGCT | 27 |
| CRABP1 | NM_00437 | S5441/CRABP1.f3 | AACTTCAAGGTCGGAGAAGG | 20 |
| CRABP1 | NM_00437 | S5442/CRABP1.r3 | TGGCTAAACTCCTGCACTTG | 20 |
| CRABP1 | NM_00437 | S5443/CRABP1.p3 | CCGTCCACGGTCTCCTCCTCA | 21 |
| CRIP2 | NM_00131 | S5676/CRIP2.f3 | GTGCTACGCCACCCTGTT | 18 |
| CRIP2 | NM_00131 | S5677/CRIP2.r3 | CAGGGGCTTCTCGTAGATGT | 20 |
| CRIP2 | NM_00131 | S5678/CRIP2.p3 | CCGATGTTCACGCCTTTGGGTC | 22 |
| CYBA | NM_00010 | S5300/CYBA.f1 | GGTGCCTACTCCATTGTGG | 19 |
| CYBA | NM_00010 | S5301/CYBA.r1 | GTGGAGCCCTTCTTCCTCTT | 20 |
| CYBA | NM_00010 | S5302/CYBA.p1 | TACTCCAGCAGGCACACAAACACG | 24 |
| DHPS | NM_01340 | S4519/DHPS.f3 | GGGAGAACGGGATCAATAGGAT | 22 |
| DHPS | NM_01340 | S4520/DHPS.r3 | GCATCAGCCAGTCCTCAAACT | 21 |
| DHPS | NM_01340 | S4521/DHPS.p3 | CTCATTGGGCACCAGCAGGTTTCC | 24 |
| DICER1 | NM_17743 | S5294/DICER1.f2 | TCCAATTCCAGCATCACTGT | 20 |
| DICER1 | NM_17743 | S5295/DICER1.r2 | GGCAGTGAAGGCGATAAAGT | 20 |
| DICER1 | NM_17743 | S5296/DICER1.p2 | AGAAAAGCTGTTTGTCTCCCCAGCA | 25 |
| DR4 | NM_00384 | S2532/DR4.f2 | TGCACAGAGGGTGTGGGTTAC | 21 |
| DR4 | NM_00384 | S2533/DR4.r2 | TCTTCATCTGATTTACAAGCTGTACATG | 28 |
| DR4 | NM_00384 | S4981/DR4.p2 | CAATGCTTCCAACAATTTGTTTGCTTGCC | 29 |
| E2F1 | NM_00522 | S3063/E2F1.f3 | ACTCCCTCTACCCTTGAGCA | 20 |
| E2F1 | NM_00522 | S3064/E2F1.r3 | CAGGCCTCAGTTCCTTCAGT | 20 |
| E2F1 | NM_00522 | S4821/E2F1.p3 | CAGAAGAACAGCTCAGGGACCCCT | 24 |
| ER2 | NM_00143 | S0109/ER2.f2 | TGGTCCATCGCCAGTTATCA | 20 |
| ER2 | NM_00143 | S0111/ER2.r2 | TGTTCTAGCGATCTTGCTTCACA | 23 |
| ER2 | NM_00143 | S5001/ER2.p2 | ATCTGTATGCGGAACCTCAAAAGAGTCCCT | 30 |
| ErbB3 | NM_00198 | S0112/ErbB3.f1 | CGGTTATGTCATGCCAGATACAC | 23 |
| ErbB3 | NM_00198 | S0114/ErbB3.r1 | GAACTGAGACCCACTGAAGAAAGG | 24 |
| ErbB3 | NM_00198 | S5002/Erb83.p1 | CCTCAAAGGTACTCCCTCCTCCCGG | 25 |
| ERBB4 | NM_00523 | S1231/ERBB4.f3 | TGGCTCTTAATCAGTTTCGTTACCT | 25 |
| ERBB4 | NM_00523 | S1232/ERBB4.r3 | CAAGGCATATCGATCCTCATAAAGT | 25 |
| ERBB4 | NM_00523 | S4891/ERBB4.p3 | TGTCCCACGAATAATGCGTAAATTCTCCAG | 30 |
| ERCC1 | NM_00198 | S2437/ERCC1.f2 | GTCCAGGTGGATGTGAAAGA | 20 |
| ERCC1 | NM_00198 | S2438/ERCC1.r2 | CGGCCAGGATACACATCTTA | 20 |
| ERCC1 | NM_00198 | S4920/ERCC1.p2 | CAGCAGGCCCTCAAGGAGCTG | 21 |
| ERK1 | Z11696 | S1560/ERK1.f3 | ACGGATCACAGTGGAGGAAG | 20 |
| ERK1 | Z11696 | S1561/ERK1.r3 | CTCATCCGTCGGGTCATAGT | 20 |
| ERK1 | Z11696 | S4882/ERK1.p3 | CGCTGGCTCACCCCTACCTG | 20 |
| ESRRG | NM_00143 | S6130/ESRRG.f3 | CCAGCACCATTGTTGAAGAT | 20 |
| ESRRG | M_00143 | S6131/ESRRG.r3 | AGTCTCTTGGGCATCGAGTT | 20 |
| ESRRG | NM_00143 | S6132/ESRRG.p3 | CCCCAGACCAAGTGTGAATACATGCT | 26 |
| fasl | NM_00063 | S0121/fasl.f2 | GCACTTTGGGATTCTTTCCATTAT | 24 |
| fasl | NM_00063 | S0123/fasl.r2 | GCATGTAAGAAGACCCTCACTGAA | 24 |
| fasl | NM_00063 | S5004/fasl.p2 | ACAACATTCTCGGTGCCTGTAACAAAGAA | 29 |
| FBXO5 | NM_01217 | S2017/FBXO5.r1 | GGATTGTAGACTGTCACCGAAATTC | 25 |
| FBXO5 | NM_01217 | S2018/FBXO5.f1 | GGCTATTCCTCATTTTCTCTACAAAGTG | 28 |
| FBXO5 | NM_01217 | S5061/FBXO5.p1 | CCTCCAGGAGGCTACCTTCTTCATGTTCAC | 30 |
| FHIT | NM_00201 | S2443/FHIT.f1 | CCAGTGGAGCGCTTCCAT | 18 |
| FHIT | NM_00201 | S2444/FHIT.r1 | CTCTCTGGGTCGTCTGAAACAA | 22 |
| FHIT | NM_00201 | S4921/FHIT.p1 | TCGGCCACTTCATCAGGACGCAG | 23 |
| FUS | NM_00496 | S2936/FUS.f1 | GGATAATTCAGACAACAACACCATCT | 26 |

(continued)

| | | | | |
|---|---|---|---|---|
| FUS | NM_00496 | S2937/FUS.r1 | TGAAGTAATCAGCCACAGACTCAAT | 25 |
| FUS | NM_00496 | S4801/FUS.p1 | TCAATTGTAACATTCTCACCCAGGCCTTG | 29 |
| FYN | NM_00203 | S5695/FYN.f3 | GAAGCGCAGATCATGAAGAA | 20 |
| FYN | NM_00203 | S5696/FYN.r3 | CTCCTCAGACACCACTGCAT | 20 |
| FYN | NM_00203 | S5697/FYN.p3 | CTGAAGCACGACAAGCTGGTCCAG | 24 |
| G-Catenin | NM_00223 | S2153/G-Cate.f1 | TCAGCAGCAAGGGCATCAT | 19 |
| G-Catenin | NM_00223 | S2154/G-Cate.r1 | GGTGGTTTTCTTGAGCGTGTACT | 23 |
| G-Catenin | NM_00223 | S5044/G-Cate.p1 | CGCCCGCAGGCCTCATCCT | 19 |
| GATA3 | NM_00205 | S0127/GATA3.f3 | CAAAGGAGCTCACTGTGGTGTCT | 23 |
| GATA3 | NM_00205 | S0129/GATA3.r3 | GAGTCAGAATGGCTTATTCACAGATG | 26 |
| GATA3 | NM_00205 | S5005/GATA3.p3 | TGTTCCAACCACTGAATCTGGACC | 24 |
| GBP1 | NM_00206 | S5698/GBP1.f1 | TTGGGAAATATTTGGGCATT | 20 |
| GBP1 | NM_00205 | S5699/GBP1.r1 | AGAAGCTAGGGTGGTTGTCC | 20 |
| GBP1 | NM_00205 | S5700/GBP1.p1 | TTGGGACATTGTAGACTTGGCCAGAC | 26 |
| GBP2 | NM_00412 | S5707/GBP2.f2 | GCATGGGAACCATCAACCA | 19 |
| GBP2 | NM_00412 | S5708/GBP2.r2 | TGAGGAGTTTGCCTTGATTCG | 21 |
| GBP2 | NM_00412 | S5709/GBP2.p2 | CCATGGACCAACTTCACTATGTGACAGAGC | 30 |
| GGPS1 | NM_00483 | S1590/GGPS1.f1 | CTCCGACGTGGCTTTCCA | 18 |
| GGPS1 | NM_00483 | S1591/GGPS1.r1 | CGTAATTGGCAGAATTGATGACA | 23 |
| GGPS1 | NM_00483 | S4896/GGPS1.p1 | TGGCCCACAGCATCTATGGAATCCC | 25 |
| GRB7 | NM_00531 | S0130/GRB7.f2 | CCATCTGCATCCATCTTGTT | 20 |
| GRB7 | NM_00531 | S0132/GRB7.r2 | 'GGCCACCAGGGTATTATCTG | 20 |
| GRB7 | NM_00531 | S4726/GRB7.p2 | CTCCCCACCCTTGAGAAGTGCCT | 23 |
| Hepsin | NM_00215 | S2269/Hepsin.f1 | AGGCTGCTGGAGGTCATCTC | 20 |
| Hepsin | NM_00215 | S2270/Hepsin.r1 | CTTCCTGCGGCCACAGTCT | 19 |
| Hepsin | NM_00215 | S2271/Hepsin.p1 | CCAGAGGCCGTTTCTTGGCCG | 21 |
| HLA-DPB1 | NM_00212 | S4573/HLA-DP.f1 | TCCATGATGGTTCTGCAGGTT | 21 |
| HLA-DPB1 | NM_00212 | S4574/HLA-DP.r1 | TGAGCAGCACCATCAGTAACG | 21 |
| HLA-DPB1 | NM_00212 | S4575/HLA-DP.p1 | CCCCGGACAGTGGCTCTGACG | 21 |
| ID2 | NM_00216 | S0151/ID2.f4 | AACGACTGCTACTCCAAGCTCAA | 23 |
| ID2 | NM_00216 | S0153/ID2.r4 | GGATTTCCATCTTGCTCACCTT | 22 |
| ID2 | NM_00216 | S5009/ID2.p4 | TGCCCAGCATCCCCCAGAACAA | 22 |
| IGF1R | NM_00087 | S1249/IGF1R.f3 | GCATGGTAGCCGAAGATTTCA | 21 |
| IGF1R | NM_00087 | S1250/IGF1R.r3 | TTTCCGGTAATAGTCTGTCTCATAGATATC | 30 |
| IGF1R | NM_00087 | S4895/IGF1R.p3 | CGCGTCATACCAAAATCTCCGATTTTGA | 28 |
| IL6 | NM_00060 | S0760/IL6.f3 | CCTGAACCTTCCAAAGATGG | 20 |
| IL6 | NM_00060 | S0761/IL6.r3 | ACCAGGCAAGTCTCCTCATT | 20 |
| IL6 | NM_00060 | S4800/IL6.p3 | CCAGATTGGAAGCATCCATCTTTTTCA | 27 |
| ILT-2 | NM_00666 | S1611/ILT-2.f2 | AGCCATCACTCTCAGTGCAG | 20 |
| ILT-2 | NM_00666 | S1612/ILT-2.r2 | ACTGCAGAGTCAGGGTCTCC | 20 |
| ILT-2 | NM_00666 | S4904/ILT-2.p2 | CAGGTCCTATCGTGGCCCCTGA | 22 |
| IRS1 | NM_00554 | S1943/IRS1.f3 | CCACAGCTCACCTTCTGTCA | 20 |
| IRS1 | NM_00554 | S1944/IRS1.r3 | CCTCAGTGCCAGTCTCTTCC | 20 |
| IRS1 | NM_00554 | S5050/IRS1.p3 | TCCATCCCAGCTCCAGCCAG | 20 |
| KRT18 | NM_00022 | S1710/KRT18.f2 | AGAGATCGAGGCTCTCAAGG | 20 |
| KRT18 | NM_00022 | S1711/KRT18.r2 | GGCCTTTTACTTCCTCTTCG | 20 |
| KRT18 | NM_00022 | S4762/KRT18.p2 | TGGTTCTTCTTCATGAAGAGCAGCTCC | 27 |
| MAPK14 | NM_13901 | S5557/MAPK14.f2 | TGAGTGGAAAAGCCTGACCTATG | 23 |
| MAPK14 | NM_13901 | S5558/MAPK14.r2 | GGACTCCATCTCTTCTTGGTCAA | 23 |
| MAPK14 | NM_13901 | S5559/MAPK14.p2 | TGAAGTCATCAGCTTTGTGCCACCACC | 27 |
| MCM2 | NM_00452 | S1602/MCM2.f2 | GACTTTTGCCCGCTACCTTTC | 21 |

(continued)

| | | | | |
|---|---|---|---|---|
| MCM2 | NM_00452 | S1603/MCM2.r2 | GCCACTAACTGCTTCAGTATGAAGAG | 26 |
| MCM2 | NM_00452 | S4900/MCM2.p2 | ACAGCTCATTGTTGTCACGCCGGA | 24 |
| MCM6 | NM_00591 | S1704/MCM6.f3 | TGATGGTCCTATGTGTCACATTCA | 24 |
| MCM6 | NM_00591 | S1705/MCM6.r3 | TGGGACAGGAAACACACCAA | 20 |
| MCM6 | NM_00591 | S4919/MCM6.p3 | CAGGTTTCATACCAACACAGGCTTCAGCAC | 30 |
| MCP1 | NM_00298 | S1955/MCP1.f1 | CGCTCAGCCAGATGCAATC | 19 |
| MCP1 | NM_00298 | S1956/MCP1.r1 | GCACTGAGATCTTCCTATTGGTGAA | 25 |
| MCP1 | NM_00298 | S5052/MCP1.p1 | TGCCCCAGTCACCTGCTGTTA | 21 |
| MGMT | NM_00241 | S1922/MGMT.f1 | GTGAAATGAAACGCACCACA | 20 |
| MGMT | NM_00241 | S1923/MGMT.r1 | GACCCTGCTCACAACCAGAC | 20 |
| MGMT | NM_00241 | S5045/MGMT.p1 | CAGCCCTTTGGGGAAGCTGG | 20 |
| MMP12 | NM_00242 | S4381/MMP12.f2 | CCAACGCTTGCCAAATCCT | 19 |
| MMP12 | NM_00242 | S4382/MMP12.r2 | ACGGTAGTGACAGCATCAAAACTC | 24 |
| MMP12 | NM_00242 | S4383/MMP12.p2 | AACCAGCTCTCTGTGACCCCAATT | 24 |
| MSH3 | NM_00243 | S5940/MSH3.f2 | TGATTACCATCATGGCTCAGA | 21 |
| MSH3 | NM_00243 | S5941/MSH3.r2 | CTTGTGAAAATGCCATCCAC | 20 |
| MSH3 | NM_00243 | S5942/MSH3.p2 | TCCCAATTGTCGCTTCTTCTGCAG | 24 |
| MTA1 | NM_00468 | S2369/MTA1.f1 | CCGCCCTCACCTGAAGAGA | 19 |
| MTA1 | NM_00468 | S2370/MTA1.r1 | GGAATAAGTTAGCCGCGCTTCT | 22 |
| MTA1 | NM_00468 | S4855/MTA1.p1 | CCCAGTGTCCGCCAAGGAGCG | 21 |
| MUC1 | NM_00245 | S0782/MUC1.f2 | GGCCAGGATCTGTGGTGGTA | 20 |
| MUC1 | NM_00245 | S0783/MUC1.r2 | CTCCACGTCGTGGACATTGA | 20 |
| MUC1 | NM_00245 | S4807/MUC1.p2 | CTCTGGCCTTCCGAGAAGGTACC | 23 |
| NPD009 (A) | NM_02068 | S4474/NPD009.f3 | GGCTGTGGCTGAGGCTGTAG | 20 |
| NPD009 (A) | NM_02068 | S4475/NPD009.r3 | GGAGCATTCGAGGTCAAATCA | 21 |
| NPD009 (A) | NM_02068 | S4476/NPD009.p3 | TTCCCAGAGTGTCTCACCTCCAGCAGAG | 28 |
| PR | NM_00092 | S1336/PR.f6 | GCATCAGGCTGTCATTATGG | 20 |
| PR | NM_00092 | S1337/PR.r6 | AGTAGTTGTGCTGCCCTTCC | 20 |
| PR | NM_00092 | S4743/PR.p6 | TGTCCTTACCTGTGGGAGCTGTAAGGTC | 28 |
| PRKCD | NM_00625 | S1738/PRKCD.f2 | CTGACACTTGCCGCAGAGAA | 20 |
| PRKCD | NM_00625 | S1739/PRKCD.r2 | AGGTGGTCCTTGGTCTGGAA | 20 |
| PRKCD | NM_00625 | S4923/PRKCD.p2 | CCCTTTCTCACCCACCTCATCTGCAC | 26 |
| PTPD1 | NM_00703 | S3069/PTPD1.f2 | CGCTTGCCTAACTCATACTTTCC | 23 |
| PTPD1 | NM_00703 | S3070/PTPD1.r2 | CCATTCAGACTGCGCCACTT | 20 |
| PTPD1 | NM_00703 | S4822/PTPD1.p2 | TCCACGCAGCGTGGCACTG | 19 |
| RAB6C | NM_03214 | S5535/RAB6C.f1 | GCGACAGCTCCTCTAGTTCCA | 21 |
| RAB6C | NM_03214 | S5537/RAB6C.p1 | TTCCCGAAGTCTCCGCCCG | 19 |
| RAB6C | NM_03214 | S5538/RAB6C.r1 | GGAACACCAGCTTGAATTTCCT | 22 |
| RALBP1 | NM_00678 | S5853/RALBP1.f1 | GGTGTCAGATATAAATGTGCAAATGC | 26 |
| RALBP1 | NM_00678 | S6854/RALBP1.r1 | TTCGATATTGCCAGCAGCTATAAA | 24 |
| RALBP1 | NM_00678 | S5855/RALBP1.p1 | TGCTGTCCTGTCGGTCTCAGTACGTTCA | 28 |
| RAP1GDS | NM_02115 | S5306/RAP1 GD.f2 | TGTGGATGCTGGATTGATTT | 20 |
| RAP1GDS | NM_02115 | S5307/RAP1GD.r2 | AAGCAGCACTTCCTGGTCTT | 20 |
| RAP1GDS | NM_02115 | S5308/RAP1 GD.p2 | CCACTGGTGCAGCTGCTAAATAGCA | 25 |
| RASSF1 | NM_00718 | S2393/RASSF1.f3 | AGTGGGAGACACCTGACCTT | 20 |
| RASSF1 | NM_00718 | S2384/RASSF1.r3 | TGATCTGGGCATTGTACTCC | 20 |
| RASSF1 | NM_00718 | S4909/RASSF1.p3 | TTGATCTTCTGCTCAATCTCAGCTTGAGA | 29 |
| rhoC | NM_00516 | S2162/rhoC.f1 | CCCGTTCGGTCTGAGGAA | 18 |
| rhoC | NM_00516 | S2163/rhoC.r1 | GAGCACTCAAGGTAGCCAAAGG | 22 |
| rhoC | NM_00516 | S5042/rhoC.p1 | TCCGGTTCGCCATGTCCCG | 19 |
| RUNX1 | NM_00175 | S4588/RUNX1.f2 | AACAGAGACATTGCCAACCA | 20 |

(continued)

| | | | | |
|---|---|---|---|---|
| RUNX1 | NM_00175 | S4589/RUNX1.r2 | GTGATTTGCCCAGGAAGTTT | 20 |
| RUNX1 | NM_00175 | S4590/RUNX1.p2 | TTGGATCTGCTTGCTGTCCAAACC | 24 |
| SEMA3F | NM_00418 | S2857/SEMA3F.f3 | CGCGAGCCCCTCATTATACA | 20 |
| SEMA3F | NM_00418 | S2858/SEMA3F.r3 | CACTCGCCGTTGACATCCT | 19 |
| SEMA3F | NM_00418 | S4972/SEMA3F.p3 | CTCCCCACAGCGCATCGAGGAA | 22 |
| SGCB | NM_00023 | S5752/SGCB.f1 | CAGTGGAGACCAGTTGGGTAGTG | 23 |
| SGCB | NM_00023 | S5753/SGCB.r1 | CCTTGAAGAGCGTCCCATCA | 20 |
| SGCB | NM_00023 | S5754/SGCB.p1 | CACACATGCAGAGCTTGTAGCGTACCCA | 28 |
| STAT1 | NM_00731 | S1542/STAT1.f3 | GGGCTCAGCTTTCAGAAGTG | 20 |
| STAT1 | NM_00731 | S1543/STAT1.r3 | ACATGTTCAGCTGGTCCACA | 20 |
| STAT1 | NM_00731 | S4878/STAT1.p3 | TGGCAGTTTTCTTCTGTCACCAAAA | 25 |
| STAT3 | NM_00315 | S1545/STAT3.f1 | TCACATGCCACTTTGGTGTT | 20 |
| STAT3 | NM_00315 | S1546/STAT3.r1 | CTTGCAGGAAGCGGCTATAC | 20 |
| STAT3 | NM_00315 | S4881/STAT3.p1 | TCCTGGGAGAGATTGACCAGCA | 22 |
| TBP | NM_00319 | S0262/TBP.f1 | GCPCGAAACGCCGAATATA | 19 |
| TBP | NM_00319 | S0284/TBP.r1 | CGTGGCTCTCTTATCCTCATGAT | 23 |
| TBP | NM_00319 | S4751/TBP.p1 | TACCGCAGCAAACCGCTTGGG | 21 |
| TK1 | NM_00325 | S08TK1.f2 | GCCGGGAAGACCGTAATTGT | 20 |
| TK1 | NM_00325 | S0327/TK1.r2 | CAGCGGCACCAGGTTCAG | 18 |
| TK1 | NM_00325 | S4798/TK1.p2 | CAAATGGCTTCCTCTGGAAGGTCCCA | 26 |
| TP53BP1 | NM_00565 | S1747/TP53BP.f2 | TGCTGTTGCTGAGTCTGTTG | 20 |
| TP53BP1 | NM_00565 | S1748/TP53BP.r2 | CTTGCCTGGCTTCACAGATA | 20 |
| TP53BP1 | NM_00565 | S4924/TP53BP.p2 | CCAGTCCCCAGAAGACCATGTCTG | 24 |
| TUBB | NM_00106 | S5826/TUBB.f3 | TGTGGTGAGGAAGGAGTCAG | 20 |
| TUBB | NM_00106 | S5827/TUBB.r3 | CCCAGAGAGTGGGTCAGC | 18 |
| TUBB | NM_00106 | S5828/TUBB.p3 | CTGTGACTGTCTCCAGGGCTTCCA | 24 |
| VCAM1 | NM_00107 | S3505/VCAM1.f1 | TGGCTTCAGGAGCTGAATACC | 21 |
| VCAM1 | NM_00107 | S3506A/CAM1.r1 | TGCTGTCGTGATGAGAAAATAGTG | 24 |
| VCAM1 | NM_00107 | S3507/VCAM1.p1 | CAGGCACACACAGGTGGGACACAAAT | 26 |
| Wnt-5a | NM_00339 | S6183/Wnt-5a.f1 | GTATCAGGACCACATGCAGTACATC | 25 |
| Wnt-5a | NM_00339 | S6184/Wnt-5a.r1 | TGTCGGAATTGATACTGGCATT | 22 |
| Wnt-5a | NM_00339 | S6185/Wnt-5a.p1 | TTGATGCCTGTCTTCGCGCCTTCT | 24 |
| ZNF38 | NM_14591 | S5593/ZNF38.f3 | TTTCCAAACATCAGCGAGTC | 20 |
| ZNF38 | NM_14591 | S6594/ZNF38.r3 | AACAGGAGCGCTTGAAAGTT | 20 |
| ZNF38 | NM_14591 | S5595/ZNF38.p3 | ACGGTGCTTCTCCCTCTCCAGTG | 23 |

**TABLE 3**

| | | Sequence |
|---|---|---|
| A-Catenin | NM_00190 | CGTTCCGATCCTCTATACTGCATCCCAGGCATGCCTACAGCACCCTGATGTCGCAGCCTATAAGGCCAACAGGGACCT |
| ABCC5 | NM_00568 | TGCAGACTGTACCATGCTGACCATTGCCCATCGCCTGCACACGGTTCTAGGCTCCGATAGGATTATGGTGCTGGCC |
| AK055699 | AK05S699 | CTGCATGTGATTGAATAAGAAACAAGAAAGTGACCACACCAAAGCCTCCCTGGCTGGTGTACAGGGATCAGGTCCACA |
| AKAP-2 | NM_00720 | ACGAATTGTCGGTGAGGTCTCAGGATACCACAGTCCTG GAGACCCTATCCAATGATTTCAGCATGGAC |
| AKT1 | MM_00516 | CGCTTCTATGGGGCTGAGATTGTGTCAGCCCTGGACTACCTGCACTCGGAGAAGAACGTGGTGTACCGGGA |
| AKT2 | NM_00162 | TCCTGCCACCCTTCAAACCTCAGGTCACGTCCGAGGTCGACACAAGGTACTTCGATGATGAATTTACCGCC |
| APC | NM_00003 | GGACAGCAGGAATGTGTTTCTCCATACAGGTCACGGGGAGCCAATGGTTCAGAAACAAATCGAGTGGGT |
| BAD | NM_03298 | GGGTCAGGTGCCTCGAGATCGGGCTTGGGCCCAGAGCATGlTCCAGATCCCAGAGTTTGAGCCGAGTGAGCAG |
| BAG1 | NM_00432 | CGTTGTCAGCACTTGGAATACAAGATGGTTGCCGGGTCATGTTAATTGGGAAAAAGAACAGTCCACAGGAAGAGGTTGAAC |
| BBC3 | NM_01441 | CCTGGAGGGTCCTGTACAATCTCATCATGGGACTCCTGCCCTTACCCAGGOGCCACAGAGCCCCCGAGATGGAGCCCAATTAG |
| Bcl2 | NM_00063 | CAGATGGACCTAGTACCCACTGAGATTTCCACGCCGAAGGACAGCGATGGGAAAAATGCCCTTAAATCATAGG |
| CCND1 | NM_00175 | GCATGTTCGTGGCCTCTAAGATGAAGGAGACCATCCCCCTGACGGCCGAGAAGCTGTGCATCTACACCG |
| CD18 | NM_00021 | CGTCAGGACCCACCATGTCTGCCCCATCACGCGGCCGAGACATGGCTTGGCCACAGCTCTTGAGGATGTCACCAATTAACC |
| CD31 | NM_00044 | TGTATlTCAAGACCTCTGTGCACTTATTTATGAACCTGCCCTGCTCCCACAGAACACAGCAATTCCTCAGGCTAA |
| CD3z | NM_00073 | AGATGAAGTGGAAGGCGCTTTTCACCGCGGCCATCCTGCAGGCACAGTTGCCGATTACAGAGGCA |
| CD9 | NM_00176 | GGGCGTGGAACAGTTTATCTCAGACATCTGCCCCAAGAAGGACGTACTCGAAAGCTTCACCGTG |
| CDC20 | NM_00125 | TGGATTGGAGTTCTGGGAATGTACTGGCCGTGGCACTGGACAACAGTGTGTACCTGTGGAGTGCAAGC |
| CDC25B | NM_02187 | AAACGAGCAGTTTGCCATCAGACGCTTCCAGTCTATGCCGGTGAGGCTGCTGGGCCACAGCCCCGTGCTTCGGAACATCACCAAC |
| CEGP1 | NM_02097 | TGACAATCAGCACACCTGCATTCACCGCTCGGAAGAGGGCCTGAGCTGCATGAATAAGGATCACGGCTGTAGTCACA |
| CGA (CHG) | NM_00127 | CTGAAGGAGCTCCAAGACCTCGCTCTOCAAGGCGCCAAGGAGAGGGCACATCAGCAGAAGAAACACAGCGGTTTTG |
| COL1A1 | NM_00008 | GTGGCCATCCAGCTGACCTTCCTGCGCCTGATGTCGACCGAGGCCTCCGAGAACATGACCCTACCACTG |
| COL1A2 | NM_00008 | CAGCCAAGAACTGGTATAGGAGCTCCAAGGACAAGAAACACGTCTGGCTAGGAGAAACTATCAATGCTGGCAGCCAGTTT |
| Contig 510 | XM_05894 | CGACAGTTGCGATGAAAGTTCTAATCTCTTCCCTCCTCCTGTTGCTGCCACTAATGCTGATGTCCATGGTCTCTAGCAGCC |
| CRABP1 | NM_00437 | AACTTCAAGGTCGGAGAAGGCTTTGAGGAGGAGACCGTGGACGGACGCAAGTGCAGGAGTTTAGCCA |
| CRIP2 | NM_00131 | GTGCTACGCCACCCTGTTCGGACCCAAAGGCGTGAACATCGGGGGCGCGGGCTCCTACATCTACGAGAAGCCCCTG |
| CYBA | NM_00010 | GGTGCCTACTCCATTGTGGCGGGGGTGTTTGTGTGCCTGCTGGAGTACCCCCGGGGGAAGAGGAAGAAGGGCTCCAC |
| DHPS | NM_01340 | GGGAGAACGGGATCAATAGGATCGGAAACCTGCTGGTGCCCAATGAGAATTACTGCAAGTTTGAGGACTGGCTGATGC |
| DICER1 | NM_17743 | TCCAATTCCAGCATCACTGTGGAGAAAAGCTGTTTGTCTCCCCCAGCATACTTTATCGCCTTCACTGCC |
| DR4 | NM_M384 | TGCACAGAGGGTGTGGGTTACACCAATGCTTCCAACAATTTGTTTGCTTGCCCCCATGTACAGCTTGTAAATCAGATGAAGA |
| E2F1 | NM_00522 | ACTCCCTCTACCCTTGAGCAAGGGCAGGGOTCCCTGAGCTGTTCTTCTCTACTGAAGGAACTGAGGCCTG |
| ER2 | NM_00143 | TGGTCCATCGCCAGTTATCACATCTGTATGCGGAACCTCAAAAGAGTCCCTGGTGTGAAGCAAGATCGCTAGAACA |
| ErbB3 | NM_00198 | CGGTTATGTCATGCCAGATACACACCTCAAAGGTACTCCCTCCTCCCGGGAAGGCACCCTTTCTTCAGTGGGTCTCAGTTC |
| ERBB4 | NM_00523 | TGGCTCTTAATCAGTTTCGTTACCTGCCTCTGGAGAATTTACGCATTATTCGTGGGACAAAACTTTATGAGGATCGATATGCCTTG |

(continued)

| | | Sequence |
|---|---|---|
| ERCC1 | NM_00198 | GTCCAGGTGGATGTGAAAGATCCCCAGCAGGCCCTCAAGGAGCTGGCTAAGATGTGTATCCTG |
| ERK1 | Z11696 | ACGGATCACAGTGGAGGAAGCGCTGGCTCACCCCTACCTGGAGCAGTACTATGACCCGACGGATGAG |
| ESRRG | NM_00143 | CCAGCACCATTGTTGAAGATCCCCAGACCAAGTGTGAATACATGCTCAACTCGATGCCCAAGAGACT |
| fasi | NM_00063 | GCACTTTGGGATTCTTTCCATTATGATTCTTTGTTACAGGCACCGAGAATGTTGTATTCAGTGAGGGTCTTCTTACATGC |
| FBXO5 | NM_01217 | GGCTATTCCTCATTTTCTCTACAAAGTGGCCrCAGTGAACATGAAGAAGGTAOOCTCCTGGAGGAGAATTTtGGTGACAGTCTACMTCC |
| FHIT | NM_00201 | CCAGTGGAGCGCTTCCATGACCTGCGTCCTGATGAAGTGGCCGATTTGTTTCAGACGACCCAGAGAG |
| FUS | NM_00496 | GGATAATTGAGACAACAACACCATCTTTGTGCAAGGCCTGGGTGAGAATGTTACAATTGAGTCTGTGGCTGATTACTTCA |
| FYN | NM_00203 | GAAGCGCAGATCATGAAGAAGCTGAAGCACGACAAGCTGGTCCAGCTCTATGCAGTGGTCTCTGAGGAG |
| G-Catenin | NM_00223 | TCAGCAGCAAGGGCATCATGGAGGAGGATGAGGCCTGCGGGCGCCAGTACACGCTCAAGAAAACCACC |
| GATA3 | NM_00205 | CAAAGGAGCTCACTGTGGTGTCTGTGTTCCAACCACTGAATCTGGACCCTCTGTGAATAAGCATTCTGATC |
| G8P1 | NM_00205 | TTGGGAAATATTTGGGCATTGGTCTGGCCAAGTCTACAATGTCCCAATATCAAGGACAACCACCCTAGCTTCT |
| GBP2 | NM_00412 | GCATGGGAACCATCAACCAGCAGGCCATGGACCAACTTCACTATGTGACAGAGCTGACAGATCGAATCAAGGCAAACTCCTCA |
| GGPS1 | NM_00483 | CTCCGACGTGGGTTTCCAGTGGCCGACAGCATCTATGGAATCCCATCTGTCATCAATTCTGCCAATTACG |
| GRB7 | NM_00531 | CCATCTGCATCCATCTTGTTTGGGCTCCCCACCCTTGAGAAGTGCCTCAGATAATACCCTGGTGGCC |
| Hepsin | NM_00215 | AGGCTGCTGGAGGTCATCTCCGTGTGTGATTGCCCCAGAGGCCGTTTCTTGGCCGCCATCTGCCAAGACTGTGGCCGCAGGAAG |
| HILA-DPB1 | NM_00212 | TCCATGATGGTTCTGCAGGTTTCTGCGGCCCCCCGGACAGTGGCTCTGACGGCGTTACTGATGGTGCTGCTCA |
| ID2 | NM_00216 | AACGACTGCTACTCCAAGCTGAAGGAGCTGGTGGCCAGCATCCCCCAGAACAAGAAGGTGAGCAGATGGAAATCC |
| IGF1R | NM_00087 | GCATGGTAGCCGAAGATTTCACAGTCAAAATCGGAGATTTTGGTATGACGCGAGATATCTATGAGACAGACTATTACCGGAAA |
| IL6 | NM_00060 | CCTGAACCTTCCAAAGATGGCTGAAAAAGATGGATGCTTCCAATCTGGATTCAATGAGGAGACTTGCCTGGT |
| ILT-2 | NM_00668 | AGCCATCACTCTCAGTGCAGCCAGGTCCTATCGTGGCCCCTGAGGAGACCCTGACTCTGCAGT |
| IRS1 | NM_00554 | CCACAGCTCACCTTCTGTCAGGTGTCCATCCCAGCTCCAGCCAGGTCCGAGAGGAAGAGACTGGCACTGAGG |
| KRT18 | NM_00022 | AGAGATCGAGGCTCTCAAGGAGGAGCTGCTCTTCATGAAGAAGAACCACGAAQAGGAAGTAAAAGGCC |
| MAPK14 | MM_13901 | TGAGTGGAAAAGCCTGACCTATGATGAAGTCATCAGCTTTGTGCCACCACCCCTTGACCAAGAAGAGATGGAGTCC |
| MCM2 | NM_00452 | GACTTTT GCCCGCTACCTTTCATTCCGGCGTGACAACAATGAGCTGTTGCTCTTCATACTGAAGCAGTTAGTGGC |
| MCM6 | NM_00591 | TGATGGTCCTATGTGTCACATTCATCACAGGTTTCATACCAACACAGGCTTCAGCACTTCCTTTGGTGTGTTTCCTGTCCCA |
| MCP1 | NM_00298 | CGTCAGCCAGATGCAATCAATGOCCCAGTCACCTGCTGTTATAACTTCACCAATAGGAAGATCTCAGTGC |
| MGMT | NM_00241 | GTGAAATGAAACGCACCACACTGGACGCCCTTTGGGGAAGCTGGAGCTGTCTGGTTGTGAGCAGGGTC |
| MMP12 | NM_00242 | CCAACGCTTGCCAAATCCTGACAATTCAGAACCAGCTCTCTGTGACCCCAATTTGAGTTTTGATGCTGTCACTACCGT |
| MSH3 | NM_00243 | TGATTACCATCATGGCTCAGATTGGCTCCTATGTTCCTGCAGAAGAAGCGACAATTGGGATTGTGGATGGCATTTTCACAAG |
| MTA1 | NM_00468 | CCGCCGTGCCTGAAGAGAAACGCGCTCCTTGGCGGACACTGGGGGAGGAGAGGAAGAAGCGCGGCTAACTTATTCC |
| MUC1 | NM_00245 | GGCCAGGATCTGTGGTAGGTACAATTGACTCTGGCCTTCCGAGAAGGTACCATCAATGTCCACGACGTGGAG |
| NPD009 (A) | NM_02068 | GGCTGTGGCTGAGGCTGTAGCATCTCTGCTGGAGGTGAGACACTCTGGGAACTGATTTGACCTCGAATGCTCC |

(continued)

| | | Sequence |
|---|---|---|
| PR | NM_00092 | GCATCAGGCTGTCATTATGGTGTCCTTACCTGTGGGAGCTGTAAGGTCTTCTTTAAGAGGGCAATGGAAGGGCAGCACAACTACT |
| PRKCD | NM_006251 | CTGACACTTGCCGCAGAGAATCCCTTTCTCACCCACCTCATCTGCACCTTCCAGACCAAGGACCACT |
| PTPD1 | NM_00703 | CGCTTGCCTAACTCATACTTTCCCGTTGACACTTGATCGCGGGCGTGGCACTGGGACGTAAGTGGCGCAGTCTGAATGG |
| RAB6C | NM_03214 | GCGACAGCTCCTCTAGTTCGCGTGTCCGCGGGCCGAGACTTCGGGAATCCGCTGAGGAAATTCAAGCTGGTGTTCC |
| RALBP1 | NM_00678 | GGTGTCAGATATAAATGTGCAAATGCCTTCTTGGTGTCGGTGTOGGTCTCAGTACGTTCACTTTATAGCTGCTGGCAATATCGAA |
| RAP(GDS | NM_02115 | TGTGGATGCTGGATTGATTTCACCACTGGTGCAGCTGCTAAATAGCAAAGACCAGGAAGTGCTGCTT |
| RASSF1 | NM_00718 | AGTGGGAGACACCTGACCTTTCTCAAGCTGAGATTGAGCAGAAGATCAAGGAGTACAATGCCCAGATCA |
| rhoC | NM_00516 | CCCGTTCGGTCTGAGGAAGGCCGGGAGTGGCGAACCGGATCAGTGCCTTTGGCTACGTTGAGTGCTC |
| RUNX1 | NM_00175 | AACAGAGACATTGCCAACCATATTGGATCTGCTTGCTGTCCAAACCAGCAAACTTCCTGGGCAAATCAC |
| SEMA3F | NM_00418 | CGCGAGCCCCTCATTATACACTGGGCAGCCTOCCCACAGCGCATCGAGGAATGCGTGCTCTCAGGCMGGATGTCAACGGCGAGT G |
| SGCB | NM_00023 | CAGTGGAGACCAGTTGGGTAGTGGTGACTGGGTACGCTACAAGCTCTGCATGTGTGCTGATGGGACGCTCTTCAAGG |
| STAT1 | NM_00731 | GGGCTCAGCTTTCAGAAGTGCTGAGTTGGCAGTTTTCTTCTGTCACCAAAAGAGGTCTCAATGTGGACCAGCTGAACATGT |
| STAT3 | NM_00315 | TCACATGCCACTTTGGTGTTTCATAATCTCCTGGGAGAGATTGACCAGCAGTATAGCCGCTTCCTGCAAG |
| TBP | NM_00319 | GCCCGAAACGCAATATAATCCCAATCGGTTTGCTGCGGTAATCATCAGGATAAGAGAGCCACG |
| TK1 | NM_00325 | GCCGGGAAGACCGTAATTGTGGCTGCACTGGATGGGACCTTCCAGAGGAAGCCATTTGGGGCCATCCTGAACCTGGTGCCGCTG |
| TP538P1 | NM_00565 | TGCTGTTGCTGAGTCTGTTGCCAGTCCCCAGAAGACCATGTCTGTGTTGAGCTGTATCTGTGAAGCCAGGCAAG |
| TUBB | NM_00106 | TGTGGTGAGGAAGGAGTCAGAGAGCTGTGACTGTCTCCAGGGCTTCCAGCTGACCCACTCTCTGGG |
| VCAM1 | NM_00107 | TGGGTTGGGAGCTGAATACCCTCCGGGCACAAATAAGGGTTTTGGAACCACTATTTTCTCATCACGACAGCA |
| Wnt-5a | NM_00339 | GTATCAGGACCACATGCAGTACATCGGAGAAGGCGCGAAGACAGGCATCAAAGAATGCCAGTATCAATTCCGACA |
| 2NF38 | NM_14591 | TTTCCAAACATCAGCGAGTCCACACTGGAGAGGGAGAAGCACCGTAACTTTCAAGCGCTCCTGTT |

According to another aspect of the present invention, there is provided a method for predicting the response of a subject diagnosed with cancer to chemotherapy comprising determining the expression level of one or more prognostic RNA transcripts or their expression products in a biological sample comprising cancer cells obtained from said subject, wherein the predictive RNA transcript is the transcript of one or more genes selected from the group consisting of TBP; ILT.2; ABCC5; CD18; GATA3; DICER1; MSH3; GBP1; IRS1; CD3z; fas1; TUBB; BAD; ERCC1; MCM6; PR; APC; GGPS1; KRT18; ESRRG; E2F1; AKT2; A.Catenin; CEGP1; NPD009; MAPK14; RUNX1; ID2; G.Catenin; FBXO5; FHIT; MTA1; ERBB4; FUS; BBC3; IGF1R; CD9; TP53BP1; MUC1; IGFBP5; rhoC; RALBP1; CDC20; STAT3; ERK1; HLA.DPB1; SGCB; CGA; DHPS; MGMT; CRIP2; MMP12; ErbB3; RAP1GDS1; CDC25B; IL6; CCND1; CYBA; PRKCD; DR4; Hepsin; CRABP1; AK055699; Contig.51037; VCAM1; FYN; GRB7; AKAP.2; RASSF1; MCP1; ZNF38; MCM2; GBP2; SEMA3F; CD31; COL1A1; ER2; BAG1; AKT1; COL1A2; STAT1; Wnt.5a; PTPD1; RAB6C; TK1, ErbB2, CCNB1, BIRC5, STK6, MKI67, MYBL2, MMP11, CTSL2, CD68, GSTM1, BCL2, ER1 wherein

(a) for every unit of increased expression of one or more of ILT.2; CD18; GBP1; CD3z; fas1; MCM6; E2F1; ID2; FBXO5; CDC20; HLA.DPB1; CGA; MMP12; CDC25B; IL6; CYBA; DR4; CRABP1; Contig.51037; VCAM1; FYN; GRB7; AKAP.2; RASSF1; MCP1; MCM2; GBP2; CD31; ER2; STAT1; TK1; ERBB2, CCNB1, BIRC5, STK6, MKI67, MYBL2, MMP11, CTSL2 and CD68; or the corresponding expression product, said subject is predicted to have an increased likelihood of response to chemotherapy; and

(b) for every unit of increased expression of one or more of TBP; ABCC5; GATA3; DICER1; MSH3; IRS1; TUBB; BAD; ERCC1; PR; APC; GGPS1; KRT18; ESRRG; AKT2; A.Catenin; CEGP1; NPD009; MAPK14; RUNX1; G.Catenin; FHIT; MTA1; ErbB4; FUS; BBC3; IGF1R; CD9; TP53BP1; MUC1; IGFBP5; rhoC; RALBP1; STAT3; ERK1; SGCB; DHPS; MGMT; CRIP2; ErbB3; RAP1GDS1; CCND1; PRKCD; Hepsin; AK055699; ZNF38; SEMA3F; COL1A1; BAG1; AKT1; COL1A2; Wnt.5a; PTPD1; RAB6C; GSTM1, BCL2, BSR1; or the corresponding expression product, said subject is predicted to have a decreased likelihood of response to chemotherapy.

The predictive RNA transcript may be the transcript of one or more genes selected from the group consisting of TBP; ILT.2; ABCC5; CD18; GATA3;
DICER1; MSH3; GBP1; IRS1; CD3z; fas1; TUBB; BAD; ERCC1; MCM6; PR; APC; GGPS1; KRT18; ESRRG; E2F1; AKT2; A.Catenin; CEGP1; NPD009; MAPK14; RUNX1; ID2; G.Catenin; FBXO5; FHIT; MTA1; ERBB4; FUS; BBC3; IGF1R; CD9; TP53BP1; MUC1; IGFBP5; rhoC; RALBP1; CDC20; STAT3; ERK1; HLA.DPB1; SGCB; CGA; DHPS; MGMT; CRIP2; MMP12; ErbB3; RAP1GDS1; CDC25B; IL6; CCND1; CYBA; PRKCD; DR4; Hepsin; CRAB1; AK055699; Contig.51037; VCAM1; FYN; GRB77; AKAP.2; RASSF1; MCP1; ZNF38; MCM2; GBP2; SEMA3F; CD31; COL1A1; BR2; BAG1; AKT1; COL1A2; STAT1; Wnt.5a; PTPD1; RAB6C; and TK1.

The response may be a complete pathological response.

The subject may be a human patient.

The cancer may be selected from the group consisting of breast cancer, ovarian cancer, gastric cancer, colon cancer, pancreatic cancer, prostate cancer and lung cancer.

The cancer may be invasive breast cancer.

The cancer may be stage II or stage III breast cancer.

The chemotherapy may be adjuvant chemotherapy.

The chemotherapy may be neoadjuvant chemotherapy.

The neoadjuvant chemotherapy may comprise the administration of a taxane derivative.

The taxane may be docetacal or paclitaxel.

The chemotherapy may further comprise the administration of an additional anti-cancer agent.

The additional anti-cancer agent may be a member of the anthracyline class of anti-cancer agents.

The additional anti-cancer agent may be doxorubicin.

The additional anti-cancer agent may be a topoisomerase inhibitor.

The method may comprise determining the expression levels of at least two of said prognostic transcripts of their expression products.

The method may comprise determining the expression levels of at least five of said prognostic transcripts of their expression products.

The method may comprise determining the expression levels of all of said prognostic transcripts or their expression products.

The biological sample may be a tissue sample comprising cancer cells. The tissue may be fixed, paraffin-embedded, or fresh, or frozen.

The tissue may be from fine needle, core, or other types of biopsy.

The tissue sample may be obtained by fine needle aspiration, bronchial lavage, or transbronchial biopsy.

The expression level of said prognostic RNA transcript or transcripts may be determined by RT-PCR or another PCR-based method.

The expression level of said expression product or products may be determined by immunohistochemistry.

The expression level of said expression product or products may be determined by proteomics techniques.

The assay for the measurement of said prognostic RNA transcripts or their expression products may be provided in the form of a kit or kits.

The predictive transcript may comprise an intron-based sequence the expression of which correlates with the expression of a corresponding exon sequence.

According to another aspect of the present invention, there is provided an array comprising polynucleotides hybridizing to a plurality of the following genes: TBP; ILT.2; ABCC5; CD18; GATA3; DICER1; MSH3; GBP1; IRS1; CD3z; fasl; TUBB; BAD; ERCC1; MCM6; PR; APC; GGPS1; KRT18; ESRRG; E2F1; AKT2; A.Catenin; CEGP1; NPD009; MAPK14; RUNX1; ID2; G.Catenin; FBXO5 FHIT; MTA1; ERBB4; FUS; BBC3; IGF1R; CD9; TP53BP1; MUC1; IGF8P5; rhoC; RALBP1; CDC20; STAT3; ERK1; HLA.DPB1; SGCB; CGA; DHPS; MGMT; CRIP2; MMP12; ErbB3; RAF1GDS1; CDC25B; IL6; CCND1; CYBA; PRKCD; DR4; Hepsin; CRABP1; AK055699; Contig.51037; VCAM1; FYN; GRB7; AKAP.2; RASSF1; MCP1; ZNF38; MCM2; GBP2; SEMA3F; CD31; COL1A1; ER2; BAG1; AKT1; COL1A2; STAT1; Wnt.5a; PTPD1; RAB6C; TK1, ErbB2, CCNB1, BIRC5, STK6, MKI67, MYBL2, MMP11, CTSL2, CD68, GSTM1, BCL2, ESR1.

The array may comprise polynucleotides hybridizing to a plurality of the following genes: TBP; ILT.2; ABCC5; CD18; GATA3; DICER1; MSH3; GBP1; IRS1; CD3z; fas1; TUBB; BAD; ERCC1; MCM6; PR; APC; GGPS1; KRT18; ESRRG; E2F1; AKT2; A.Catenin; CEGP1; NPD009; MAPK14; RUNX1; ID2; G.Catenin; FBXO5; FHIT; MTA1; ERBB4; FUS; BBC3; IGF1R; CD9; TP53BP1; MUC1; IGFBP5; rhoC; RALBP1; CDC20; STAT3; ERK1; HLA.DPB1; SGCB; CGA; DHPS; MGMT; CRIP2; MMP12; ErbB3; RAP1GDS1; CDC25B; IL6; CCND1; CYBA; PRKCD; DR4; Hepsin; CRABP1; AK055699; Contig.51037; VCAM1; FYN; GRB7; AKAP.2; RASSF1; MCP1; ZNF38; MCM2; GBP2; SEMA3F; CD31; COL1A1; ER2; BAG1; AKT1; COL1A2; STAT1; Wnt.5a; PTPD1; RAB6C; TK1.

The array may comprise polynucleotides hybridizing to a plurality of the following genes: ILT.2; CD18; GBP1; CD3z; fas1; MCM6; E2F1; ID2; FBXO5 CDC20; HLA.DPB1; CGA; MMP12; CDC25B; IL6; CYBA; DR4; CRABP1; Contig.51037; VCAM1; FYN; GRB7; AKAP.2; RASSF1; MCP1; MCM2; GBP2; CD31; ER2; STAT1; TK1; ERBB2, CCNB1, BIRC5, STK6, MKI67, MYBL2, MMP11, CTSL2 and CD68.

The array may comprise polynucleotides hybridizing to a plurality of the following genes: ILT.2; CD18; GBP1; CD3z; fas1; MCM6; E2F1; ID2; FBXO5 CDC20; HLA.DPB1; CGA; MMP12; CDC25B; IL6; CYBA; DR4; CRABP1; Contig.51037; VCAM1; FYN; GRB7; AKAP.2; RASSF1; MCP1; MCM2; GBP2; CD31; ER2; STAT1; TK1.

The array may comprise polynucleotides hybridizing to a plurality of the following genes: TBP; ABCC5; GATA3; DICER1; MSH3; IRS1; TUBB; BAD; ERCC1; PR; APC; GGPS1; KRT18; ESRRG; AKT2; A.Catenin; CEGP1; NPD009; MAPK14; RUNX1; G.Catenin; FHIT; MTA1; ErbB4; FUS; BBC3; IGF1R; CD9; TP53BP1; MUC1; IGFBP5; rhoC; RALBP1; STAT3; ERK1; SGCB; DHPS; MGMT; CRIP2; ErbB3; RAP1GDS1; CCND1; PRKCD; Hepsin; AK055699; ZNF38; SEMA3F; COL1A1; BAG1; AKT1; COL1A2; Wnt.5a; PTPD1; RAB6C; GSTM1, BCL2, ESR1.

The array may comprise polynucleotides hybridizing to a plurality of the following genes: TBP; ABCC5; GATA3; DICER1; MSH3; IRS1; TUBB; BAD; EPCC1; PR; APC; GGPS1; KRT18; ESRRG; AKT2; A.Catenin; CEGP1; NPD009; MAPK14; RUNX1; G.Catenin; FHIT; MTA1; ErbB4; FUS; BBC3; IGF1R; CD9; TP53BP1; MUC1; IGFBP5; rhoC; RALBP1; STAT3; ERK1; SGCB; DHPS; MGMT; CRIP2; ErbB3; RAP1GDS1; CCND1; PRKCD; Hepsin; AK055699; ZNF38; SEMA3F; COL1A1; BAG1; AKT1; COL1A2; Wnt.5a; PTPD1; RAB6C.

The array may comprise at least five of said polynucleotides.

The array may comprise at least 10 of said polynucleotides.

The array may comprise at least 15 of said polynucleotides.

The array may comprise polynucleotides hybridizing to all of said genes.

The array may comprise more than one polynucleotide hybridizing to the same gene.

At least one of the said polynucleotides may comprise an intron-based sequence the expression of which correlates with the expression of a corresponding exon sequence.

The polynucleotides may be cDNAs.

The polynucleotides may be oligonucleotides.

According to another aspect of the present invention, there is provided a method of preparing a personalized genomics profile for a patient comprising the steps of:

(a) determining the normalized expression levels of the RNA transcripts or the expression products of a gene or gene set selected from the group consisting of TBP; ILT.2; ABCC5; CD18; GATA3; DICER1; MSH3; GBP1; IRS1; CD3z; fas1; TUBB; BAD; ERCC1; MCM6; PR; APC; GGPS1; KRT18; ESRRG; E2F1; AKT2; A.Catenin; CEGP1; NPD009; MAPK14; RUNX1; ID2; G.Catenin; FBXO5 FHIT; MTA1; ERBB4; FUS; BBC3; IGF1R; CD9; TP53BP1; MUC1; IGFBP5; rhoC; RALBP1; CDC20; STAT3; ERK1; HLA.DPB1; SGCB; CGA; DHPS; MGMT; CRIP2; MMP12; ErbB3; RAP1GDS1; CDC25B; IL6; CCND1; CYBA; PRKCD; DR4; Hepsin; CRABP1; AK055699; Contig.51037; VCAM1; FYN; GRB7; AKAP.2; RASSF1; MCP1; ZNF38; MCM2; GBP2; SEMA3F; CD31; COL1A1; ER2; BAG1;

AKT1; COL1A2; STAT1; Wnt.5a, PTPD1; RAB6C; TK1, ErbB2, CCNB1, BIRC5, STK6, . MKI67, MYBL2, MMP11, CTSL2, CD68, GSTM1, BCL2, ESR1, in a cancer cell obtained from said patient; and
(b) creating a report summarizing the data obtained by said gene expression analysis.

The cancer cell may be obtained from a solid tumour.
The solid tumour may be selected from the group consisting of breast cancer, ovarian cancer, gastric cancer, colon cancer, pancreatic cancer, prostate cancer, and lung cancer.
The cancer cell may be obtained from a fixed, paraffin-embedded biopsy sample of said tumour.
The RNA may be fragmented.
The report may include recommendation for a treatment modality for said patient.
In an embodiment of the method, if increased expression of one or more of ILT.2; CD18; GBP1; CD3z; fas1, MCM6; E2F1; ID2; FBXO5 CDC20; HLA.DPB1; CGA; MMP12; CDC25B; IL6; CYBA; DR4; CRABP1; Contig.51037; VCAM1; FYN; GRB7; AKAP.2; RASSF1; MCP1; MCM2; GBP2; CD31; ER2; STAT1; TK1; ERBB2, CCNB1, BIRC5, STK6, MKI67, MYBL2, MMP11, CTSL2 and CD68; or the corresponding expression product, is determined, said report includes a prediction that said subject has an increased likelihood of response to chemotherapy.
The method may comprise the step of treating said patient with a chemotherapeutic agent.
The patient may be subjected to adjuvant chemotherapy.
The patient may be subjected to neoadjuvant chemotherapy.
The neoadjuvant chemotherapy may comprise the administration of a taxane derivative.
The taxane may be docetaxel or paclitaxel.
The chemotherapy may further comprise the administration of an additional anti-cancer agent.
The additional anti-cancer agent may be a member of the anthracycline class of anti-cancer agents.
The anti-cancer agent is doxorubicin.
The additional anti-cancer agent may be a topoisomerase inhibitor.
In an embodiment of the method if increased expression of one or more of TBP; ABCC5; GATA3; DICER1; MSH3; IRS1; TUBB; BAD; ERCC1; PR; APC; GGPS1; KRT18; ESRRG; AKT2; A.Catenin; CEGP1; NPD009; MAPK14; RUNX1; G.Catenin; FHIT; MTA1; ErbB4; FUS; BBC3; IGF1R; CD9; TP53BP1; MUC1; IGFBP5; rhoC; RALBP1; STAT3; BRK1; SGCB; DHPS; MGMT; CRIP2; ErbB3; RAP1GDS1; CCND1; PRKCD; Hepsin; AK055699; ZNF38; SEMA3F; COL1A1; BAG1; AKT1; COL1A2; Wnt.5a; PTPD1; RAB6C; GSTM1, BCL2, ESR1; or the corresponding expression product, is determined, said report includes a prediction that said subject has a decreased likelihood of response to chemotherapy.
The RNA transcript may comprise an intron-based sequence the expression of which correlates with the expression of a corresponding exon sequence.
According to another aspect of the present invention, there is provided a method for determining the likelihood of the response of a patient to chemotherapy, comprising:

(a) determining the expression levels of the RNA transcripts of following genes ACTB, BAG1, BCL2, CCNB1, CD68, SCUBE2, CTSL2, ESR1, GAPD, GRB7, GSTM1, GUSB, ERBB2, MKI67, MYBL2, PGR, RPLPO, STK6, MMP11, BIRC5, TFRC, or their expression products, and
(b) calculating the recurrence score (RS).

In an embodiment patients having an RS > 50 are in the upper 50 percentile of patients who are likely to respond to chemotherapy.
In an embodiment patients having an RS < 35 are in the lower 50 percentile of patients who are likely to response to chemotherapy.
RS may be determined by creating the following gene subsets:

(i) growth factor subset: GRB7 and HER2;
(ii) estrogen receptor subset: ER, PR, Bcl2, and CEGP1;
(iii) proliferation subset: SURV, Ki.67, MYBL2, CCNB1, and STK15; and
(iv) invasion subset: CTSL2, and STMY3;

wherein a gene within any of subsets (i)-(iv) can be substituted by substitute gene which coexpresses with said gene in said tumor with a Pearson correlation coefficient of ≥ 0.40; and
calculating the recurrence score (RS) for said subject by weighting the contributions of each of subsets (i) - (iv), to breast cancer recurrence.
The method may further comprise determining the RNA transcripts of CD68, GSTM1 and BAG1 or their expression products, or corresponding substitute genes or their expression products, and including the contribution of said genes or substitute genes to breast cancer recurrence in calculating the RS.

RS may be determined by using the following equation:

$$RS = (0.23 \text{ to } 0.70) \times GRB7axisthresh - (0.17 \text{ to } 0.55) \times ERaxis + (0.52 \text{ to } 1.56) \times prolifaxisthresh + (0.07 \text{ to } 0.21) \times invasionaxis + (0.03 \text{ to } 0.15) \times CD68 - (0.04 \text{ to } 0.25) \times GSTM1 - (0.05 \text{ to } 0.22) \times BAG1$$

wherein

(i) GRB7 axis = (0.45 to 1.35) x GRB7 + (0.05 to 0.15) x HER2;
(ii) if GRB7 axis < -2, then GRB7 axis thresh = -2, and if GRB7 axis ≥ -2, then GRB7 axis thresh = GRB7 axis;
(iii) ER axis = (Est1 + PR + Bcl2 + CEGP1)/4;
(iv) prolifaxis = (SURV + Ki.67 + MYBL2 + CCNB1 + STK15)/5;
(v) if prolifaxis < -3.5, then prolifaxisthresh = -3.5, if prolifaxis ≥ -3.5, then prolifaxishresh = prolifaxis; and
(vi) invasionaxis = (CTSL2 + STMY3)/2,

wherein the individual contributions of the genes in (iii), (iv) and (vi) are weighted by a factor of 0.5 to 1.5, and wherein a higher RS represents an increased likelihood of breast cancer recurrence.

RS may be determined by using the following equation:

$$\begin{aligned} RS\ (\text{range, } 0-100) = & + 0.47 \times \text{HER2 Group Score} \\ & - 0.34 \times \text{ER Group Score} \\ & + 1.04 \times \text{Proliferation Group Score} \\ & + 0.10 \times \text{Invasion Group Score} \\ & + 0.05 \times \text{CD68} \\ & - 0.08 \times \text{GSTM1} \\ & - 0.07 \times \text{BAG1} \end{aligned}$$

The RNA transcript may comprise an intron-based sequence the expression of which correlates with the expression of a corresponding exon sequence.

According to another aspect of the present invention, there is provided a method for predicting the response of a subject diagnosed with cancer to chemotherapy comprising determining the expression level of one prognostic RNA transcript or its expression product in a biological sample comprising cancer cells obtained from said subject, wherein the predictive RNA transcript is the transcript of BAG1, wherein for every unit of increased expression of BAG1 or the corresponding expression product, said subject is predicted to have a decreased likelihood of response to chemotherapy.

The cancer may be breast cancer and/or the subject may be a human patient.

The chemotherapy may be neoadjuvant chemotherapy, particularly wherein said neoadjuvant chemotherapy comprises the administration of a member or members of the anthracycline class of anti-cancer agents.

The neoadjuvant chemotherapy may comprise the administration of doxorubicin.

The neoadjuvant chemotherapy may comprise administration of a taxane derivative, particularly wherein the taxane is docetaxel and/or paclitaxel.

The biological sample may be fixed, paraffin-embedded, or fresh, or frozen.

The RNA may be isolated from a fixed, paraffin-embedded breast cancer tissue specimen of said patient.

The beneficial response may be a clinical complete response.

The beneficial response may be a pathological complete response.

The expression level of said RNA transcript may be determined by RT-PCR.

According to another aspect of the present invention, there is provided a method for determining the likelihood of the response of a patient to chemotherapy, comprising:

(a) determining the expression levels of the RNA transcripts of following genes BAG1, ACTB, BCL2, CCNB1, CD68, SCUBE2, CTSL2, ESR1, GAPD, GRB7, GSTM1, GUSB, ERBB2, MKI67, MYBL2, PGR, RPLPO, STK6, MMP11, BIRC5, TFRC, or their expression products, and
(b) calculating the recurrence score (RS).

RS may be determined by creating the following gene subsets:

(i) growth factor subset: GRB7 and HER2;
(ii) estrogen receptor subset: ER, PR, Bcl2, and CEGP1;
(iii) proliferation subset: SURV, Ki.67, MYBL2, CCNB1, and STK15; and
(iv) invasion subset: CTSL2, and STMY3;

wherein a gene within any of subsets (i)-(iv) can be substituted by substitute gene which coexpresses with said gene in said tumour with a Pearson correlation coefficient of ≥ 0.40; and
calculating the RS for said subject by weighting contributions of each of subsets (i)-(iv), to breast cancer recurrence.
The method may further comprise determining the RNA transcripts of CD68, GSTM1 and BAG1, or their expression products, or corresponding substitute genes or their expression products, and including the contribution of said genes or substitute genes to breast cancer recurrence in calculating the RS.
RS may be determined by using the following equation:

$$RS = (0.23 \text{ to } 0.70) \times GRB7axisthresh - (0.17 \text{ to } 0.55) \times ERaxis + (0.52 \text{ to } 1.56) \times prolifaxisthresh + (0.07 \text{ to } 0.21) \times invasionaxis + (0.03 \text{ to } 0.15) \times CD68 - (0.04 \text{ to } 0.25) \times GSTM1 - (0.05 \text{ to } 0.22) \times BAG1$$

wherein

(i) GRB7 axis = (0.45 to 1.35) x GRB7 + (0.05 to 0.15) x HER2;
(ii) if GRB7 axis < -2, then GRB7 axis thresh = -2, and if GRB7 axis ≥ -2, then GRB7 axis thresh = GRB7 axis;
(iii) ER axis = (Est1 +PR + Bcl2 + CEGP1)/4;
(iv) prolifaxis = (SURV + Ki.67 + MYBL2 +CCNB1 + STK15)/5;
(v) if prolifaxis < -3.5, then prolifaxisthresh = -3.5, if prolifaxis ≥ -3.5, then prolifaxisthresh = prolifaxis; and
(vi) invasionaxis = (CTSL2 + STMY3)/2,

wherein the individual contributions of the genes in (iii), (iv) and (vi) are weighted by a factor of 0.5 to 1.5, and wherein a higher RS represents an increased likelihood of breast cancer recurrence.
RS may be determined by using the following equation:

$$\begin{aligned} RS\ (\text{range}, 0-100) = \quad & +\ 0.47 \times \text{HER2 Group Score} \\ & -\ 0.34 \times \text{ER Group Score} \\ & +\ 1.04 \times \text{Proliferation Group Score} \\ & +\ 0.10 \times \text{Invasion Group Score} \\ & +\ 0.05 \times \text{CD68} \\ & -\ 0.08 \times \text{GSTM1} \\ & -\ 0.07 \times \text{BAG1} \end{aligned}$$

## Claims

1. A method for predicting likelihood of response to chemotherapy of a human patient diagnosed with breast cancer comprising;
   determining the expression level of an RNA transcript of GBP1 in a sample comprising cancer cells obtained from said patient before chemotherapy, and
   normalizing the level of the GBP1 RNA transcript to obtain a normalized GBP1 expression level,
   wherein the normalized GBP1 expression level from the patient is associated with an increased likelihood of response to chemotherapy if the normalised GBP1 expression level from the patient is increased compared to the normalised GBP1 expression level in breast cancer patients from samples with no subsequent pathologic complete response to chemotherapy.

2. The method as claimed in claim 1, wherein the cancer is invasive breast cancer.

3. The method as claimed in claim 1 or 2, wherein the chemotherapy is adjuvant chemotherapy.

4. The method as claimed in claim 1, 2 or 3, wherein the chemotherapy is neoadjuvant chemotherapy.

5. The method as claimed in claim 4, wherein the neoadjuvant chemotherapy comprises the administration of a taxane derivative.

6. The method as claimed in claim 5 wherein the taxane derivative is docetaxel or paclitaxel.

7. The method as claimed in claim 1 or 2, wherein the chemotherapy further comprises administration of an additional anti-cancer agent.

8. The method as claimed in claim 7, wherein the additional anti-cancer agent is a member of the anthracycline class of anti-cancer agents.

9. The method as claimed in claim 8 wherein the additional anti-cancer agent is doxorubicin.

10. The method as claimed in claim 7, wherein the additional anti-cancer agent is a topoisomerase inhibitor.

11. The method as claimed in any preceding claim wherein the sample is fixed, paraffin-embedded tissue (PET).

12. The method as claimed in any preceding claim, wherein the expression level of the RNA transcript is determined by a PCR-based method.

13. The method as claimed in claim 12, wherein the expression level of the RNA transcript is determined by RT-PCR.

## Patentansprüche

1. Verfahren zur Vorhersage der Wahrscheinlichkeit eines Ansprechens auf eine Chemotherapie bei einem menschlichen Patienten, der mit Brustkrebs diagnostiziert wurde, wobei das Verfahren Folgendes umfasst:

   Bestimmen des Expressionsniveaus eines RNA-Transkripts von GBP1 in einer Probe, die Krebszellen umfasst, die von dem Patienten vor einer Chemotherapie erhalten wurden, und
   Normieren des Niveaus des GBP1-RNA-Transkripts, um ein normiertes GBP1-Expressionsniveau zu erhalten, wobei das normierte GBP1-Expressionsniveau von dem Patienten mit einer erhöhten Wahrscheinlichkeit eines Ansprechens auf eine Chemotherapie assoziiert ist, wenn das normierte GBP1-Expressionsniveau von dem Patienten erhöht ist im Vergleich zu dem normierten GBP1-Expressionsniveau von Proben bei Brustkrebspatienten ohne ein anschließendes pathologisches komplettes Ansprechen auf eine Chemotherapie.

**2.** Verfahren nach Anspruch 1, wobei die Krebserkrankung invasiver Brustkrebs ist.

**3.** Verfahren nach Anspruch 1 oder 2, wobei die Chemotherapie adjuvante Chemotherapie ist.

**4.** Verfahren nach Anspruch 1, 2 oder 3, wobei die Chemotherapie neoadjuvante Chemotherapie ist.

**5.** Verfahren nach Anspruch 4, wobei die neoadjuvante Chemotherapie die Verabreichung eines Taxanderivats umfasst.

**6.** Verfahren nach Anspruch 5, wobei das Taxanderivat Docetaxel oder Paclitaxel ist.

**7.** Verfahren nach Anspruch 1 oder 2, wobei die Chemotherapie weiterhin eine Verabreichung eines zusätzlichen antineoplastischen Mittels umfasst.

**8.** Verfahren nach Anspruch 7, wobei das zusätzliche antineoplastische Mittel ein Mitglied der Anthracyclin-Klasse von antineoplastischen Mitteln ist.

**9.** Verfahren nach Anspruch 8, wobei das zusätzliche antineoplastische Mittel Doxorubicin ist.

**10.** Verfahren nach Anspruch 7, wobei das zusätzliche antineoplastische Mittel ein Topoisomerasehemmer ist.

**11.** Verfahren nach einem vorhergehenden Anspruch, wobei die Probe fixiertes, in Paraffin eingebettetes Gewebe (PET) ist.

**12.** Verfahren nach einem vorhergehenden Anspruch, wobei das Expressionsniveau des RNA-Transkripts mittels eines PCR-basierten Verfahrens bestimmt wird.

**13.** Verfahren nach Anspruch 12, wobei das Expressionsniveau des RNA-Transkripts mittels RT-PCR bestimmt wird.

## Revendications

**1.** Procédé de prédiction de la probabilité de réponse à une chimiothérapie d'un patient humain chez qui un cancer du sein a été diagnostiqué, le procédé consistant à:

déterminer le niveau d'expression d'un transcrit d'ARN de GBP1 dans un échantillon comprenant des cellules cancéreuses obtenues dudit patient avant chimiothérapie ; et
normaliser le niveau du transcrit d'ARN de GBP1 afin d'obtenir un niveau d'expression de GBP1 normalisé ;
le niveau d'expression de GBP1 normalisé du patient étant associé à une augmentation de la probabilité de réponse à une chimiothérapie si le niveau d'expression de GBP1 normalisé du patient est augmenté par comparaison avec le niveau d'expression de GBP1 normalisé chez des patients atteints d'un cancer du sein dans des échantillons sans réponse complète pathologique à une chimiothérapie.

**2.** Procédé selon la revendication 1, dans lequel le cancer est un cancer du sein invasif.

**3.** Procédé selon la revendication 1 ou 2, dans lequel la chimiothérapie est une chimiothérapie adjuvante.

**4.** Procédé selon la revendication 1, 2 ou 3, dans lequel la chimiothérapie est une chimiothérapie néo-adjuvante.

**5.** Procédé selon la revendication 4, dans lequel la chimiothérapie néo-adjuvante comprend l'administration d'un dérivé de taxane.

**6.** Procédé selon la revendication 5, dans lequel le dérivé de taxane est le docétaxel ou le paclitaxel.

**7.** Procédé selon la revendication 1 ou 2, dans lequel la chimiothérapie comprend en outre l'administration d'un agent anticancéreux supplémentaire.

**8.** Procédé selon la revendication 7, dans lequel l'agent anticancéreux supplémentaire est un membre de la classe d'anthracyclines d'agents anticancéreux.

**9.** Procédé selon la revendication 8, dans lequel l'agent anticancéreux supplémentaire est la doxorubicine.

**10.** Procédé selon la revendication 7, dans lequel l'agent anticancéreux supplémentaire est un inhibiteur de topoisomérase.

**11.** Procédé selon l'une quelconque des revendications précédentes, dans lequel l'échantillon est un tissu enrobé à la paraffine (PET) et fixé.

**12.** Procédé selon l'une quelconque des revendications précédentes, dans lequel le niveau d'expression du transcrit d'ARN est déterminé par un procédé basé sur une PCR.

**13.** Procédé selon la revendication 12, dans lequel le niveau d'expression du transcrit d'ARN est déterminé par RT-PCR.

FIGURE 1

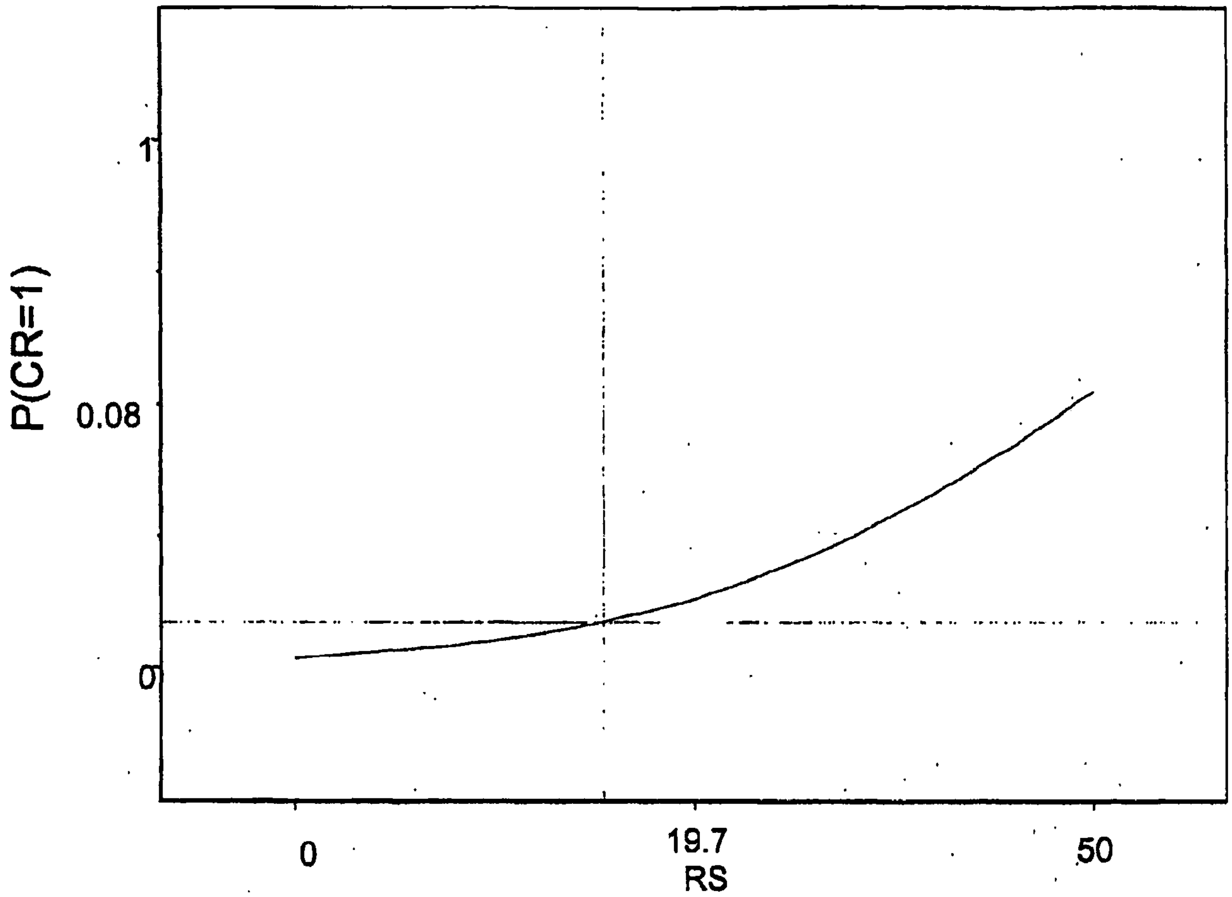

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 60486302 A
- US 486302 P
- US 09014283 B
- US 60561035 B


### Non-patent literature cited in the description


- **GOLUB et al.** *Science,* 1999, vol. 286, 531-537
- **BHATTACHARJAE et al.** *Proc. Natl. Acad. Sci. USA,* 2001, vol. 98, 13790-13795
- **CHEN-HSIANG et al.** *Bioinformatics,* 2001, vol. 17 (1), S316-S322
- **RAMASWAMY et al.** *Proc. Natl. Acad. Sci. USA,* 2001, vol. 98, 15149-15154
- **MARTIN et al.** *Cancer Res.,* 2000, vol. 60, 2232-2238
- **WEST et al.** *Proc. Natl. Acad. Sci. USA,* 2001, vol. 98, 11462-11467
- **SORLIE et al.** *Proc. Natl. Acad. Sci. USA,* 2001, vol. 98, 10869-10874
- **YAN et al.** *Cancer Res.,* 2001, vol. 61, 8375-8380
- **PEROU et al.** *Nature,* 2000, vol. 406, 747-752
- **SINGLETON et al.** Dictionary of Microbiology and Molecular Biology. J. Wiley & Sons, 1994
- **MARCH.** Advanced Organic Chemistry Reactions, Mechanisms and Structure. John Wiley & Sons, 1992
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **SAMBROOK et al.** Molecular Cloning: A Laboratory Manual. 1989
- Oligonucleotide Synthesis. 1984
- Animal Cell Culture. 1987
- Methods in Enzymology. Academic Press, Inc,
- Handbook of Experimental Immunology. Blackwell Science Inc, 1987
- Gene Transfer Vectors for Mammalian Cells. 1987
- Current Protocols in Molecular Biology. 1987
- PCR: The Polymerase Chain Reaction. 1994
- **PARKER ; BARNES.** *Methods in Molecular Biology,* 1999, vol. 106, 247-283
- **HOD.** *Biotechniques,* 1992, vol. 13, 852-854
- **WEIS et al.** *Trends in Genetics,* 1992, vol. 8, 263-264
- **AUSUBEL et al.** Current Protocols of Molecular Biology. John Wiley and Sons, 1997
- **RUPP ; LOCKER.** *Lab Invest.,* 1987, vol. 56, A67
- **DE ANDRÉS et al.** *BioTechniques,* 1995, vol. 18, 42044
- **HELD et al.** *Genome Research,* 1996, vol. 6, 986-994
- **DING ; CANTOR.** *Proc. Natl. Acad. Sci. USA,* 2003, vol. 100, 3059-3064
- **LIANG ; PARDEE.** *Science,* 1992, vol. 257, 967-971
- **KAWAMOTO et al.** *Genome Res.,* 1999, vol. 12, 1305-1312
- **OLIPHANT et al.** *Discovery of Markers for Disease (Supplement to Biotechniques),* June 2002
- **FERGUSON et al.** *Analytical Chemistry,* 2000, vol. 72, 5618
- **YANG et al.** *Genome Res.,* 2001, vol. 11, 1888-1898
- **FUKUMURA et al.** *Nucl. Acids. Res.,* 2003, vol. 31 (16), e94
- **SCHENA et al.** *Proc. Natl. Acad. Sci. USA,* 1996, vol. 93 (2), 106-149
- **VELCULOSCU et al.** *Science,* 1995, vol. 270, 484-487
- **VELCULESCU et al.** *Cell,* 1997, vol. 88, 243-51
- **BRENNER et al.** *Nature Biotechnology,* 2000, vol. 18, 630-634
- **T.E. GODFREY et al.** *J. Molec. Diagnostics,* 2000, vol. 2, 84-91
- **K. SPECHT et al.** *Am. J. Pathol.,* 2001, vol. 158, 419-29
- **FISHER et al.** *J. Clin. Oncology,* 1997, vol. 15, 2002-2004
- **FISHER et al.** *J. Clin. Oncology,* 1998, vol. 16, 2672-2685
- **VAN DER HAGE et al.** *J. Clin. Oncol.,* 2001, vol. 19, 4224-4237
- **HOLMES et al.** *J. Natl. Cancer Inst.,* 1991, vol. 83, 1797-1805

- **MOLITERNI et al.** *Seminars in Oncology,* 1999, vol. 24, 17-10, S-17-14
- **CLEATOR et al.** *Endocrine-Related Cancer,* 2002, vol. 9, 183-195

SEQUENCE LISTING

<110> GENOMIC HEALTH, INC. FONDAZIONE IRCCS ISTITUTO NAZIONALE DEI TUMORI

<120> GENE EXPRESSION MARKERS FOR PREDICTING RESPONSE TO CHEMOTHERAPY

<130> KE/N32061

<140> Divisional of 09014283.7
<141> 2005-04-07

<150> US 60/561,035
<151> 2004-04-09

<160> 340

<170> FastSEQ for Windows Version 4.0

<210> 1
<211> 23
<212> DNA
<213> Artificial Sequence

<220>
<223> Synthetic Oligonucleotide Forward Primer

<400> 1
cgttccgatc ctctatactg cat 23

<210> 2
<211> 22
<212> DNA
<213> Artificial Sequence

<220>
<223> Synthetic Oligonucleotide Reverse Primer

<400> 2
aggtccctgt tggccttata gg 22

<210> 3
<211> 25
<212> DNA
<213> Artificial Sequence

<220>
<223> Synthetic Oligonucleotide Probe

<400> 3
atgcctacag caccctgatg tcgca 25

<210> 4
<211> 20
<212> DNA
<213> Artificial Sequence

<220>
<223> Synthetic Oligonucleotide Forward Primer

<400> 4
tgcagactgt accatgctga 20

<210> 5
<211> 20

<212> DNA
<213> Artificial Sequence

<220>
<223> Synthetic Oligonucleotide Reverse Primer

<400> 5
ggccagcacc ataatcctat 20

<210> 6
<211> 22
<212> DNA
<213> Artificial Sequence

<220>
<223> Synthetic Oligonucleotide Probe

<400> 6
ctgcacacgg ttctaggctc cg 22

<210> 7
<211> 27
<212> DNA
<213> Artificial Sequence

<220>
<223> Synthetic Oligonucleotide Forward Primer

<400> 7
ctgcatgtga ttgaataaga aacaaga 27

<210> 8
<211> 22
<212> DNA
<213> Artificial Sequence

<220>
<223> Synthetic Oligonucleotide Reverse Primer

<400> 8
tgtggacctg atccctgtac ac 22

<210> 9
<211> 23
<212> DNA
<213> Artificial Sequence

<220>
<223> Synthetic Oligonucleotide Probe

<400> 9
tgaccacacc aaagcctccc tgg 23

<210> 10
<211> 20
<212> DNA
<213> Artificial Sequence

<220>
<223> Synthetic Oligonucleotide Forward Primer

<400> 10
acgaattgtc ggtgaggtct 20

<210> 11
<211> 20
<212> DNA
<213> Artificial Sequence

<220>
<223> Synthetic Oligonucleotide Reverse Primer

<400> 11
gtccatgctg aaatcattgg 20

<210> 12
<211> 25
<212> DNA
<213> Artificial Sequence

<220>
<223> Synthetic Oligonucleotide Probe

<400> 12
caggatacca cagtcctgga gaccc 25

<210> 13
<211> 20
<212> DNA
<213> Artificial Sequence

<220>
<223> Synthetic Oligonucleotide Forward Primer

<400> 13
cgcttctatg gcgctgagat 20

<210> 14
<211> 20
<212> DNA
<213> Artificial Sequence

<220>
<223> Synthetic Oligonucleotide Reverse Primer

<400> 14
tcccggtaca ccacgttctt 20

<210> 15
<211> 24
<212> DNA
<213> Artificial Sequence

<220>
<223> Synthetic Oligonucleotide Probe

<400> 15
cagccctgga ctacctgcac tcgg 24

<210> 16
<211> 19
<212> DNA
<213> Artificial Sequence

<220>
<223> Synthetic Oligonucleotide Forward Primer

<400> 16
tcctgccacc cttcaaacc 19

<210> 17
<211> 21
<212> DNA
<213> Artificial Sequence

<220>
<223> Synthetic Oligonucleotide Reverse Primer

<400> 17
ggcggtaaat tcatcatcga a 21

<210> 18
<211> 24
<212> DNA
<213> Artificial Sequence

<220>
<223> Synthetic Oligonucleotide Probe

<400> 18
caggtcacgt ccgaggtcga caca 24

<210> 19
<211> 20
<212> DNA
<213> Artificial Sequence

<220>
<223> Synthetic Oligonucleotide Forward Primer

<400> 19
ggacagcagg aatgtgtttc 20

<210> 20
<211> 20
<212> DNA
<213> Artificial Sequence

<220>
<223> Synthetic Oligonucleotide Reverse Primer

<400> 20
acccactcga tttgtttctg 20

<210> 21
<211> 22
<212> DNA
<213> Artificial Sequence

<220>
<223> Synthetic Oligonucleotide Probe

<400> 21
cattggctcc ccgtgacctg ta 22
<210> 22
<211> 19
<212> DNA
<213> Artificial Sequence

<220>
<223> Synthetic Oligonucleotide Forward Primer

<400> 22
gggtcaggtg cctcgagat 19

<210> 23
<211> 21
<212> DNA
<213> Artificial Sequence

<220>
<223> Synthetic Oligonucleotide Reverse Primer

<400> 23
ctgctcactc ggctcaaact c 21

<210> 24
<211> 24
<212> DNA
<213> Artificial Sequence

<220>
<223> Synthetic Oligonucleotide Probe

<400> 24
tgggcccaga gcatgttcca gatc 24

<210> 25
<211> 23
<212> DNA
<213> Artificial Sequence

<220>
<223> Synthetic Oligonucleotide Forward Primer

<400> 25
cgttgtcagc acttggaata caa 23

<210> 26
<211> 24
<212> DNA
<213> Artificial Sequence

<220>
<223> Synthetic Oligonucleotide Reverse Primer

<400> 26
gttcaacctc ttcctgtgga ctgt 24

<210> 27
<211> 26
<212> DNA
<213> Artificial Sequence

<220>
<223> Synthetic Oligonucleotide Probe

<400> 27
cccaattaac atgacccggc aaccat 26

<210> 28
<211> 20
<212> DNA
<213> Artificial Sequence

<220>
<223> Synthetic Oligonucleotide Forward Primer

<400> 28
cctggagggt cctgtacaat 20

<210> 29
<211> 19
<212> DNA
<213> Artificial Sequence

<220>
<223> Synthetic Oligonucleotide Reverse Primer

<400> 29
ctaattgggc tccatctcg 19

<210> 30
<211> 24
<212> DNA
<213> Artificial Sequence

<220>
<223> Synthetic Oligonucleotide Probe

<400> 30
catcatggga ctcctgccct tacc 24

<210> 31
<211> 25
<212> DNA
<213> Artificial Sequence

<220>
<223> Synthetic Oligonucleotide Forward Primer

<400> 31
cagatggacc tagtacccac tgaga 25

<210> 32
<211> 24
<212> DNA
<213> Artificial Sequence

<220>
<223> Synthetic Oligonucleotide Reverse Primer

<400> 32
cctatgattt aagggcattt ttcc 24

<210> 33
<211> 22
<212> DNA
<213> Artificial Sequence

<220>
<223> Synthetic Oligonucleotide Probe

<400> 33
ttccacgccg aaggacagcg at 22

<210> 34
<211> 21
<212> DNA
<213> Artificial Sequence

<220>
<223> Synthetic Oligonucleotide Forward Primer

<400> 34
gcatgttcgt ggcctctaag a 21

<210> 35
<211> 22
<212> DNA
<213> Artificial Sequence

<220>
<223> Synthetic Oligonucleotide Reverse Primer

<400> 35
cggtgtagat gcacagcttc tc 22

<210> 36
<211> 23
<212> DNA
<213> Artificial Sequence

<220>
<223> Synthetic Oligonucleotide Probe

<400> 36
aaggagacca tccccctgac ggc 23

<210> 37
<211> 20
<212> DNA
<213> Artificial Sequence

<220>
<223> Synthetic Oligonucleotide Forward Primer

<400> 37
cgtcaggacc caccatgtct 20

<210> 38
<211> 24
<212> DNA
<213> Artificial Sequence

<220>
<223> Synthetic Oligonucleotide Reverse Primer

<400> 38
ggttaattgg tgacatcctc aaga 24

<210> 39
<211> 20
<212> DNA
<213> Artificial Sequence

<220>
<223> Synthetic Oligonucleotide Probe

<400> 39
cgcggccgag acatggcttg 20

<210> 40
<211> 25
<212> DNA
<213> Artificial Sequence

<220>
<223> Synthetic Oligonucleotide Forward Primer

<400> 40
tgtatttcaa gacctctgtg cactt 25

<210> 41
<211> 23
<212> DNA
<213> Artificial Sequence

<220>
<223> Synthetic Oligonucleotide Reverse Primer

<400> 41
ttagcctgag gaattgctgt gtt 23

<210> 42
<211> 25
<212> DNA

<213> Artificial Sequence

<220>
<223> Synthetic Oligonucleotide Probe

<400> 42
tttatgaacc tgccctgctc ccaca 25

<210> 43
<211> 20
<212> DNA
<213> Artificial Sequence

<220>
<223> Synthetic Oligonucleotide Forward Primer

<400> 43
agatgaagtg gaaggcgctt 20

<210> 44
<211> 21
<212> DNA
<213> Artificial Sequence

<220>
<223> Synthetic Oligonucleotide Reverse Primer

<400> 44
tgcctctgta atcggcaact g 21

<210> 45
<211> 18
<212> DNA
<213> Artificial Sequence

<220>
<223> Synthetic Oligonucleotide Probe

<400> 45
caccgcggcc atcctgca 18

<210> 46
<211> 20
<212> DNA
<213> Artificial Sequence

<220>
<223> Synthetic Oligonucleotide Forward Primer

<400> 46
gggcgtggaa cagtttatct 20

<210> 47
<211> 19
<212> DNA
<213> Artificial Sequence

<220>

<223> Synthetic Oligonucleotide Reverse Primer

<400> 47
cacggtgaag gtttcgagt 19

<210> 48
<211> 24
<212> DNA
<213> Artificial Sequence

<220>
<223> Synthetic Oligonucleotide Probe

<400> 48
agacatctgc cccaagaagg acgt 24

<210> 49
<211> 21
<212> DNA
<213> Artificial Sequence

<220>
<223> Synthetic Oligonucleotide Forward Primer

<400> 49
tggattggag ttctgggaat g 21

<210> 50
<211> 22
<212> DNA
<213> Artificial Sequence

<220>
<223> Synthetic Oligonucleotide Reverse Primer

<400> 50
gcttgcactc cacaggtaca ca 22

<210> 51
<211> 23
<212> DNA
<213> Artificial Sequence

<220>
<223> Synthetic Oligonucleotide Probe

<400> 51
actggccgtg gcactggaca aca 23

<210> 52
<211> 21
<212> DNA
<213> Artificial Sequence

<220>
<223> Synthetic Oligonucleotide Forward Primer

<400> 52

aaacgagcag tttgccatca g 21

<210> 53
<211> 20
<212> DNA
<213> Artificial Sequence

<220>
<223> Synthetic Oligonucleotide Reverse Primer

<400> 53
gttggtgatg ttccgaagca 20

<210> 54
<211> 24
<212> DNA
<213> Artificial Sequence

<220>
<223> Synthetic Oligonucleotide Probe

<400> 54
cctcaccggc atagactgga agcg 24

<210> 55
<211> 21
<212> DNA
<213> Artificial Sequence

<220>
<223> Synthetic Oligonucleotide Forward Primer

<400> 55
tgacaatcag cacacctgca t 21

<210> 56
<211> 23
<212> DNA
<213> Artificial Sequence

<220>
<223> Synthetic Oligonucleotide Reverse Primer

<400> 56
tgtgactaca gccgtgatcc tta 23

<210> 57
<211> 20
<212> DNA
<213> Artificial Sequence

<220>
<223> Synthetic Oligonucleotide Probe

<400> 57
caggccctct tccgagcggt 20

<210> 58

<211> 20
<212> DNA
<213> Artificial Sequence

<220>
<223> Synthetic Oligonucleotide Forward Primer

<400> 58
ctgaaggagc tccaagacct 20

<210> 59
<211> 20
<212> DNA
<213> Artificial Sequence

<220>
<223> Synthetic Oligonucleotide Reverse Primer

<400> 59
caaaaccgct gtgtttcttc 20

<210> 60
<211> 22
<212> DNA
<213> Artificial Sequence

<220>
<223> Synthetic Oligonucleotide Probe

<400> 60
tgctgatgtg ccctctcctt gg 22
<210> 61
<211> 18
<212> DNA
<213> Artificial Sequence

<220>
<223> Synthetic Oligonucleotide Forward Primer

<400> 61
gtggccatcc agctgacc 18

<210> 62
<211> 23
<212> DNA
<213> Artificial Sequence

<220>
<223> Synthetic Oligonucleotide Reverse Primer

<400> 62
cagtggtagg tgatgttctg gga 23

<210> 63
<211> 21
<212> DNA
<213> Artificial Sequence

<220>
<223> Synthetic Oligonucleotide Probe

<400> 63
tcctgcgcct gatgtccacc g 21

<210> 64
<211> 24
<212> DNA
<213> Artificial Sequence

<220>
<223> Synthetic Oligonucleotide Forward Primer

<400> 64
cagccaagaa ctggtatagg agct 24

<210> 65
<211> 19
<212> DNA
<213> Artificial Sequence

<220>
<223> Synthetic Oligonucleotide Reverse Primer

<400> 65
aaactggctg ccagcattg 19

<210> 66
<211> 30
<212> DNA
<213> Artificial Sequence

<220>
<223> Synthetic Oligonucleotide Probe

<400> 66
tctcctagcc agacgtgttt cttgtccttg 30

<210> 67
<211> 24
<212> DNA
<213> Artificial Sequence

<220>
<223> Synthetic Oligonucleotide Forward Primer

<400> 67
cgacagttgc gatgaaagtt ctaa 24

<210> 68
<211> 22
<212> DNA
<213> Artificial Sequence

<220>
<223> Synthetic Oligonucleotide Reverse Primer

<400> 68
ggctgctaga gaccatggac at 22

<210> 69
<211> 27
<212> DNA
<213> Artificial Sequence

<220>
<223> Synthetic Oligonucleotide Probe

<400> 69
cctcctcctg ttgctgccac taatgct 27

<210> 70
<211> 20
<212> DNA
<213> Artificial Sequence

<220>
<223> Synthetic Oligonucleotide Forward Primer

<400> 70
aacttcaagg tcggagaagg 20

<210> 71
<211> 20
<212> DNA
<213> Artificial Sequence

<220>
<223> Synthetic Oligonucleotide Reverse Primer

<400> 71
tggctaaact cctgcacttg 20

<210> 72
<211> 21
<212> DNA
<213> Artificial Sequence

<220>
<223> Synthetic Oligonucleotide Probe

<400> 72
ccgtccacgg tctcctcctc a 21

<210> 73
<211> 18
<212> DNA
<213> Artificial Sequence

<220>
<223> Synthetic Oligonucleotide Foward Primer

<400> 73
gtgctacgcc accctgtt 18

<210> 74
<211> 20
<212> DNA
<213> Artificial Sequence

<220>
<223> Synthetic Oligonucleotide Reverse Primer

<400> 74
caggggcttc tcgtagatgt 20

<210> 75
<211> 22
<212> DNA
<213> Artificial Sequence

<220>
<223> Synthetic Oligonucleotide Probe

<400> 75
ccgatgttca cgcctttggg tc 22

<210> 76
<211> 19
<212> DNA
<213> Artificial Sequence

<220>
<223> Synthetic Oligonucleotide Foward Primer

<400> 76
ggtgcctact ccattgtgg 19

<210> 77
<211> 20
<212> DNA
<213> Artificial Sequence

<220>
<223> Synthetic Oligonucleotide Reverse Primer

<400> 77
gtggagccct tcttcctctt 20

<210> 78
<211> 24
<212> DNA
<213> Artificial Sequence

<220>
<223> Synthetic Oligonucleotide Probe

<400> 78
tactccagca ggcacacaaa cacg 24

<210> 79
<211> 22
<212> DNA

<213> Artificial Sequence

<220>
<223> Synthetic Oligonucleotide Forward Primer

<400> 79
gggagaacgg gatcaatagg at 22

<210> 80
<211> 21
<212> DNA
<213> Artificial Sequence

<220>
<223> Synthetic Oligonucleotide Reverse Primer

<400> 80
gcatcagcca gtcctcaaac t 21

<210> 81
<211> 24
<212> DNA
<213> Artificial Sequence

<220>
<223> Synthetic Oligonucleotide Probe

<400> 81
ctcattgggc accagcaggt ttcc 24

<210> 82
<211> 20
<212> DNA
<213> Artificial Sequence

<220>
<223> Synthetic Oligonucleotide Forward Primer

<400> 82
tccaattcca gcatcactgt 20

<210> 83
<211> 20
<212> DNA
<213> Artificial Sequence

<220>
<223> Synthetic Oligonucleotide Reverse Primer

<400> 83
ggcagtgaag gcgataaagt 20
<210> 84
<211> 25
<212> DNA
<213> Artificial Sequence

<220>
<223> Synthetic Oligonucleotide Probe

<400> 84
agaaaagctg tttgtctccc cagca 25

<210> 85
<211> 21
<212> DNA
<213> Artificial Sequence

<220>
<223> Synthetic Oligonucleotide Forward Primer

<400> 85
tgcacagagg gtgtgggtta c 21

<210> 86
<211> 28
<212> DNA
<213> Artificial Sequence

<220>
<223> Synthetic Oligonucleotide Reverse Primer

<400> 86
tcttcatctg atttacaagc tgtacatg 28

<210> 87
<211> 29
<212> DNA
<213> Artificial Sequence

<220>
<223> Synthetic Oligonucleotide Probe

<400> 87
caatgcttcc aacaatttgt ttgcttgcc 29

<210> 88
<211> 20
<212> DNA
<213> Artificial Sequence

<220>
<223> Synthetic Oligonucleotide Forward Primer

<400> 88
actccctcta cccttgagca 20

<210> 89
<211> 20
<212> DNA
<213> Artificial Sequence

<220>
<223> Synthetic Oligonucleotide Reverse Primer

<400> 89
caggcctcag ttccttcagt 20

<210> 90
<211> 24
<212> DNA
<213> Artificial Sequence

<220>
<223> Synthetic Oligonucleotide Probe

<400> 90
cagaagaaca gctcagggac ccct 24

<210> 91
<211> 20
<212> DNA
<213> Artificial Sequence

<220>
<223> Synthetic Oligonucleotide Forward Primer

<400> 91
tggtccatcg ccagttatca 20

<210> 92
<211> 23
<212> DNA
<213> Artificial Sequence

<220>
<223> Synthetic Oligonucleotide Reverse Primer

<400> 92
tgttctagcg atcttgcttc aca 23

<210> 93
<211> 30
<212> DNA
<213> Artificial Sequence

<220>
<223> Synthetic Oligonucleotide Probe

<400> 93
atctgtatgc ggaacctcaa aagagtccct 30

<210> 94
<211> 23
<212> DNA
<213> Artificial Sequence

<220>
<223> Synthetic Oligonucleotide Forward Primer

<400> 94
cggttatgtc atgccagata cac 23

<210> 95
<211> 24
<212> DNA

<213> Artificial Sequence

<220>
<223> Synthetic Oligonucleotide Reverse Primer

<400> 95
gaactgagac ccactgaaga aagg 24

<210> 96
<211> 25
<212> DNA
<213> Artificial Sequence

<220>
<223> Synthetic Oligonucleotide Probe

<400> 96
cctcaaaggt actccctcct cccgg 25

<210> 97
<211> 25
<212> DNA
<213> Artificial Sequence

<220>
<223> Synthetic Oligonucleotide Forward Primer

<400> 97
tggctcttaa tcagtttcgt tacct 25

<210> 98
<211> 25
<212> DNA
<213> Artificial Sequence

<220>
<223> Synthetic Oligonucleotide Reverse Primer

<400> 98
caaggcatat cgatcctcat aaagt 25

<210> 99
<211> 30
<212> DNA
<213> Artificial Sequence

<220>
<223> Synthetic Oligonucleotide Probe

<400> 99
tgtcccacga ataatgcgta aattctccag 30

<210> 100
<211> 20
<212> DNA
<213> Artificial Sequence

<220>

<223> Synthetic Oligonucleotide Forward Primer

<400> 100
gtccaggtgg atgtgaaaga 20

<210> 101
<211> 20
<212> DNA
<213> Artificial Sequence

<220>
<223> Synthetic Oligonucleotide Reverse Primer

<400> 101
cggccaggat acacatctta 20

<210> 102
<211> 21
<212> DNA
<213> Artificial Sequence

<220>
<223> Synthetic Oligonucleotide Probe

<400> 102
cagcaggccc tcaaggagct g 21

<210> 103
<211> 20
<212> DNA
<213> Artificial Sequence

<220>
<223> Synthetic Oligonucleotide Forward Primer

<400> 103
acggatcaca gtggaggaag 20

<210> 104
<211> 20
<212> DNA
<213> Artificial Sequence

<220>
<223> Synthetic Oligonucleotide Reverse Primer

<400> 104
ctcatccgtc gggtcatagt 20

<210> 105
<211> 20
<212> DNA
<213> Artificial Sequence

<220>
<223> Synthetic Oligonucleotide Probe

<400> 105

cgctggctca cccctacctg 20

<210> 106
<211> 20
<212> DNA
<213> Artificial Sequence

<220>
<223> Synthetic Oligonucleotide Forward Primer

<400> 106
ccagcaccat tgttgaagat 20

<210> 107
<211> 20
<212> DNA
<213> Artificial Sequence

<220>
<223> Synthetic Oligonucleotide Reverse Primer

<400> 107
agtctcttgg gcatcgagtt 20

<210> 108
<211> 26
<212> DNA
<213> Artificial Sequence

<220>
<223> Synthetic Oligonucleotide Probe

<400> 108
ccccagacca agtgtgaata catgct 26

<210> 109
<211> 24
<212> DNA
<213> Artificial Sequence

<220>
<223> Synthetic Oligonucleotide Forward Primer

<400> 109
gcactttggg attctttcca ttat 24

<210> 110
<211> 24
<212> DNA
<213> Artificial Sequence

<220>
<223> Synthetic Oligonucleotide Reverse Primer

<400> 110
gcatgtaaga agaccctcac tgaa 24

<210> 111

<211> 29
<212> DNA
<213> Artificial Sequence

<220>
<223> Synthetic Oligonucleotide Probe

<400> 111
acaacattct cggtgcctgt aacaaagaa 29

<210> 112
<211> 25
<212> DNA
<213> Artificial Sequence

<220>
<223> Synthetic Oligonucleotide Reverse Primer

<400> 112
ggattgtaga ctgtcaccga aattc 25

<210> 113
<211> 28
<212> DNA
<213> Artificial Sequence

<220>
<223> Synthetic Oligonucleotide Forward Primer

<400> 113
ggctattcct cattttctct acaaagtg 28

<210> 114
<211> 30
<212> DNA
<213> Artificial Sequence

<220>
<223> Synthetic Oligonucleotide Probe

<400> 114
cctccaggag gctaccttct tcatgttcac 30

<210> 115
<211> 18
<212> DNA
<213> Artificial Sequence

<220>
<223> Synthetic Oligonucleotide Forward Primer

<400> 115
ccagtggagc gcttccat 18

<210> 116
<211> 22
<212> DNA
<213> Artificial Sequence

<220>
<223> Synthetic Oligonucleotide Reverse Primer

<400> 116
ctctctgggt cgtctgaaac aa 22

<210> 117
<211> 23
<212> DNA
<213> Artificial Sequence

<220>
<223> Synthetic Oligonucleotide Probe

<400> 117
tcggccactt catcaggacg cag 23

<210> 118
<211> 26
<212> DNA
<213> Artificial Sequence

<220>
<223> Synthetic Oligonucleotide Forward Primer

<400> 118
ggataattca gacaacaaca ccatct 26

<210> 119
<211> 25
<212> DNA
<213> Artificial Sequence

<220>
<223> Synthetic Oligonucleotide Reverse Primer

<400> 119
tgaagtaatc agccacagac tcaat 25

<210> 120
<211> 29
<212> DNA
<213> Artificial Sequence

<220>
<223> Synthetic Oligonucleotide Probe

<400> 120
tcaattgtaa cattctcacc caggccttg 29

<210> 121
<211> 20
<212> DNA
<213> Artificial Sequence

<220>
<223> Synthetic Oligonucleotide Forward Primer

<400> 121
gaagcgcaga tcatgaagaa 20

<210> 122
<211> 20
<212> DNA
<213> Artificial Sequence

<220>
<223> Synthetic Oligonucleotide Reverse Primer

<400> 122
ctcctcagac accactgcat 20

<210> 123
<211> 24
<212> DNA
<213> Artificial Sequence

<220>
<223> Synthetic Oligonucleotide Probe

<400> 123
ctgaagcacg acaagctggt ccag 24
<210> 124
<211> 19
<212> DNA
<213> Artificial Sequence

<220>
<223> Synthetic Oligonucleotide Forward Primer

<400> 124
tcagcagcaa gggcatcat 19

<210> 125
<211> 23
<212> DNA
<213> Artificial Sequence

<220>
<223> Synthetic Oligonucleotide Reverse Primer

<400> 125
ggtggttttc ttgagcgtgt act 23

<210> 126
<211> 19
<212> DNA
<213> Artificial Sequence

<220>
<223> Synthetic Oligonucleotide Probe

<400> 126
cgcccgcagg cctcatcct 19

<210> 127

<211> 23
<212> DNA
<213> Artificial Sequence

<220>
<223> Synthetic Oligonucleotide Forward Primer

<400> 127
caaaggagct cactgtggtg tct 23

<210> 128
<211> 26
<212> DNA
<213> Artificial Sequence

<220>
<223> Synthetic Oligonucleotide Reverse Primer

<400> 128
gagtcagaat ggcttattca cagatg 26

<210> 129
<211> 24
<212> DNA
<213> Artificial Sequence

<220>
<223> Synthetic Oligonucleotide Probe

<400> 129
tgttccaacc actgaatctg gacc 24

<210> 130
<211> 20
<212> DNA
<213> Artificial Sequence

<220>
<223> Synthetic Oligonucleotide Forward Primer

<400> 130
ttgggaaata tttgggcatt 20

<210> 131
<211> 20
<212> DNA
<213> Artificial Sequence

<220>
<223> Synthetic Oligonucleotide Reverse Primer

<400> 131
agaagctagg gtggttgtcc 20

<210> 132
<211> 26
<212> DNA
<213> Artificial Sequence

<220>
<223> Synthetic Oligonucleotide Probe

<400> 132
ttgggacatt gtagacttgg ccagac 26

<210> 133
<211> 19
<212> DNA
<213> Artificial Sequence

<220>
<223> Synthetic Oligonucleotide Forward Primer

<400> 133
gcatgggaac catcaacca 19

<210> 134
<211> 21
<212> DNA
<213> Artificial Sequence

<220>
<223> Synthetic Oligonucleotide Reverse Primer

<400> 134
tgaggagttt gccttgattc g 21

<210> 135
<211> 30
<212> DNA
<213> Artificial Sequence

<220>
<223> Synthetic Oligonucleotide Probe

<400> 135
ccatggacca acttcactat gtgacagagc 30

<210> 136
<211> 18
<212> DNA
<213> Artificial Sequence

<220>
<223> Synthetic Oligonucleotide Forward Primer

<400> 136
ctccgacgtg gctttcca 18

<210> 137
<211> 23
<212> DNA
<213> Artificial Sequence

<220>
<223> Synthetic Oligonucleotide Reverse Primer

<400> 137
cgtaattggc agaattgatg aca 23

<210> 138
<211> 25
<212> DNA
<213> Artificial Sequence

<220>
<223> Synthetic Oligonucleotide Probe

<400> 138
tggcccacag catctatgga atccc 25

<210> 139
<211> 20
<212> DNA
<213> Artificial Sequence

<220>
<223> Synthetic Oligonucleotide Forward Primer

<400> 139
ccatctgcat ccatcttgtt 20

<210> 140
<211> 20
<212> DNA
<213> Artificial Sequence

<220>
<223> Synthetic Oligonucleotide Reverse Primer

<400> 140
ggccaccagg gtattatctg 20

<210> 141
<211> 23
<212> DNA
<213> Artificial Sequence

<220>
<223> Synthetic Oligonucleotide Probe

<400> 141
ctccccaccc ttgagaagtg cct 23

<210> 142
<211> 20
<212> DNA
<213> Artificial Sequence

<220>
<223> Synthetic Oligonucleotide Forward Primer

<400> 142
aggctgctgg aggtcatctc 20

<210> 143
<211> 19
<212> DNA
<213> Artificial Sequence

<220>
<223> Synthetic Oligonucleotide Reverse Primer

<400> 143
cttcctgcgg ccacagtct 19

<210> 144
<211> 21
<212> DNA
<213> Artificial Sequence

<220>
<223> Synthetic Oligonucleotide Probe

<400> 144
ccagaggccg tttcttggcc g 21

<210> 145
<211> 21
<212> DNA
<213> Artificial Sequence

<220>
<223> Synthetic Oligonucleotide Forward Primer

<400> 145
tccatgatgg ttctgcaggt t 21

<210> 146
<211> 21
<212> DNA
<213> Artificial Sequence

<220>
<223> Synthetic Oligonucleotide Reverse Primer

<400> 146
tgagcagcac catcagtaac g 21
<210> 147
<211> 21
<212> DNA
<213> Artificial Sequence

<220>
<223> Synthetic Oligonucleotide Probe

<400> 147
ccccggacag tggctctgac g 21

<210> 148
<211> 23
<212> DNA
<213> Artificial Sequence

<220>
<223> Synthetic Oligonucleotide Forward Primer

<400> 148
aacgactgct actccaagct caa 23

<210> 149
<211> 22
<212> DNA
<213> Artificial Sequence

<220>
<223> Synthetic Oligonucleotide Reverse Primer

<400> 149
ggatttccat cttgctcacc tt 22

<210> 150
<211> 22
<212> DNA
<213> Artificial Sequence

<220>
<223> Synthetic Oligonucleotide Probe

<400> 150
tgcccagcat cccccagaac aa 22

<210> 151
<211> 21
<212> DNA
<213> Artificial Sequence

<220>
<223> Synthetic Oligonucleotide Forward Primer

<400> 151
gcatggtagc cgaagatttc a 21

<210> 152
<211> 30
<212> DNA
<213> Artificial Sequence

<220>
<223> Synthetic Oligonucleotide Reverse Primer

<400> 152
tttccggtaa tagtctgtct catagatatc 30

<210> 153
<211> 28
<212> DNA
<213> Artificial Sequence

<220>
<223> Synthetic Oligonucleotide Probe

<400> 153
cgcgtcatac caaaatctcc gattttga 28

<210> 154
<211> 20
<212> DNA
<213> Artificial Sequence

<220>
<223> Synthetic Oligonucleotide Forward Primer

<400> 154
cctgaacctt ccaaagatgg 20

<210> 155
<211> 20
<212> DNA
<213> Artificial Sequence

<220>
<223> Synthetic Oligonucleotide Reverse Primer

<400> 155
accaggcaag tctcctcatt 20

<210> 156
<211> 27
<212> DNA
<213> Artificial Sequence

<220>
<223> Synthetic Oligonucleotide Probe

<400> 156
ccagattgga agcatccatc tttttca 27

<210> 157
<211> 20
<212> DNA
<213> Artificial Sequence

<220>
<223> Synthetic Oligonucleotide Forward Primer

<400> 157
agccatcact ctcagtgcag 20

<210> 158
<211> 20
<212> DNA
<213> Artificial Sequence

<220>
<223> Synthetic Oligonucleotide Reverse Primer

<400> 158
actgcagagt cagggtctcc 20

<210> 159
<211> 22
<212> DNA
<213> Artificial Sequence

<220>
<223> Synthetic Oligonucleotide Probe

<400> 159
caggtcctat cgtggcccct ga 22

<210> 160
<211> 20
<212> DNA
<213> Artificial Sequence

<220>
<223> Synthetic Oligonucleotide Foward Primer

<400> 160
ccacagctca ccttctgtca 20

<210> 161
<211> 20
<212> DNA
<213> Artificial Sequence

<220>
<223> Synthetic Oligonucleotide Reverse Primer

<400> 161
cctcagtgcc agtctcttcc 20

<210> 162
<211> 20
<212> DNA
<213> Artificial Sequence

<220>
<223> Synthetic Oligonucleotide Probe

<400> 162
tccatcccag ctccagccag 20

<210> 163
<211> 20
<212> DNA
<213> Artificial Sequence

<220>
<223> Synthetic Oligonucleotide Forward Primer

<400> 163
agagatcgag gctctcaagg 20

<210> 164
<211> 20
<212> DNA

<213> Artificial Sequence

<220>
<223> Synthetic Oligonucleotide Reverse Primer

<400> 164
ggccttttac ttcctcttcg 20

<210> 165
<211> 27
<212> DNA
<213> Artificial Sequence

<220>
<223> Synthetic Oligonucleotide Probe

<400> 165
tggttcttct tcatgaagag cagctcc 27

<210> 166
<211> 23
<212> DNA
<213> Artificial Sequence

<220>
<223> Synthetic Oligonucleotide Forward Primer

<400> 166
tgagtggaaa agcctgacct atg 23

<210> 167
<211> 23
<212> DNA
<213> Artificial Sequence

<220>
<223> Synthetic Oligonucleotide Reverse Primer

<400> 167
ggactccatc tcttcttggt caa 23

<210> 168
<211> 27
<212> DNA
<213> Artificial Sequence

<220>
<223> Synthetic Oligonucleotide Probe

<400> 168
tgaagtcatc agctttgtgc caccacc 27

<210> 169
<211> 21
<212> DNA
<213> Artificial Sequence

<220>

<223> Synthetic Oligonucleotide Forward Primer

<400> 169
gacttttgcc cgctaccttt c 21

<210> 170
<211> 26
<212> DNA
<213> Artificial Sequence

<220>
<223> Synthetic Oligonucleotide Reverse Primer

<400> 170
gccactaact gcttcagtat gaagag 26

<210> 171
<211> 24
<212> DNA
<213> Artificial Sequence

<220>
<223> Synthetic Oligonucleotide Probe

<400> 171
acagctcatt gttgtcacgc cgga 24

<210> 172
<211> 24
<212> DNA
<213> Artificial Sequence

<220>
<223> Synthetic Oligonucleotide Forward Primer

<400> 172
tgatggtcct atgtgtcaca ttca 24

<210> 173
<211> 20
<212> DNA
<213> Artificial Sequence

<220>
<223> Synthetic Oligonucleotide Reverse Primer

<400> 173
tgggacagga aacacaccaa 20

<210> 174
<211> 30
<212> DNA
<213> Artificial Sequence

<220>
<223> Synthetic Oligonucleotide Probe

<400> 174

caggtttcat accaacacag gcttcagcac 30

<210> 175
<211> 19
<212> DNA
<213> Artificial Sequence

<220>
<223> Synthetic Oligonucleotide Forward Primer

<400> 175
cgctcagcca gatgcaatc 19

<210> 176
<211> 25
<212> DNA
<213> Artificial Sequence

<220>
<223> Synthetic Oligonucleotide Reverse Primer

<400> 176
gcactgagat cttcctattg gtgaa 25

<210> 177
<211> 21
<212> DNA
<213> Artificial Sequence

<220>
<223> Synthetic Oligonucleotide Probe

<400> 177
tgccccagtc acctgctgtt a 21

<210> 178
<211> 20
<212> DNA
<213> Artificial Sequence

<220>
<223> Synthetic Oligonucleotide Forward Primer

<400> 178
gtgaaatgaa acgcaccaca 20

<210> 179
<211> 20
<212> DNA
<213> Artificial Sequence

<220>
<223> Synthetic Oligonucleotide Reverse Primer

<400> 179
gaccctgctc acaaccagac 20

<210> 180

<211> 20
<212> DNA
<213> Artificial Sequence

<220>
<223> Synthetic Oligonucleotide Probe

<400> 180
cagccctttg gggaagctgg 20

<210> 181
<211> 19
<212> DNA
<213> Artificial Sequence

<220>
<223> Synthetic Oligonucleotide Forward Primer

<400> 181
ccaacgcttg ccaaatcct 19

<210> 182
<211> 24
<212> DNA
<213> Artificial Sequence

<220>
<223> Synthetic Oligonucleotide Reverse Primer

<400> 182
acggtagtga cagcatcaaa actc 24

<210> 183
<211> 24
<212> DNA
<213> Artificial Sequence

<220>
<223> Synthetic Oligonucleotide Probe

<400> 183
aaccagctct ctgtgacccc aatt 24

<210> 184
<211> 21
<212> DNA
<213> Artificial Sequence

<220>
<223> Synthetic Oligonucleotide Forward Primer

<400> 184
tgattaccat catggctcag a 21

<210> 185
<211> 20
<212> DNA
<213> Artificial Sequence

<220>
<223> Synthetic Oligonucleotide Reverse Primer

<400> 185
cttgtgaaaa tgccatccac 20

<210> 186
<211> 24
<212> DNA
<213> Artificial Sequence

<220>
<223> Synthetic Oligonucleotide Probe

<400> 186
tcccaattgt cgcttcttct gcag 24
<210> 187
<211> 19
<212> DNA
<213> Artificial Sequence

<220>
<223> Synthetic Oligonucleotide Forward Sequence

<400> 187
ccgccctcac ctgaagaga 19

<210> 188
<211> 22
<212> DNA
<213> Artificial Sequence

<220>
<223> Synthetic Oligonucleotide Reverse Sequence

<400> 188
ggaataagtt agccgcgctt ct 22

<210> 189
<211> 21
<212> DNA
<213> Artificial Sequence

<220>
<223> Synthetic Oligonucleotide Probe

<400> 189
cccagtgtcc gccaaggagc g 21

<210> 190
<211> 20
<212> DNA
<213> Artificial Sequence

<220>
<223> Synthetic Oligonucleotide Forward Primer

<400> 190

ggccaggatc tgtggtggta 20

<210> 191
<211> 20
<212> DNA
<213> Artificial Sequence

<220>
<223> Synthetic Oligonucleotide Reverse Primer

<400> 191
ctccacgtcg tggacattga 20

<210> 192
<211> 23
<212> DNA
<213> Artificial Sequence

<220>
<223> Synthetic Oligonucleotide Probe

<400> 192
ctctggcctt ccgagaaggt acc 23

<210> 193
<211> 20
<212> DNA
<213> Artificial Sequence

<220>
<223> Synthetic Oligonucleotide Forward Primer

<400> 193
ggctgtggct gaggctgtag 20

<210> 194
<211> 21
<212> DNA
<213> Artificial Sequence

<220>
<223> Synthetic Oligonucleotide Reverse Primer

<400> 194
ggagcattcg aggtcaaatc a 21

<210> 195
<211> 28
<212> DNA
<213> Artificial Sequence

<220>
<223> Synthetic Oligonucleotide Probe

<400> 195
ttcccagagt gtctcacctc cagcagag 28

<210> 196

<211> 20
<212> DNA
<213> Artificial Sequence

<220>
<223> Synthetic Oligonucleotide Forward Primer

<400> 196
gcatcaggct gtcattatgg 20

<210> 197
<211> 20
<212> DNA
<213> Artificial Sequence

<220>
<223> Synthetic Oligonucleotide Reverse Primer

<400> 197
agtagttgtg ctgcccttcc 20

<210> 198
<211> 28
<212> DNA
<213> Artificial Sequence

<220>
<223> Synthetic Oligonucleotide Probe

<400> 198
tgtccttacc tgtgggagct gtaaggtc 28

<210> 199
<211> 20
<212> DNA
<213> Artificial Sequence

<220>
<223> Synthetic Oligonucleotide Forward Primer

<400> 199
ctgacacttg ccgcagagaa 20

<210> 200
<211> 20
<212> DNA
<213> Artificial Sequence

<220>
<223> Synthetic Oligonucleotide Reverse Primer

<400> 200
aggtggtcct tggtctggaa 20

<210> 201
<211> 26
<212> DNA
<213> Artificial Sequence

<220>
<223> Synthetic Oligonucleotide Probe

<400> 201
ccctttctca cccacctcat ctgcac 26

<210> 202
<211> 23
<212> DNA
<213> Artificial Sequence

<220>
<223> Synthetic Oligonucleotide Forward Primer

<400> 202
cgcttgccta actcatactt tcc 23

<210> 203
<211> 20
<212> DNA
<213> Artificial Sequence

<220>
<223> Synthetic Oligonucleotide Reverse Primer

<400> 203
ccattcagac tgcgccactt 20

<210> 204
<211> 19
<212> DNA
<213> Artificial Sequence

<220>
<223> Synthetic Oligonucleotide Probe

<400> 204
tccacgcagc gtggcactg 19

<210> 205
<211> 21
<212> DNA
<213> Artificial Sequence

<220>
<223> Synthetic Oligonucleotide Forward Primer

<400> 205
gcgacagctc ctctagttcc a 21

<210> 206
<211> 19
<212> DNA
<213> Artificial Sequence

<220>
<223> Synthetic Oligonucleotide Probe

<400> 206
ttcccgaagt ctccgcccg 19

<210> 207
<211> 22
<212> DNA
<213> Artificial Sequence

<220>
<223> Synthetic Oligonucleotide Reverse Primer

<400> 207
ggaacaccag cttgaatttc ct 22

<210> 208
<211> 26
<212> DNA
<213> Artificial Sequence

<220>
<223> Synthetic Oligonucleotide Forward Primer

<400> 208
ggtgtcagat ataaatgtgc aaatgc 26

<210> 209
<211> 24
<212> DNA
<213> Artificial Sequence

<220>
<223> Synthetic Oligonucleotide Reverse Primer

<400> 209
ttcgatattg ccagcagcta taaa 24
<210> 210
<211> 28
<212> DNA
<213> Artificial Sequence

<220>
<223> Synthetic Oligonucleotide Probe

<400> 210
tgctgtcctg tcggtctcag tacgttca 28

<210> 211
<211> 20
<212> DNA
<213> Artificial Sequence

<220>
<223> Synthetic Oligonucleotide Forward Primer

<400> 211
tgtggatgct ggattgattt 20

<210> 212

<211> 20
<212> DNA
<213> Artificial Sequence

<220>
<223> Synthetic Oligonucleotide Reverse Primer

<400> 212
aagcagcact tcctggtctt 20

<210> 213
<211> 25
<212> DNA
<213> Artificial Sequence

<220>
<223> Synthetic Oligonucleotide Probe

<400> 213
ccactggtgc agctgctaaa tagca 25

<210> 214
<211> 20
<212> DNA
<213> Artificial Sequence

<220>
<223> Synthetic Oligonucleotide Forward Primer

<400> 214
agtgggagac acctgacctt 20

<210> 215
<211> 20
<212> DNA
<213> Artificial Sequence

<220>
<223> Synthetic Oligonucleotide Reverse Primer

<400> 215
tgatctgggc attgtactcc 20

<210> 216
<211> 29
<212> DNA
<213> Artificial Sequence

<220>
<223> Synthetic Oligonucleotide Probe

<400> 216
ttgatcttct gctcaatctc agcttgaga 29

<210> 217
<211> 18
<212> DNA
<213> Artificial Sequence

<220>
<223> Synthetic Oligonucleotide Forward Primer

<400> 217
cccgttcggt ctgaggaa 18

<210> 218
<211> 22
<212> DNA
<213> Artificial Sequence

<220>
<223> Synthetic Oligonucleotide Reverse Primer

<400> 218
gagcactcaa ggtagccaaa gg 22

<210> 219
<211> 19
<212> DNA
<213> Artificial Sequence

<220>
<223> Synthetic Oligonucleotide Probe

<400> 219
tccggttcgc catgtcccg 19

<210> 220
<211> 20
<212> DNA
<213> Artificial Sequence

<220>
<223> Synthetic Oligonucleotide Forward Primer

<400> 220
aacagagaca ttgccaacca 20

<210> 221
<211> 20
<212> DNA
<213> Artificial Sequence

<220>
<223> Synthetic Oligonucleotide Reverse Primer

<400> 221
gtgatttgcc caggaagttt 20

<210> 222
<211> 24
<212> DNA
<213> Artificial Sequence

<220>
<223> Synthetic Oligonucleotide Probe

<400> 222
ttggatctgc ttgctgtcca aacc 24

<210> 223
<211> 20
<212> DNA
<213> Artificial Sequence

<220>
<223> Synthetic Oligonucleotide Forward Primer

<400> 223
cgcgagcccc tcattataca 20

<210> 224
<211> 19
<212> DNA
<213> Artificial Sequence

<220>
<223> Synthetic Oligonucleotide Reverse Primer

<400> 224
cactcgccgt tgacatcct 19

<210> 225
<211> 22
<212> DNA
<213> Artificial Sequence

<220>
<223> Synthetic Oligonucleotide Probe

<400> 225
ctccccacag cgcatcgagg aa 22

<210> 226
<211> 23
<212> DNA
<213> Artificial Sequence

<220>
<223> Synthetic Oligonucleotide Forward Primer

<400> 226
cagtggagac cagttgggta gtg 23

<210> 227
<211> 20
<212> DNA
<213> Artificial Sequence

<220>
<223> Synthetic Oligonucleotide Reverse Primer

<400> 227
ccttgaagag cgtcccatca 20

<210> 228
<211> 28
<212> DNA
<213> Artificial Sequence

<220>
<223> Synthetic Oligonucleotide Probe

<400> 228
cacacatgca gagcttgtag cgtaccca 28

<210> 229
<211> 20
<212> DNA
<213> Artificial Sequence

<220>
<223> Synthetic Oligonucleotide Forward Primer

<400> 229
gggctcagct ttcagaagtg 20

<210> 230
<211> 20
<212> DNA
<213> Artificial Sequence

<220>
<223> Synthetic Oligonucleotide Reverse Primer

<400> 230
acatgttcag ctggtccaca 20

<210> 231
<211> 25
<212> DNA
<213> Artificial Sequence

<220>
<223> Synthetic Oligonucleotide Probe

<400> 231
tggcagtttt cttctgtcac caaaa 25

<210> 232
<211> 20
<212> DNA
<213> Artificial Sequence

<220>
<223> Synthetic Oligonucleotide Forward Primer

<400> 232
tcacatgcca ctttggtgtt 20

<210> 233
<211> 20
<212> DNA

<213> Artificial Sequence

<220>
<223> Synthetic Oligonucleotide Reverse Primer

<400> 233
cttgcaggaa gcggctatac 20

<210> 234
<211> 22
<212> DNA
<213> Artificial Sequence

<220>
<223> Synthetic Oligonucleotide Probe

<400> 234
tcctgggaga gattgaccag ca 22

<210> 235
<211> 19
<212> DNA
<213> Artificial Sequence

<220>
<223> Synthetic Oligonucleotide Forward Primer

<400> 235
gcccgaaacg ccgaatata 19

<210> 236
<211> 23
<212> DNA
<213> Artificial Sequence

<220>
<223> Synthetic Oligonucleotide Reverse Primer

<400> 236
cgtggctctc ttatcctcat gat 23

<210> 237
<211> 21
<212> DNA
<213> Artificial Sequence

<220>
<223> Synthetic Oligonucleotide Probe

<400> 237
taccgcagca aaccgcttgg g 21

<210> 238
<211> 20
<212> DNA
<213> Artificial Sequence

<220>

<223> Synthetic Oligonucleotide Forward Primer

<400> 238
gccgggaaga ccgtaattgt 20

<210> 239
<211> 18
<212> DNA
<213> Artificial Sequence

<220>
<223> Synthetic Oligonucleotide Reverse Primer

<400> 239
cagcggcacc aggttcag 18

<210> 240
<211> 26
<212> DNA
<213> Artificial Sequence

<220>
<223> Synthetic Oligonucleotide Probe

<400> 240
caaatggctt cctctggaag gtccca 26

<210> 241
<211> 20
<212> DNA
<213> Artificial Sequence

<220>
<223> Synthetic Oligonucleotide Forward Primer

<400> 241
tgctgttgct gagtctgttg 20

<210> 242
<211> 20
<212> DNA
<213> Artificial Sequence

<220>
<223> Synthetic Oligonucleotide Reverse Primer

<400> 242
cttgcctggc ttcacagata 20

<210> 243
<211> 24
<212> DNA
<213> Artificial Sequence

<220>
<223> Synthetic Oligonucleotide Probe

<400> 243

ccagtcccca gaagaccatg tctg 24

<210> 244
<211> 20
<212> DNA
<213> Artificial Sequence

<220>
<223> Synthetic Oligonucleotide Forward Primer

<400> 244
tgtggtgagg aaggagtcag 20

<210> 245
<211> 18
<212> DNA
<213> Artificial Sequence

<220>
<223> Synthetic Oligonucleotide Reverse Primer

<400> 245
cccagagagt gggtcagc 18

<210> 246
<211> 24
<212> DNA
<213> Artificial Sequence

<220>
<223> Synthetic Oligonucleotide Probe

<400> 246
ctgtgactgt ctccagggct tcca 24

<210> 247
<211> 21
<212> DNA
<213> Artificial Sequence

<220>
<223> Synthetic Oligonucleotide Forward Primer

<400> 247
tggcttcagg agctgaatac c 21

<210> 248
<211> 24
<212> DNA
<213> Artificial Sequence

<220>
<223> Synthetic Oligonucleotide Reverse Primer

<400> 248
tgctgtcgtg atgagaaaat agtg 24

<210> 249

<211> 26
<212> DNA
<213> Artificial Sequence

<220>
<223> Synthetic Oligonucleotide Probe

<400> 249
caggcacaca caggtgggac acaaat 26
<210> 250
<211> 25
<212> DNA
<213> Artificial Sequence

<220>
<223> Synthetic Oligonucleotide Forward Primer

<400> 250
gtatcaggac cacatgcagt acatc 25

<210> 251
<211> 22
<212> DNA
<213> Artificial Sequence

<220>
<223> Synthetic Oligonucleotide Reverse Primer

<400> 251
tgtcggaatt gatactggca tt 22

<210> 252
<211> 24
<212> DNA
<213> Artificial Sequence

<220>
<223> Synthetic Oligonucleotide Probe

<400> 252
ttgatgcctg tcttcgcgcc ttct 24

<210> 253
<211> 20
<212> DNA
<213> Artificial Sequence

<220>
<223> Synthetic Oligonucleotide Forward Primer

<400> 253
tttccaaaca tcagcgagtc 20

<210> 254
<211> 20
<212> DNA
<213> Artificial Sequence

<220>
<223> Synthetic Oligonucleotide Reverse Primer

<400> 254
aacaggagcg cttgaaagtt 20

<210> 255
<211> 23
<212> DNA
<213> Artificial Sequence

<220>
<223> Synthetic Oligonucleotide Probe

<400> 255
acggtgcttc tccctctcca gtg 23

<210> 256
<211> 78
<212> DNA
<213> Artificial Sequence

<220>
<223> Amplicon

<400> 256

cgttccgatc ctctatactg catcccaggc atgcctacag caccctgatg tcgcagccta 60
taaggccaac agggacct 78

<210> 257
<211> 76
<212> DNA
<213> Artificial Sequence

<220>
<223> Amplicon

<400> 257

tgcagactgt accatgctga ccattgccca tcgcctgcac acggttctag gctccgatag 60
gattatggtg ctggcc 76

<210> 258
<211> 78
<212> DNA
<213> Artificial Sequence

<220>
<223> Amplicon

<400> 258

ctgcatgtga ttgaataaga aacaagaaag tgaccacacc aaagcctccc tggctggtgt 60
acagggatca ggtccaca 78

<210> 259
<211> 68
<212> DNA
<213> Artificial Sequence

<220>
<223> Amplicon

<400> 259

```
acgaattgtc ggtgaggtct caggatacca cagtcctgga gaccctatcc aatgatttca 60
gcatggac                                                          68
```

<210> 260
<211> 71
<212> DNA
<213> Artificial Sequence

<220>
<223> Amplicon

<400> 260

```
cgcttctatg gcgctgagat tgtgtcagcc ctggactacc tgcactcgga gaagaacgtg 60
gtgtaccggg a                                                      71
```

<210> 261
<211> 71
<212> DNA
<213> Artificial Sequence

<220>
<223> Amplicon

<400> 261

```
tcctgccacc cttcaaacct caggtcacgt ccgaggtcga cacaaggtac ttcgatgatg 60
aatttaccgc c                                                      71
```

<210> 262
<211> 69
<212> DNA
<213> Artificial Sequence

<220>
<223> Amplicon

<400> 262

```
ggacagcagg aatgtgtttc tccatacagg tcacggggag ccaatggttc agaaacaaat 60
cgagtgggt                                                         69
```

<210> 263
<211> 73

<212> DNA
<213> Artificial Sequence

<220>
<223> Amplicon

<400> 263

```
gggtcaggtg cctcgagatc gggcttgggc ccagagcatg ttccagatcc cagagtttga 60
gccgagtgag cag                                                    73
```

<210> 264
<211> 81
<212> DNA
<213> Artificial Sequence

<220>
<223> Amplicon

<400> 264

```
cgttgtcagc acttggaata caagatggtt gccgggtcat gttaattggg aaaaagaaca 60
gtccacagga agaggttgaa c                                           81
```

<210> 265
<211> 83
<212> DNA
<213> Artificial Sequence

<220>
<223> Amplicon

<400> 265

```
cctggagggt cctgtacaat ctcatcatgg gactcctgcc cttacccagg ggccacagag 60
cccccgagat ggagcccaat tag                                         83
```

<210> 266
<211> 73
<212> DNA
<213> Artificial Sequence

<220>
<223> Amplicon

<400> 266

```
cagatggacc tagtacccac tgagatttcc acgccgaagg acagcgatgg gaaaaatgcc 60
cttaaatcat agg                                                    73
```

<210> 267
<211> 69
<212> DNA
<213> Artificial Sequence

<220>
<223> Amplicon

<400> 267

```
gcatgttcgt ggcctctaag atgaaggaga ccatccccct gacggccgag aagctgtgca 60
tctacaccg                                                          69
```

<210> 268
<211> 81
<212> DNA
<213> Artificial Sequence

<220>
<223> Amplicon

<400> 268

```
cgtcaggacc caccatgtct gccccatcac gcggccgaga catggcttgg ccacagctct 60
tgaggatgtc accaattaac c                                            81
```

<210> 269
<211> 75
<212> DNA
<213> Artificial Sequence

<220>
<223> Amplicon

<400> 269

```
tgtatttcaa gacctctgtg cacttattta tgaacctgcc ctgctcccac agaacacagc 60
aattcctcag gctaa                                                   75
```

<210> 270
<211> 65
<212> DNA
<213> Artificial Sequence

<220>
<223> Amplicon

<400> 270

```
agatgaagtg gaaggcgctt ttcaccgcgg ccatcctgca ggcacagttg ccgattacag 60
aggca                                                              65
```

<210> 271
<211> 64
<212> DNA
<213> Artificial Sequence

<220>
<223> Amplicon

<400> 271

```
gggcgtggaa cagtttatct cagacatctg ccccaagaag gacgtactcg aaaccttcac 60
cgtg                                                              64
```

<210> 272
<211> 68
<212> DNA
<213> Artificial Sequence

<220>
<223> Amplicon

<400> 272

```
tggattggag ttctgggaat gtactggccg tggcactgga caacagtgtg tacctgtgga 60
gtgcaagc                                                          68
```

<210> 273
<211> 85
<212> DNA
<213> Artificial Sequence

<220>
<223> Amplicon

<400> 273

```
aaacgagcag tttgccatca gacgcttcca gtctatgccg gtgaggctgc tgggccacag 60
ccccgtgctt cggaacatca ccaac                                       85
```

<210> 274
<211> 77
<212> DNA
<213> Artificial Sequence

<220>
<223> Amplicon

<400> 274

```
tgacaatcag cacacctgca ttcaccgctc ggaagagggc ctgagctgca tgaataagga 60
tcacggctgt agtcaca                                                77
```

<210> 275
<211> 76
<212> DNA
<213> Artificial Sequence

<220>
<223> Amplicon

<400> 275

```
ctgaaggagc tccaagacct cgctctccaa ggcgccaagg agagggcaca tcagcagaag 60
aaacacagcg gttttg                                                 76
```

<210> 276
<211> 68
<212> DNA
<213> Artificial Sequence

<220>
<223> Amplicon

<400> 276

```
gtggccatcc agctgacctt cctgcgcctg atgtccaccg aggcctccca gaacatcacc 60
taccactg                                                          68
```

<210> 277
<211> 80
<212> DNA
<213> Artificial Sequence

<220>
<223> Amplicon

<400> 277

```
cagccaagaa ctggtatagg agctccaagg acaagaaaca cgtctggcta ggagaaacta 60
tcaatgctgg cagccagttt                                             80
```

<210> 278
<211> 81
<212> DNA
<213> Artificial Sequence

<220>
<223> Amplicon

<400> 278

```
cgacagttgc gatgaaagtt ctaatctctt ccctcctcct gttgctgcca ctaatgctga 60
tgtccatggt ctctagcagc c                                           81
```

<210> 279
<211> 67
<212> DNA
<213> Artificial Sequence

<220>
<223> Amplicon

<400> 279

aacttcaagg tcggagaagg ctttgaggag gagaccgtgg acggacgcaa gtgcaggagt 60
ttagcca 67

<210> 280
<211> 76
<212> DNA
<213> Artificial Sequence

<220>
<223> Amplicon

<400> 280

gtgctacgcc accctgttcg gacccaaagg cgtgaacatc gggggcgcgg gctcctacat 60
ctacgagaag cccctg 76

<210> 281
<211> 77
<212> DNA
<213> Artificial Sequence

<220>
<223> Amplicon

<400> 281

ggtgcctact ccattgtggc gggcgtgttt gtgtgcctgc tggagtaccc ccgggggaag 60
aggaagaagg gctccac 77

<210> 282
<211> 78
<212> DNA
<213> Artificial Sequence

<220>
<223> Amplicon

<400> 282

gggagaacgg gatcaatagg atcggaaacc tgctggtgcc caatgagaat tactgcaagt 60
ttgaggactg gctgatgc 78

<210> 283
<211> 68
<212> DNA
<213> Artificial Sequence

<220>
<223> Amplicon

<400> 283

tccaattcca gcatcactgt ggagaaaagc tgtttgtctc cccagcatac tttatcgcct 60
tcactgcc 68

<210> 284
<211> 83
<212> DNA
<213> Artificial Sequence

<220>
<223> Amplicon

<400> 284

tgcacagagg gtgtgggtta caccaatgct tccaacaatt tgtttgcttg cctcccatgt 60
acagcttgta aatcagatga aga 83

<210> 285
<211> 75
<212> DNA
<213> Artificial Sequence

<220>
<223> Amplicon

<400> 285

actccctcta cccttgagca agggcagggg tccctgagct gttcttctgc cccatactga 60
aggaactgag gcctg 75

<210> 286
<211> 76
<212> DNA
<213> Artificial Sequence

<220>
<223> Amplicon

<400> 286

tggtccatcg ccagttatca catctgtatg cggaacctca aaagagtccc tggtgtgaag 60
caagatcgct agaaca 76

<210> 287
<211> 81
<212> DNA
<213> Artificial Sequence

<220>
<223> Amplicon

<400> 287

```
cggttatgtc atgccagata cacacctcaa aggtactccc tcctcccggg aaggcaccct 60
ttcttcagtg ggtctcagtt c                                           81
```

<210> 288
<211> 86
<212> DNA
<213> Artificial Sequence

<220>
<223> Amplicon

<400> 288

```
tggctcttaa tcagtttcgt tacctgcctc tggagaattt acgcattatt cgtgggacaa 60
aactttatga ggatcgatat gccttg                                      86
```

<210> 289
<211> 67
<212> DNA
<213> Artificial Sequence

<220>
<223> Amplicon

<400> 289

```
gtccaggtgg atgtgaaaga tccccagcag gccctcaagg agctggctaa gatgtgtatc 60
ctggccg                                                            67
```

<210> 290
<211> 67
<212> DNA
<213> Artificial Sequence

<220>
<223> Amplicon

<400> 290

```
acggatcaca gtggaggaag cgctggctca cccctacctg gagcagtact atgacccgac 60
ggatgag                                                            67
```

<210> 291
<211> 67
<212> DNA
<213> Artificial Sequence

<220>
<223> Amplicon

<400> 291

ccagcaccat tgttgaagat ccccagacca agtgtgaata catgctcaac tcgatgccca 60
agagact 67

<210> 292
<211> 80
<212> DNA
<213> Artificial Sequence

<220>
<223> Amplicon

<400> 292

gcactttggg attctttcca ttatgattct ttgttacagg caccgagaat gttgtattca 60
gtgagggtct tcttacatgc 80

<210> 293
<211> 90
<212> DNA
<213> Artificial Sequence

<220>
<223> Amplicon

<400> 293

ggctattcct cattttctct acaaagtggc ctcagtgaac atgaagaagg tagcctcctg 60
gaggagaatt tcggtgacag tctacaatcc 90

<210> 294
<211> 67
<212> DNA
<213> Artificial Sequence

<220>
<223> Amplicon

<400> 294

ccagtggagc gcttccatga cctgcgtcct gatgaagtgg ccgatttgtt tcagacgacc 60
cagagag 67

<210> 295
<211> 80
<212> DNA
<213> Artificial Sequence

<220>
<223> Amplicon

<400> 295

```
ggataattca gacaacaaca ccatctttgt gcaaggcctg ggtgagaatg ttacaattga 60
gtctgtggct gattacttca                                              80
```

<210> 296
<211> 69
<212> DNA
<213> Artificial Sequence

<220>
<223> Amplicon

<400> 296

```
gaagcgcaga tcatgaagaa gctgaagcac gacaagctgg tccagctcta tgcagtggtg 60
tctgaggag                                                          69
```

<210> 297
<211> 68
<212> DNA
<213> Artificial Sequence

<220>
<223> Amplicon

<400> 297

```
tcagcagcaa gggcatcatg gaggaggatg aggcctgcgg gcgccagtac acgctcaaga 60
aaaccacc                                                           68
```

<210> 298
<211> 75
<212> DNA
<213> Artificial Sequence

<220>
<223> Amplicon

<400> 298

```
caaaggagct cactgtggtg tctgtgttcc aaccactgaa tctggacccc atctgtgaat 60
aagccattct gactc                                                   75
```

<210> 299
<211> 73
<212> DNA
<213> Artificial Sequence

<220>
<223> Amplicon

<400> 299

```
ttgggaaata tttgggcatt ggtctggcca agtctacaat gtcccaatat caaggacaac 60
caccctagct tct                                                     73
```

<210> 300
<211> 83
<212> DNA
<213> Artificial Sequence

<220>
<223> Amplicon

<400> 300

```
gcatgggaac catcaaccag caggccatgg accaacttca ctatgtgaca gagctgacag 60
atcgaatcaa ggcaaactcc tca                                         83
```

<210> 301
<211> 70
<212> DNA
<213> Artificial Sequence

<220>
<223> Amplicon

<400> 301

```
ctccgacgtg gctttccagt ggcccacagc atctatggaa tcccatctgt catcaattct 60
gccaattacg                                                        70
```

<210> 302
<211> 67
<212> DNA
<213> Artificial Sequence

<220>
<223> Amplicon

<400> 302

```
ccatctgcat ccatcttgtt tgggctcccc acccttgaga agtgcctcag ataataccct 60
ggtggcc                                                           67
```

<210> 303
<211> 84
<212> DNA
<213> Artificial Sequence

<220>
<223> Amplicon

<400> 303

```
aggctgctgg aggtcatctc cgtgtgtgat tgccccagag gccgtttctt ggccgccatc 60
tgccaagact gtggccgcag gaag                                        84
```

<210> 304
<211> 73

<212> DNA
<213> Artificial Sequence

<220>
<223> Amplicon

<400> 304

```
tccatgatgg ttctgcaggt ttctgcggcc ccccggacag tggctctgac ggcgttactg 60
atggtgctgc tca                                                    73
```

<210> 305
<211> 76
<212> DNA
<213> Artificial Sequence

<220>
<223> Amplicon

<400> 305

```
aacgactgct actccaagct caaggagctg gtgcccagca tcccccagaa caagaaggtg 60
agcaagatgg aaatcc                                                 76
```

<210> 306
<211> 83
<212> DNA
<213> Artificial Sequence

<220>
<223> Amplicon

<400> 306

```
gcatggtagc cgaagatttc acagtcaaaa tcggagattt tggtatgacg cgagatatct 60
atgagacaga ctattaccgg aaa                                         83
```

<210> 307
<211> 72
<212> DNA
<213> Artificial Sequence

<220>
<223> Amplicon

<400> 307

```
cctgaacctt ccaaagatgg ctgaaaaaga tggatgcttc caatctggat tcaatgagga 60
gacttgcctg gt                                                     72
```

<210> 308
<211> 63
<212> DNA
<213> Artificial Sequence

<220>
<223> Amplicon

<400> 308

agccatcact ctcagtgcag ccaggtccta tcgtggcccc tgaggagacc ctgactctgc 60
agt 63

<210> 309
<211> 74
<212> DNA
<213> Artificial Sequence

<220>
<223> Amplicon

<400> 309

ccacagctca ccttctgtca ggtgtccatc ccagctccag ccagctccca gagaggaaga 60
gactggcact gagg 74

<210> 310
<211> 68
<212> DNA
<213> Artificial Sequence

<220>
<223> Amplicon

<400> 310

agagatcgag gctctcaagg aggagctgct cttcatgaag aagaaccacg aagaggaagt 60
aaaaggcc 68

<210> 311
<211> 76
<212> DNA
<213> Artificial Sequence

<220>
<223> Amplicon

<400> 311

tgagtggaaa agcctgacct atgatgaagt catcagcttt gtgccaccac cccttgacca 60
agaagagatg gagtcc 76

<210> 312
<211> 75
<212> DNA
<213> Artificial Sequence

<220>
<223> Amplicon

<400> 312

```
gacttttgcc cgctaccttt cattccggcg tgacaacaat gagctgttgc tcttcatact 60
gaagcagtta gtggc                                                  75
```

<210> 313
<211> 82
<212> DNA
<213> Artificial Sequence

<220>
<223> Amplicon

<400> 313

```
tgatggtcct atgtgtcaca ttcatcacag gtttcatacc aacacaggct tcagcacttc 60
ctttggtgtg tttcctgtcc ca                                          82
```

<210> 314
<211> 71
<212> DNA
<213> Artificial Sequence

<220>
<223> Amplicon

<400> 314

```
cgctcagcca gatgcaatca atgccccagt cacctgctgt tataacttca ccaataggaa 60
gatctcagtg c                                                      71
```

<210> 315
<211> 69
<212> DNA
<213> Artificial Sequence

<220>
<223> Amplicon

<400> 315

```
gtgaaatgaa acgcaccaca ctggacagcc ctttggggaa gctggagctg tctggttgtg 60
agcagggtc                                                         69
```

<210> 316
<211> 78
<212> DNA
<213> Artificial Sequence

<220>
<223> Amplicon

<400> 316

```
ccaacgcttg ccaaatcctg acaattcaga accagctctc tgtgacccca atttgagttt 60
tgatgctgtc actaccgt                                                78
```

<210> 317
<211> 82
<212> DNA
<213> Artificial Sequence

<220>
<223> Amplicon

<400> 317

```
tgattaccat catggctcag attggctcct atgttcctgc agaagaagcg acaattggga 60
ttgtggatgg cattttcaca ag                                           82
```

<210> 318
<211> 77
<212> DNA
<213> Artificial Sequence

<220>
<223> Amplicon

<400> 318

```
ccgccctcac ctgaagagaa acgcgctcct tggcggacac tgggggagga gaggaagaag 60
cgcggctaac ttattcc                                                 77
```

<210> 319
<211> 71
<212> DNA
<213> Artificial Sequence

<220>
<223> Amplicon

<400> 319

```
ggccaggatc tgtggtggta caattgactc tggccttccg agaaggtacc atcaatgtcc 60
acgacgtgga g                                                       71
```

<210> 320
<211> 73
<212> DNA
<213> Artificial Sequence

<220>
<223> Amplicon

<400> 320

ggctgtggct gaggctgtag catctctgct ggaggtgaga cactctggga actgatttga 60
cctcgaatgc tcc 73

<210> 321
<211> 85
<212> DNA
<213> Artificial Sequence

<220>
<223> Amplicon

<400> 321

gcatcaggct gtcattatgg tgtccttacc tgtgggagct gtaaggtctt ctttaagagg 60
gcaatggaag ggcagcacaa ctact 85

<210> 322
<211> 68
<212> DNA
<213> Artificial Sequence

<220>
<223> Amplicon

<400> 322

ctgacacttg ccgcagagaa tccctttctc acccacctca tctgcacctt ccagaccaag 60
gaccacct 68

<210> 323
<211> 81
<212> DNA
<213> Artificial Sequence

<220>
<223> Amplicon

<400> 323

cgcttgccta actcatactt tcccgttgac acttgatcca cgcagcgtgg cactgggacg 60
taagtggcgc agtctgaatg g 81

<210> 324
<211> 78
<212> DNA
<213> Artificial Sequence

<220>
<223> Amplicon

<400> 324

gcgacagctc ctctagttcc accatgtccg cgggcggaga cttcgggaat ccgctgagga 60
aattcaagct ggtgttcc 78

<210> 325
<211> 84
<212> DNA
<213> Artificial Sequence

<220>
<223> Amplicon

<400> 325

ggtgtcagat ataaatgtgc aaatgccttc ttgctgtcct gtcggtctca gtacgttcac 60
tttatagctg ctggcaatat cgaa 84

<210> 326
<211> 67
<212> DNA
<213> Artificial Sequence

<220>
<223> Amplicon

<400> 326

tgtggatgct ggattgattt caccactggt gcagctgcta aatagcaaag accaggaagt 60
gctgctt 67

<210> 327
<211> 69
<212> DNA
<213> Artificial Sequence

<220>
<223> Amplicon

<400> 327

agtgggagac acctgacctt tctcaagctg agattgagca gaagatcaag gagtacaatg 60
cccagatca 69

<210> 328
<211> 68
<212> DNA
<213> Artificial Sequence

<220>
<223> Amplicon

<400> 328

```
cccgttcggt ctgaggaagg ccgggacatg gcgaaccgga tcagtgcctt tggctacctt 60
gagtgctc                                                          68
```

<210> 329
<211> 69
<212> DNA
<213> Artificial Sequence

<220>
<223> Amplicon

<400> 329

```
aacagagaca ttgccaacca tattggatct gcttgctgtc caaaccagca aacttcctgg 60
gcaaatcac                                                         69
```

<210> 330
<211> 86
<212> DNA
<213> Artificial Sequence

<220>
<223> Amplicon

<400> 330

```
cgcgagcccc tcattataca ctgggcagcc tccccacagc gcatcgagga atgcgtgctc 60
tcaggcaagg atgtcaacgg cgagtg                                      86
```

<210> 331
<211> 77
<212> DNA
<213> Artificial Sequence

<220>
<223> Amplicon

<400> 331

```
cagtggagac cagttgggta gtggtgactg ggtacgctac aagctctgca tgtgtgctga 60
tgggacgctc ttcaagg                                                77
```

<210> 332
<211> 81
<212> DNA
<213> Artificial Sequence

<220>
<223> Amplicon

<400> 332

```
gggctcagct ttcagaagtg ctgagttggc agttttcttc tgtcaccaaa agaggtctca 60
atgtggacca gctgaacatg t                                           81
```

<210> 333
<211> 70
<212> DNA
<213> Artificial Sequence

<220>
<223> Amplicon

<400> 333

```
tcacatgcca ctttggtgtt tcataatctc ctgggagaga ttgaccagca gtatagccgc 60
ttcctgcaag                                                        70
```

<210> 334
<211> 65
<212> DNA
<213> Artificial Sequence

<220>
<223> Amplicon

<400> 334

```
gcccgaaacg ccgaatataa tcccaagcgg tttgctgcgg taatcatgag gataagagag 60
ccacg                                                             65
```

<210> 335
<211> 84
<212> DNA
<213> Artificial Sequence

<220>
<223> Amplicon

<400> 335

```
gccgggaaga ccgtaattgt ggctgcactg gatgggacct tccagaggaa gccatttggg 60
gccatcctga acctggtgcc gctg                                        84
```

<210> 336
<211> 74
<212> DNA
<213> Artificial Sequence

<220>
<223> Amplicon

<400> 336

tgctgttgct gagtctgttg ccagtcccca gaagaccatg tctgtgttga gctgtatctg 60
tgaagccagg caag 74

<210> 337
<211> 66
<212> DNA
<213> Artificial Sequence

<220>
<223> Amplicon

<400> 337

tgtggtgagg aaggagtcag agagctgtga ctgtctccag ggcttccagc tgacccactc 60
tctggg 66

<210> 338
<211> 89
<212> DNA
<213> Artificial Sequence

<220>
<223> Amplicon

<400> 338

tggcttcagg agctgaatac cctcccaggc acacacaggt gggacacaaa taagggtttt 60
ggaaccacta ttttctcatc acgacagca 89

<210> 339
<211> 75
<212> DNA
<213> Artificial Sequence

<220>
<223> Amplicon

<400> 339

gtatcaggac cacatgcagt acatcggaga aggcgcgaag acaggcatca aagaatgcca 60
gtatcaattc cgaca 75

<210> 340
<211> 65
<212> DNA
<213> Artificial Sequence

<220>
<223> Amplicon

<400> 340

```
tttccaaaca tcagcgagtc cacactggag agggagaagc accgtaactt tcaagcgctc 60
ctgtt                                                             65
```